(19) Europäisches Patentamt

European Patent Office

Office européen des brevets

(11) **EP 0 865 606 B1**

(12) **EUROPEAN PATENT SPECIFICATION**

(45) Date of publication and mention
of the grant of the patent:
**16.03.2005 Bulletin 2005/11**

(51) Int Cl.[7]: **G01N 21/07**, B01L 3/00,
G01N 33/487

(21) Application number: **96943641.9**

(22) Date of filing: **05.12.1996**

(86) International application number:
**PCT/US1996/019514**

(87) International publication number:
**WO 1997/021090 (12.06.1997 Gazette 1997/25)**

(54) **DEVICES AND METHODS FOR USING CENTRIPETAL ACCELERATION TO DRIVE FLUID MOVEMENT IN A MICROFLUIDICS SYSTEM WITH ON-BOARD INFORMATICS**

VORRICHTUNG UND VERFAHREN ZUM BEWEGEN VON FLUIDEN MITTELS ZENTRIFUGALBESCHLEUNIGUNG BEI DER AUTOMATISCHEN LABORBEHANDLUNG

DISPOSITIFS ET PROCEDES D'UTILISATION DE L'ACCELERATION CENTRIPETE POUR COMMANDER LE DEPLACEMENT DE LIQUIDES DANS LE TRAITEMENT DE LABORATOIRE AUTOMATISE

(84) Designated Contracting States:
**AT BE CH DE DK ES FI FR GB GR IE IT LI LU MC NL PT SE**
Designated Extension States:
**AL LT LV RO SI**

(30) Priority: **05.12.1995 US 608215**
**06.12.1995 US 608267**
**18.12.1995 US 608819**
**12.08.1996 US 23756**

(43) Date of publication of application:
**23.09.1998 Bulletin 1998/39**

(73) Proprietor: **Gamera Bioscience Corporation**
**Cambridge, MA 02139 (US)**

(72) Inventors:
• **MIAN, Alec**
**08807 Barcelona (ES)**
• **KIEFFER-HIGGINS, Stephen, G.**
**Dorchester, MA 02124 (US)**
• **COREY, George, D.**
**Newton, MA 02165 (US)**

(74) Representative: **OK pat AG**
**Chamerstrasse 50**
**6300 Zug (CH)**

(56) References cited:
**EP-A- 0 305 210**     **EP-A- 0 322 657**
**EP-A- 0 417 305**     **EP-A- 0 616 218**
**WO-A-93/22058**     **DE-A- 4 410 224**
**US-A- 3 679 367**     **US-A- 4 940 527**

• **CLINICAL CHEMISTRY, vol. 38, no. 9, 1 September 1992, pages 1665-1670, XP000319980 SCHEMBRI C T ET AL: "PORTABLE SIMULTANEOUS MULTIPLE ANALYTE WHOLE-BLOOD ANALYZER FOR POINT-OF-CARE TESTING"**
• **PATENT ABSTRACTS OF JAPAN vol. 016, no. 035 (P-1304), 28 January 1992 & JP 03 245049 A (IDEMITSU PETROCHEM CO LTD), 31 October 1991,**
• **CLINICAL CHEMISTRY, vol. 40, no. 9, 1 September 1994, pages 1805-1809, XP000444697 ARQUINT P ET AL: "MICROMACHINED ANALYZERS ON A SILICON CHIP"**

**Description**

## 1. Field of the Invention

This invention relates to methods and apparatus for performing microanalytic and microsynthetic analyses and procedures. In particular, the invention relates to microminiaturization of genetic, biochemical and chemical processes related to analysis, synthesis and purification. Specifically, the invention provides a microsystem platform and a micromanipulation device to manipulate the platform by rotation, thereby utilizing the centripetal forces resulting from rotation of the platform to motivate fluid movement through microchannels embedded in the microplatform. The microsystem platforms of the invention are also provided having system informatics and data acquisition, analysis and storage and retrieval informatics encoded on the surface of the disk opposite to the surface containing the fluidic components. Methods for performing any of a wide variety of microanalytical or microsynthetic processes using the microsystems apparatus of the invention are also provided.

## 2. Background of the Related Art

In the field of medical, biological and chemical assays, mechanical and automated fluid handling systems and instruments produced to operate on a macroscopic (*i.e.*, milliliters and milligrams) scale are known in the prior art.

U.S. Patent 4, 279,862, issued July 21, 1981 to Bertaudiere *et al.* discloses a centrifugal photometric analyzer.

U.S. Patent 4,381,291, issued April 26, 1983 to Ekins teaches analytic measurement of free ligands.

U.S. Patent 4,515,889, issued May 7, 1985 to Klose *et al.* teaches automated mixing and incubating reagents to perform analytical determinations.

U.S. Patent 4,676,952, issued June 30, 1987 to Edelmann *et al.* teaches a photometric analysis apparatus.

U.S. Patent 4,745,072, issued May 17, 1988 to Ekins discloses immunoassay in biological fluids.

U.S. Patent 5,160,702 issued November 3, 1992 to Kopf-Sill *et al.* discloses a centrifuge rotor for analyzing solids in a liquid.

U.S. Patent 5, 171,695, issued December 15, 1992 to Ekins discloses determination of analyte concentration using two labeling markers.

U.S. Patent 5,173,262 issued December 22, 1996 to Burtis *et al.* discloses a centrifuge rotor for processing liquids.

U.S. Patent 5,242,803, issued September 7, 1993 to Burtis *et al.* discloses a rotor assembly for carrying out an assay.

U.S. Patent 5,409,665, issued April 25, 1995 to Burd discloses cuvette filling in a centrifuge rotor.

U.S. Patent 5,413,732, issued May 9, 1995 to Buhl *et al.* teaches preparation of lyophilized reagent spheres for use in automated centrifugal blood analyzers.

U.S. Patent 5,432,099, issued July 11, 1995 to Ekins discloses a method for analyzing analytes in a liquid.

U.S. Patent 5,472,603 issued December 5, 1995 to Schembri discloses an analytical rotor for performing fluid separations.

Anderson, 1968, *Anal. Biochem.* 28: 545-562 teaches a multiple cuvette rotor for cell fractionation.

Renoe *et al., Clin. Chem.* 20: 955-960 teach a "minidisc" module for a centrifugal analyzer.

Burtis *et al., Clin. Chem.* 20: 932-941 teach a method for dynamic introduction of liquids into a centrifugal analyzer.

Fritsche *et al.* 1975, *Clin. Biochem.* 8: 240-246 teach enzymatic analysis of blood sugar levels using a centrifugal analyzer.

Burtis *et al., Clin. Chem.* 21: 1225-1233 disclose a multipurpose optical system for use with a centrifugal analyzer.

Hadjiioannou *et al.* 1976, *Clin. Chem,* 22: 802-805 teach automated enzymatic ethanol determination in biological fluids using a miniature centrifugal analyzer.

Lee *et al.,* 1978, *Clin. Chem.* 24: 1361-1365 teach an automated blood fractionation system.

Cho *et al.,* 1982, *Clin. Chem.* 28: 1965-1961 teach a multichannel electrochemical centrifugal analyzer.

Bertrand *et al.,* 1982, *Clinica Chimica Acta* 119: 275-284 teach automated determination of serum 5' -nucleotidase using a centrifugal analyzer.

Walters *et al.,* 1995, Basic Medical Laboratory Technologies, 3rd ed., Delmar Publishers: Boston teach a variety of automated medical laboratory analytic techniques.

Recently, microanalytical devices for performing select reaction pathways have been developed.

U.S. Patent 5,006,749, issued April 9, 1991 to White disclose methods and apparatus for using ultrasonic energy to move microminiature elements.

U.S. Patent No. 5,252,294, issued October 12, 1993 to Kroy *et al.* teach a micromechanical structure for performing certain chemical microanalyses.

U.S. Patent 5,304,487, issued April 19, 1994 to Wilding *et al.* teach fluid handling on microscale analytical devices.

U.S. Patent 5,368,704 issued November 29, 1994 to Madou *et al.* teach microelectrochemical valves.

International Application, Publication No. WO93/22053, published 11 November 1993 to University of Pennsylvania disclose microfabricated detection structures.

International Application, Publication No. WO93/22058, published 11 November 1993 to University of Pennsylvania disclose microfabricated structures for performing polynucleotide amplification.

Columbus *et al.*, 1987, *Clin. Chem.* 33: 1531-1537 teach fluid management of biological fluids.

Ekins *et al.,* 1992, *Ann. Biol. Clin.* 50: 337-353 teach a multianalytical microspot immunoassay.

Wilding *et al.,* 1994, *Clin. Chem.* 40: 43-47 disclose manipulation of fluids on straight channels micromachined into silicon.

European Patent Application, Publication No. EP0616218A to Hitachi Ltd., 21 September 1994 discloses a non-centrifugal analysis system.

Schrembri *et al*, 1992, *Clin. Chem.* 38: 1665-1670 discloses a centrifuge rotor.

International Application, Publication No. WO93/22058 to Trustees of the University of Pennsylvania discloses a non-centrifugal analysis system.

European Patent Application, Publication No. EP0305210A to Biotrack Inc., 1 March 1989 discloses a stop-flow valving.

European Patent Application, Publication No. EP0322657A to Miles Inc., 5 July 1989 discloses a capillary valving in a centrifuge rotor.

German Patent Application, Publication No. DE4410224A to Knoll Meinhard, 28 September 1995 discloses a capillary path in a non-centrifuge system.

Arquint *et al*, 1994, *Clin. Chem.* 40: 1805-1809 discloses a non-centrifugal micromachined analysis system.

Schembri *et al, Clin. Chem.* 38: 1665-1670 teach a portable whole blood analyzer which utilizes a disposable rotor made of molded polymethylmethacrylate plastic which consists of many interlinked internal chambers and passages, the movement of fluid being controlled by a series of stop junctions, capillaries, and siphons managed through the use of centrifugal force. The outside edge of the rotor is imprinted with a bar code that contains the calibration factors.

European Patent Application No. EP0417305A and Patent Abstracts of Japan, Publication No. 03245049A, to Idemitsu Petrochemical Co., 20 March 1991, disclose a liquid sample analysis apparatus which includes a rotatable disk formed with a series of open grooves in its upper surface. The disk may further be formed with pre-encoded signals or formats required for the analysis while the apparatus further comprises reading means for reading the pre-encoded signals or formats.

U.S. Patent No. 3, 679, 367, issued 25 July 1972 to Negersmith discloses a centrifuge rotor using optics. The rotor can carry a capillary glass tube which acts as a capillary chamber and defines an S-shaped configuration or a J-shaped configuration with a terminal end that is openended to allow for overflow of excess liquid introduced into the capillary chamber.

U.S. Patent No. 4,940,527, issued 10 July 1990 to Kazlauskas discloses a disposable test cartridge for a centrifuge rotor which has a sample entry port and an electrolyte exit port as well as a waste chamber and one or more chambers containing calibrants.

The prior art discloses synthetic microchips for performing microanalytic and microsynthetic methods. One drawback in the prior art microanalytical methods and apparati has been the difficulty in designing systems for moving fluids on the microchips through channels and reservoirs having diameters in the 10-100μm range. Also, the devices disclosed in the prior art have required separate data analysis and storage media to be integrated into an instrument for performing the microanalysis, thereby unnecessarily increasing the complexity of the instruments designed to use the microchips, without a concomitant increase in the flexibility or usefulness of these machines.

There remains a need for a simple, flexible, reliable, rapid and economical microanalytic and microsynthetic reaction platform for performing biological, biochemical and chemical analyses and syntheses that can move fluids within the structural components of a microsystems platform. Such a platform should be able to move nanoliter-to-microliter amounts of fluid, including reagents and reactants, at rapid rates to effect the proper mixing of reaction components, removal of reaction side products, and isolation of desired reaction products and intermediates. There is also a need for an instrument for manipulating the microsystem platform to effect fluid movement, thermal control, reagent mixing, reactant detection, data acquisition, data analysis and data and systems interface with a user. Such devices are needed, in alternative embodiments, that are sophisticated (for professional, *e.g.,* hospital, use), easy to use (for consumer, *e.g.*, at-home monitoring, uses) and portable (for field, *e.g.,* environmental testing, use). Such devices also advantageously combine "wet" chemistry capabilities with information processing, storing and manipulating ability.

## SUMMARY OF THE INVENTION

The invention is defined in the accompanying claims.

This invention provides an integrated, microanalytical/microsynthetic system for performing a wide variety of biological, biochemical and chemical analyses on a microminiature scale. The invention provides apparatus and methods for performing such microscale processes on a microplatform, whereby fluid is moved on the platform in defined channels motivated by centripetal force arising from rotation of the platform.

The microanalytic/microsynthetic system comprises a combination of two elements. The first element is a microplatform that is a rotatable structure, most preferably a disk, the disk comprising sample, inlet ports, fluid microchannels, reagent reservoirs, reaction chambers, detection chambers and sample outlet ports. The disk is rotated at speeds from about 1-30,000 rpm for generating centripetal acceleration that enables fluid movement. The disks also comprise fluid inlet ports, air outlet ports and air displacement channels. The fluid inlet ports allow samples to enter the disk for processing and/or analysis. The air outlet ports and in particular the air displacement ports provide a means for fluids to displace air, thus ensuring uninhibited movement of fluids on the disk. Specific sites on the disk also preferably comprise elements that allow fluids to be analyzed, including thermal sources, light, particularly monochromatic light, sources, and acoustic sources, as well as detectors for each of these effectors. Alternatively, some or all of these elements can be contained on a second disk that is placed in optical or direct physical contact with the first.

The second element of the invention is a micromanipulation device that is a disk player/ reader device that controls the function of the disk. This device comprises mechanisms and motors that enable the disk to be loaded and spun. In addition, the device provides means for a user to operate the microsystems in the disk and access and analyze data, preferably using a keypad and computer display.

The invention provides methods and apparatus for the manipulation of samples consisting of fluids, cells and/ or particles containing or comprising an analyte. The microplatform disks of the invention comprise microsystems composed of, but no restricted to, sample input ports, microchannels, chambers, valves, heaters, chillers, electrophoretic and detection systems upon a disk. Movement of the sample is facilitated by the judicious incorporation of air holes and air displacement channels that allow air to be displaced but prevent fluid and/or particle loss upon acceleration.

The disk may incorporate micromachined mechanical, optical, and fluidic control structures (or "systems") on a substrate that is preferably made from plastic, silica, quartz, metal or ceramic. These structures are constructed on a sub-millimeter scale by photolithography, etching, stamping or other appropriate means.

Sample movement is controlled by centripetal or linear acceleration and the selective activation of valves on the disk.

Data resulting from processing and analysis is recorded on the disk surface using digital recording means. In additional preferred embodiments, read-only memory (ROM) on the disk comprises disk information, instructions, experimental protocols, data analysis and statistical methods that can be accessed by a user operating the disk.

The process of fluid transport by centripetal acceleration and the micromanipulation device that enables such processing have a wide range of applications in the synthesis and analysis of fluids and detection of analytes comprising a fluid, particularly a biological fluid. Chemical and biochemical reactions are performed in a reaction chamber on the disk by the selective opening of contiguous reagent chambers by means of capillary, mechanical or thermal valve mechanisms. The contents of those chambers are delivered to the reaction chamber with the application of centripetal acceleration. The product of the reaction can then be used as a reagent for subsequent reactions, interrogated by detection systems or recovered.

Certain preferred embodiments of the apparatus of the invention are described in greater detail in the following sections of this application and in the drawings.

## BRIEF DESCRIPTION OF THE DRAWINGS

Figure 1A (top view) and 1B (side view) illustrate the arrangement of reservoirs (**12,14,18,20**), valves (**13,15,17,19,21,23,25**) reaction chambers (**16,22,24**), ports (**11,32**) and air vents (**29,33,34,35**) in disks comprising the microplatforms of the invention. Figure 1C shows the arrangement of a multiplicity of microsystems on a disk. Figure 2A is a graph and Figure 2B is a schematic diagram of the arrangement of a channel on a disk of the invention as described with relation to Equation 5.

Figure 3A is a graph and Figure 3B is a schematic diagram of the arrangement of a channel on a disk of the invention as described with relation to Equations 12 and 13.

Figure 4A is a graph and Figure 4B is a schematic diagram of the arrangement of a channel on a disk of the invention as described with relation to Equation 14.

Figures 5A, 5B and 5C are graphs and Figure 5D is a schematic diagram of the arrangement of a channel on a disk of the invention as described with relation to Equation 15.

Figure 6 is a schematic diagram of a piezoelectric stack microvalve.

Figure 7 is a schematic diagram of a pneumatically-activated microvalve.

Figure 8 is a schematic diagram of a device to deliver pneumatic pressure to a revolving disk.

Figure 9 is a schematic diagram of a bimetallic microvalve.

Figure 10 is a schematic diagram of a pressure-balanced microvalve.

Figure 11 is a schematic diagram of a polymeric relaxation microvalve.

Figures 12A and 12B represent two different embodiments of fluorescence detectors of the invention.

Figures 13A, 13B and 13C are a schematic diagrams of a multiple loading device for the disk.

Figures 14A through 14F illustrate laser light-activated CD-ROM capability of the disk of the invention.

Figure 15 is a flow diagram of the processor control structure of a player/reader device of the invention.

Figure 16 is a schematic diagram of a transverse spectroscopic detection chamber. Figures 17A through 17E are schematic diagrams of the different structural and functional layers of a disk of the invention configured for DNA sequencing.

Figure 17F is a schematic diagram of basic zones and design formats for analytic disks.

Figure 17G is a schematic diagram of a disk configured as a home test diagnostic disk.

Figure 17H is a schematic diagram of a disk configured as a simplified immunocapacitance assay.

Figure 17I is a schematic diagram of a disk configured as a gas and particle disk.

Figure 17J is a schematic diagram of a hybrid disk comprising separately-assembled chips.

Figure 17K is a schematic diagram of a sample authorizing disk.

Figure 17L is a schematic diagram of a disk configured for pathological applications.

Figure 17M is a schematic diagram of a disk with removable assay layers.

Figure 17N is a schematic diagram of a disk for assaying aerosols.

Figure 17O is a schematic diagram of a disk for flow cytometry.

Figure 17P is a schematic diagram of a disk for microscopy applications.

Figure 17Q is a schematic diagram of a disk for immunoassay applications.

Figure 17R is a schematic diagram of a thin-layer chromatography disk.

Figure 18 is a schematic diagram of a disk configured for hematocrit determination.

Figure 19 is a schematic diagram of a disk configured for SPLITT fractionation of blood components.

Figure 20 is a schematic diagram of a disk configured as a DNA meltometer.

Figure 21 is a schematic diagram of a disk configured for DNA amplification.

Figure 22 is a schematic diagram of a disk configured for automated restriction enzyme digestion of DNA.

Figure 23 is a schematic diagram of a portion of a disk microsystem configured for DNA synthesis.

Figure 23B is a schematic diagram of a disk configured for a multiplicity of DNA oligonucleotide syntheses.

Figure 24 is a schematic diagram of a disk configured for DNA sequencing.

Figure 25 is a schematic diagram of a disk configured for iron assay.

Figure 26 is a schematic diagram of a disk configured for solid phase reaction.

Figure 27 is a schematic diagram of a disk configured for sample extraction,

Figure 28 is a schematic diagram of a disk configured for capillary electrophoresis.

Figure 29 is a schematic diagram of a transverse optical path in a microplatform.

Figure 30 is a block diagram of process flow in controlling informatics of the invention.

Figure 31 is a more detailed schematic diagram of controlling informatics of the invention.

Figure 32 is a more detailed schematic diagram of controlling informatics of the invention.

## DETAILED DESCRIPTION OF PREFERRED EMBODIMENTS

This invention provides a microplatform and a micromanipulation device for performing microanalytical and microsynthetic assays of biological, chemical, environmental and industrial samples. For the purposes of this invention, the term "sample" will be understood to encompass any chemical or particulate species of interest, either isolated or detected as a constituent of a more complex mixture, or synthesized from precursor species. The invention provides a combination of a microplatform that is a rotatable, analytic/synthetic microvolume assay platform (collectively referred to herein as a "disk") and a micromanipulation device for manipulating the platform to achieve fluid movement on the platform arising from centripetal force on the platform as result of rotation. The platform of the invention is preferably and advantageously a circular disk; however, any platform capable of being rotated to impart centripetal for a fluid on the platform is intended to fall within the scope of the invention.

The microplatforms of the invention (preferably and hereinafter collectively referred to as "disks"; for the purposes of this invention, the terms "microplatform", "microsystems platform" and "disk" are considered to be interchangeable), are provided to comprise one or a multiplicity of microsynthetic or microanalytic systems. Such microsynthetic or microanalytic systems in turn comprise combinations of related components as described in further detail herein that are operably interconnected to allow fluid flow between components upon rotation of the disk. These components can be fabricated as described below either integral to the disk or as modules attached to, placed upon, in contact with or embedded in the disk. The invention also comprises a micromanipulation device for manipulating the disks of the invention, wherein the disk is rotated within the device to provide centripetal force to effect fluid flow on the disk. Accordingly, the device provides means for rotating the disk at a controlled rotational velocity, for stopping and starting disk rotation, and advantageously for changing the direction of rotation of the disk. Both electromechanical means and control means, as further described herein, are provided as components of the devices of the invention. User interface means (such as a keypad and a display) are also provided.

The invention provides methods and apparatus for the manipulation of samples consisting of fluids, cells and/ or particles (generically termed "sample" herein) containing an analyte of interest. The platforms of the invention consist of systems comprising sample input ports, microchannels for fluid flow, reagent reservoirs, mixing chambers, reaction chambers, optical reading chambers, valves for controlling fluid flow between components, temperature control elements, separation channels, electrophoresis channels and electrodes, air outlet ports, sample outlet ports, product outlet ports, mixing means including magnetic, acoustic and mechanical mixers, an on-board power supply such as a battery or electromagnetic generator, liquid and dry reagents, and other components as described herein or known to the skilled artisan. The movement of the sample is facilitated by the judicious incorporation of air holes or air displacement channels that allow air to be displaced but prevent fluid and/or particle loss upon acceleration. Preferably, the disk incorporates microfabricated mechanical, optical, and fluidic control components on platforms made from, *for example,* plastic, silica, quartz, metal or ceramic. For the purposes of this invention, the term "microfabricated" refers to processes that allow production of these structures on the sub-millimeter scale. These processes include but are not restricted to photolithography, etching, stamping and other means that are familiar to those skilled in the art.

Fluid (including reagents, samples and other liquid components) movement is controlled by centripetal acceleration due to rotation of the platform, and by the selective activation of valves controlling the connections between the components of the microsystems of the platform. The magnitude of centripetal acceleration required for fluid to flow at a rate and under a pressure appropriate for a particular microsystem is determined by factors including but not limited to the effective radius of the platform, the position angle of the structures on the platform with respect to the direction of rotation and the speed of rotation of the platform.

Chemical and biochemical reactions are performed in a reaction chamber by the selective opening of microvalves controlling access to contiguous reagent reservoirs. Microvalves as described in more detail below include mechanical, electrical and thermal valve mechanisms, as well as capillary microvalves wherein fluid flow is controlled by the relationship between capillary forces and centripetal forces acting on the fluid. The contents of the reagent reservoirs, that are connected a reaction chamber through microchannels controlled by such microvalves, are delivered to the reaction chamber by the coincident rotation of the microplatform and opening of the appropriate microvalves. The amount of reagent delivered to a reaction chamber is controlled by the speed of rotation and the time during which the valve to the reagent reservoirs is open. Products of the reaction performed in the reaction chamber are similarly removed from the reaction chamber to an analytical array, a second reaction chamber or a product outlet port by the controlled opening of micro valves in the reaction chamber.

Analytical arrays constituting components of the microplatforms of the invention include detection systems for detecting, monitoring, quantitating or analyzing reaction course, products or side-products. Detection systems useful in the fabrication and use of the microplatforms of the invention include, but are not limited to, fluorescent, chemiluminescent, colorimetric, electrochemical and radioactivity detecting means. Optionally, the detection system can be integral to the platform, comprise a component of the device manipulating the platform, or both.

Thus, the microplatform and micromanipulation device provided by the invention produce analytic or synthetic data to be processed. Data processing is accomplished either by a processor and memory module on the disk, by the device microprocessor and memory, or by an out board computer connected to the micromanipulation device. Removable media for data retrieval and storage is provided either by the disk itself or by the device, using computer diskette, tape, or optical media. Alternatively and advantageously, data is written on the microplatform using CD-read/write technologies and conventional optical data storage systems In such embodiments, data is written to the microplatform on the underside of the platform, opposite to the "wet" chemistry side holding the various microsystem components disclosed herein.

The physical parameters of the microplatforms of the invention are widely variable. When provided as a disk, the disk radius ranges from 1-25cm, and disk thickness ranges from 0.1mm to 10cm, more preferably 0.1 to 100mm. Preferred embodiments that are most advantageous for manufacturing and operation of the disks of the invention have dimensions within one or more of four pre-existing formats: (1) 3-inch compact disk (CD), having a radius of about 3.8cm and thickness of about 1mm: (2) 5-inch CD, having a radius of about 6cm and a thickness of 1mm; (3) 8-inch CDV (commercially termed a "Laservision" disk), having a radius of 10cm and a thickness of 2mm; and (4) 12-inch CDV disk, having a radius of 15cm and a thickness of 2mm.

Microchannel and reservoir sizes are optimally determined by specific applications and by the amount of reagent and reagent delivery rates required for each particular embodiment of the microanalytic and micro synthetic methods of the invention. For microanalytical applications, for example, disk dimensions of a 5-in CD (6cm x 1mm) are preferred, allowing reagent reservoirs to contain up to 0.5mL (close to the actual displaced by the disk). Microchannel and reservoir shapes can be trapezoid, circular or other geometric shapes as required. Microchannels preferably are embedded in a microsystem platform having a thickness of about 0.1 to 100mm, wherein the cross-sectional dimension of the the microchannels across the thickness dimention of the platform is less than $500\mu m$ and from 1 to 90 percent of said cross-sectional dimension of the platform. Reagent reservoirs, reaction chambers, detection chambers and sample inlet and outlet ports are embedded in a microsystem platform having a thickness of about 0.1 to 100mm, wherein the cross-sectional dimension of the the microchannels across the thickness dimention of the platform is from 1 to 75 percent of said cross-sectional dimension of the platform.

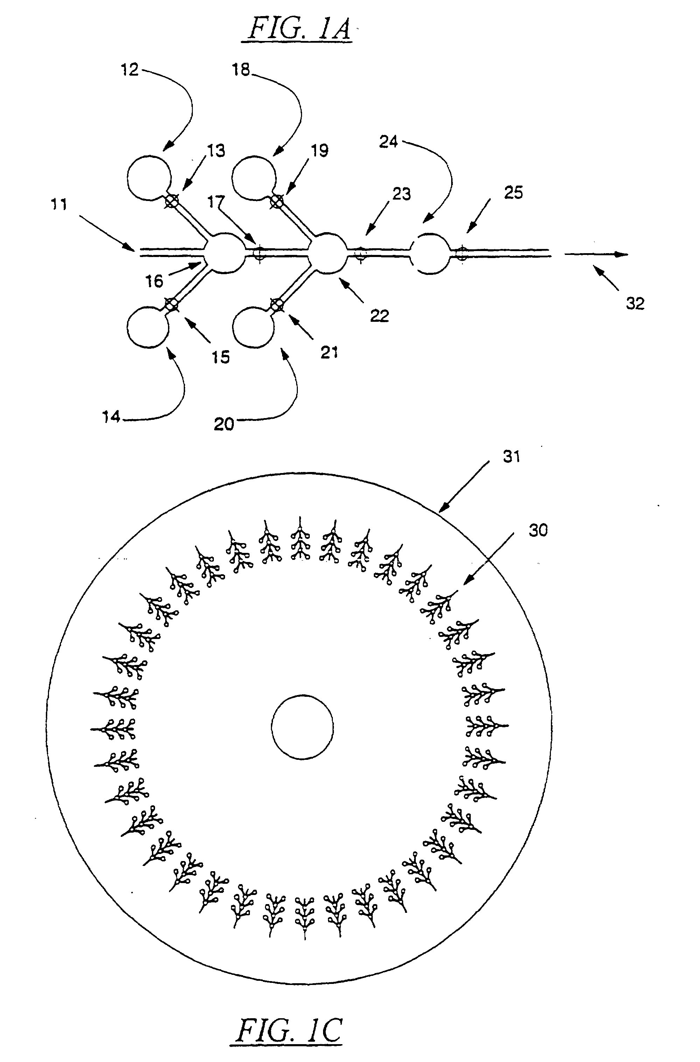

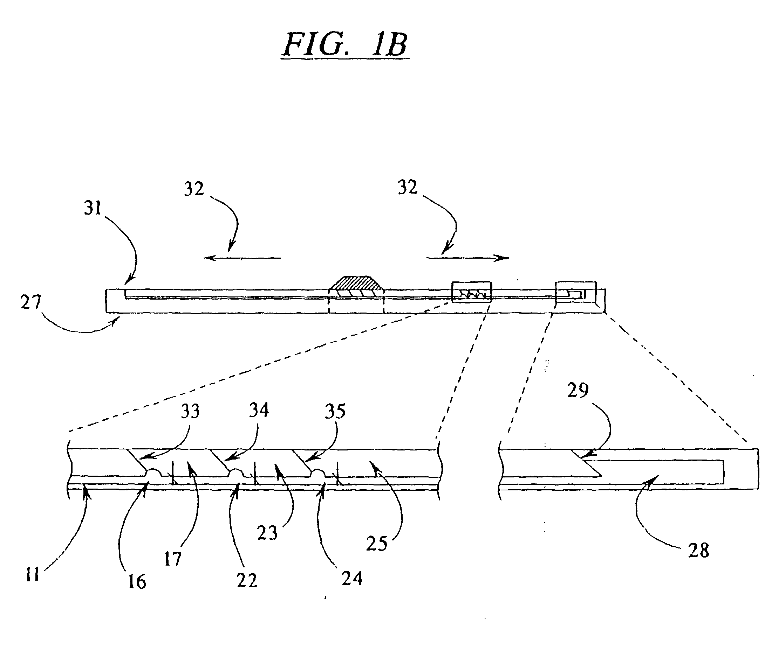

Input and output (entry and exit) ports are components of the microplatfoms of the invention that are used for the introduction of removal of a variety of fluid components. Entry ports are provided to allow samples and reagents to be placed on or injected onto the disk; these types of ports are generally located towards the center of the disk. Exit ports are provided to allow air to escape, advantageously into an on-disk "muffler" or "baffle" system, to enable uninhibited fluid movement on the disk. Also included in air handling systems on the disk are air displacement channels, whereby the movement of fluids displaces air through channels that connect to the fluid-containing microchannels retrograde to the direction of movement of the fluid, thereby providing a positive pressure to further motivate movement of the fluid. Exit ports are also provided to allow products to be removed from the disk. Port shape and design vary according specific applications. For example, sample input ports are designed, *inter alia,* to allow capillary action to efficiently draw the sample into the disk. In addition, ports can be configured to enable automated sample/reagent loading or product removal. Entry and exit ports are most advantageously provided in arrays, whereby multiple samples are applied to the disk using a specifically-designed administration tool. Similar tools are useful designed to effect product removal from the microplatform. Representative arrangements of sample ports, air vents, reagent reservoirs, reaction chambers and microvalves are shown in Figures 1A through 1C.

Operative and optimal placement of the various disk components and elements depend on the dynamics of fluid movement in relation to centripetal forces. Centripetal force is a function of platform radius, disk rotation speed and fluid density. Certain functional parameters relevant to the platform microsystems of this invention are understood in terms of the following equations. These should represent limits of system performance, because they assume both viscous and non-viscous (turbulent) losses for fully-developed fluid flow.

The driving force for fluid motion or creating fluid pressures is the force on matter which results from centripetal acceleration. A device may rotate at an angular rate of f in revolutions/see and angular frequency

$$\omega = 2\pi f \qquad (1)$$

The centripetal acceleration (or acceleration oriented along the radius at a radial distance R from the center of the uniformly-rotating disk) is

$$a_c = \omega^2 R. \qquad (2)$$

A mass m in such uniform circular motion is subject to a centripetal force

$$F_c = ma_c = m\omega^2 R \qquad (3)$$

which is directed inward along the radius to the center of rotation. If the mass is held fixed at this radius, the device causing rotation supplies this force; this is the origin of the static pressure in liquid columns discussed below. If the mass is placed on top of a trap-door above a radially-oriented tube, and the trap-door opened, the inertia of the mass will cause it to accelerate down the tube; this is the basis for driving fluids radially outward on a rotating disk.

Rotation may create a static pressure in a non-flowing fluid. Assume a column of liquid extending from an inner radius $R_0$. The tube may be along the radius or inclined at an angle to the radius. Let the pressure at position $R_0$ be defined as $P_0$. which is for example atmospheric pressure. The excess pressure due to rotation of the liquid at Position R such that $R_0 < R$ is found by integrating the centripetal force per unit area for liquid of density p from position $R_0$ to R:

$$P - P_0 = \int \rho a_c = \rho \omega^2/2 \times (R^2 - R_0^2) \qquad (4)$$

If the tube is filled, extending from the center, then this pressure is

$$P - P_0 = (2.834 \times 10^{-4}) \rho f^2 R^2 \qquad (5)$$

in pounds per square inch (psi) where R=radial position in cm, $\rho$=density in $gm/cm^3$, and f=frequency in revolutions/sec. Thus, the pressure (or the amount of centripetal force on a fluid) varies directly with the density of the fluid, and as the square of the radial position from the center of rotation as well as the square of the frequency of rotation.

To determine the velocity of liquid in motion in channels on a rotating disk, the equation of motion for the fluid must be solved. An element of fluid of radius a and length dR filling the circular channel has a mass dm subject to acceleration:

$$dm = \pi \rho a^2 \, dR \qquad (6)$$

The equation of motion for this fluid element is force=(mass) X (acceleration). The forces are centripetal forces, capillary forces due to differences in interfacial energies between the fluid and vapor and fluid and solid surfaces, and dissipative forces due to the viscosity of the liquid and nonuniformity of flow. Capillary forces are ignored; it is understood that centripetal force and/or external pressure may need to be applied to force liquid into channels which are not wetted. As an over-estimate of these dissipative forces, both the force for fully-developed laminar flow of a Newtonian fluid ($F_L$) and that due to non-uniform flow ($F_D$) are included:

$$F = ma$$

$$F_c + F_L + F_D = dma_R \qquad (7)$$

$$F_c + F_L + F_D = (\rho\pi a^2 dR)a_R$$

where $a_R$ is the acceleration of the fluid mass element along the radius and

$$F_c = (\rho\pi a^2 dR)\omega^2 R$$

$$F_L = -(8\mu\pi a^2 dR)u \qquad (8)$$

$$F_D = -(2\rho\pi a^2 dR) \, u^2$$

where $\mu$ is the viscosity and $u$ is the radial velocity of the fluid. These last two expressions are standard-mechanics expressions for fully-developed and completely undeveloped laminar flow, such as at channel entrances/exits or at the ends of a flowing droplet. Also note that for tubes or channels inclined at an angle $\theta$ with respect to the radius $F_c$ would be replaced by ($F_c$) X cos $\theta$. The final equation becomes

$$(\rho\pi a^2 dR)\omega^2 R - (8\mu\pi dR)u - (2\rho\pi a^2 \, u^2 \, dR) = (\rho\pi a^2 dR) \, (du \, / \, dt) \qquad (9)$$

where the radial acceleration of the fluid is defined by $a_R$ - $(du \, / \, dt)$. This is a differential equation for the fluid flow velocity.

This equation is solved for specific examples. Consider a droplet of fluid of length L moving in a radial channel of greater length than the droplet.

Because the fluid in the droplet all moves at the same velocity, $dR$ may be replaced by $L$ and $R$ by the average position of the droplet, $\langle R \rangle = (R+L/2)$.
Dividing out common factors:

$$(\omega^2 (R+L/2) / 2) - (8\mu / \rho a^2)u - 2(u^2 / L) = (du / dt) \tag{10}$$

This equation must be solved numerically. An approximation may be made which has been justified through comparison with numerical solutions. It consists of this: the negative terms on the left-hand-side almost entirely cancel the positive term. Then the right-hand-side can be set to 0 and a solution can be made to the resultant equation for the "terminal velocity" at position R, $u_0$

$$(\omega^2 (R+L/2) / 2) - (8\mu / \rho a^2)u_0 - {}_2(u_0{}^2 / L) = 0 \tag{11}$$

This is a quadratic equation which has the solution

$$u_0 = - (B + \sqrt{B^2 + 4AC})/2A \tag{12}$$

with

$$A = L/2$$

$$B = 8\mu / \rho a^2 \tag{13}$$

$$C = (\omega^2 (R+L/2) / 2)$$

In conventional units these become $A=2/L$, $B=320\mu /\rho D^2$ and $C=(19.74)f (2R+L)$ with $u_0$ = fluid velocity in cm/sec; L=droplet length in cm; $\mu$ = viscosity in poise; $\rho$=fluid density in $gm/cm^3$; D = 2a = tube diameter in cm; and R = radial position of the fluid droplet in cm. As described, this expression gives the approximate velocity of a droplet of fluid in a tubular channel, the volume of the droplet resulting in droplet length being shorter than the channel length. This estimate assumes both viscous and non-viscous losses. The velocity of a fluid droplet will increase with increasing density and droplet volume (length), and decrease with increased viscosity. The velocity will increase with increased channel diameter, rotational velocity, and radial position.

Fluid flow velocity in a filled channel connecting a full chamber at position $R_0$ and receiving reservoir at position $R_1$ is calculated by defining L in equation (11) and subsequent equations as the channel length, $L= R_1 - R_0$. Then equation (13) with the definitions following equation (13) are used to calculate the flow velocity in the filled chamber as a function of radius.

The rate of fluid-flow is the product of velocity and channel area:

$$Q = u_0 \pi a^2 = u_0 \pi D^2 / 4 \tag{14}$$

where $Q$ = flow in mL/sec; $u_0$ = velocity in cm/sec (calculated from equations 12 and 13); and $D$ = tube diameter in cm.

The time required to transfer a volume V from a reservoir to a receptacle through a tube or channel of length L depends on whether V is such that the tube is filled (length of a "droplet" of volume V in the tube would be longer than the tube itself) or unfilled by volume V. In the former case, this time is approximately the volume V of the fluid divided by the rate of flow Q; in the latter case it is approximately this calculated time multiplied by the ratio of the tube length to the resultant droplet length:

$$Dt = V / Q, \text{ if } L \leq (4V / \pi D^2) \tag{15}$$

$$Dt = (V / Q) \times (4\pi D^2 L/4V), \text{ if } L > (4V / \pi D^2)$$

wherein $Dt$ is the same time in seconds for fluid of volume $V$ in mL flowing at rate $Q$ in mL/sec to flow from a filled reservoir to a receptacle through a tube of length $L$ and diameter $D$ in cm. The rate of flow $Q$ is calculated from eq. (14) and by extension equations (12) and (13) and the definitions of the parameters following equation (13). The time $Dt$ increases with increasing volume transferred and decreases with increasing flow-rate.

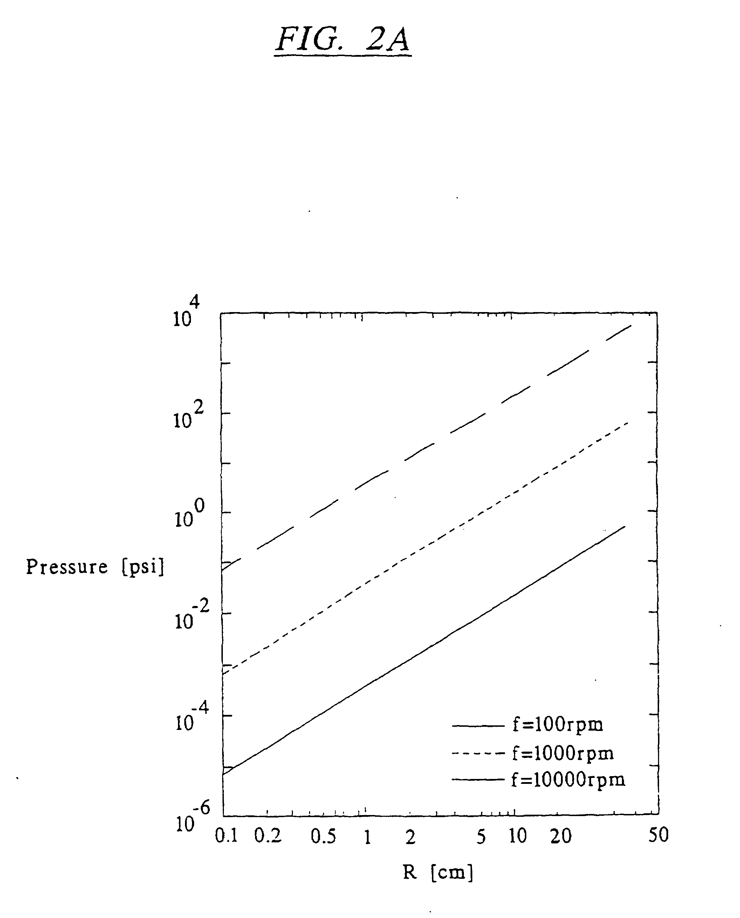

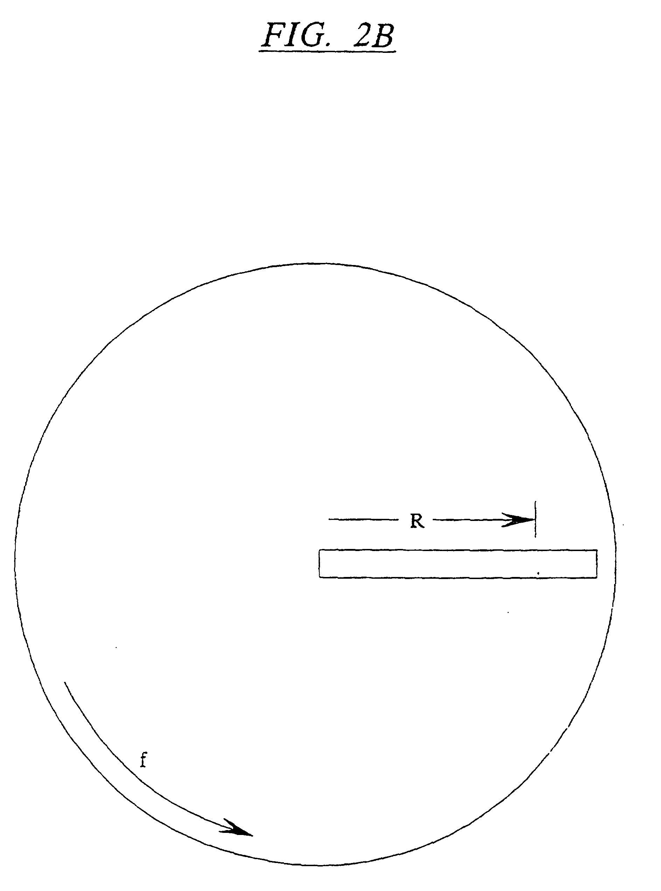

Fluid characteristics such as pressure and velocity are related to physical parameters of the disk, such as disk radius and speed of rotation, as described above. These relationships are illustrated in Figures 2-5, derived from the above equations for water at room temperature, with $p = 1$ gm / $cm^3$ and $\mu = 0.001$ poise. These figures delineate the most relevant parameters of fluid movement on a rotating disk.

Figure 2A illustrates the relationship between static pressure in a fluid-filled tube 30cm in length as a function of radial distance (R) and rotation rate (f), calculated from Equation 5. The arrangement of the tube on a rotating disk is shown in Figure 2B.It can be seen that pressures of between 0 and 10,000 psi (between 0 and 6894.76 kPa) can be generated in the tube at rotational speeds of 0 to 10,000 rpm. Pressures of this magnitude are conventionally used, for example, to drive high pressure liquid chromatography (HPLC).

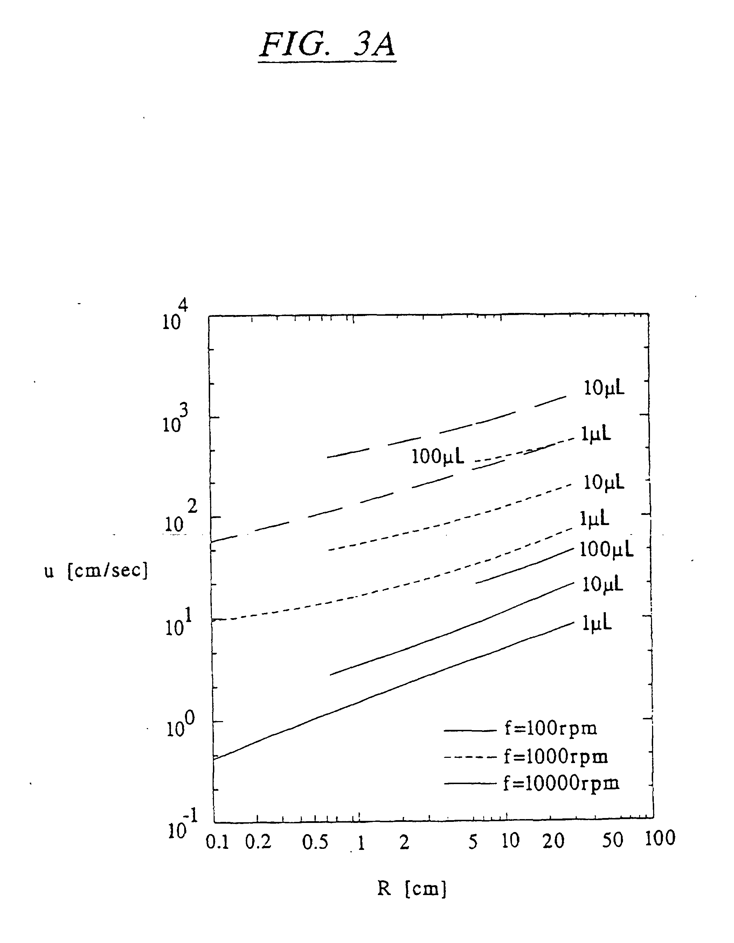

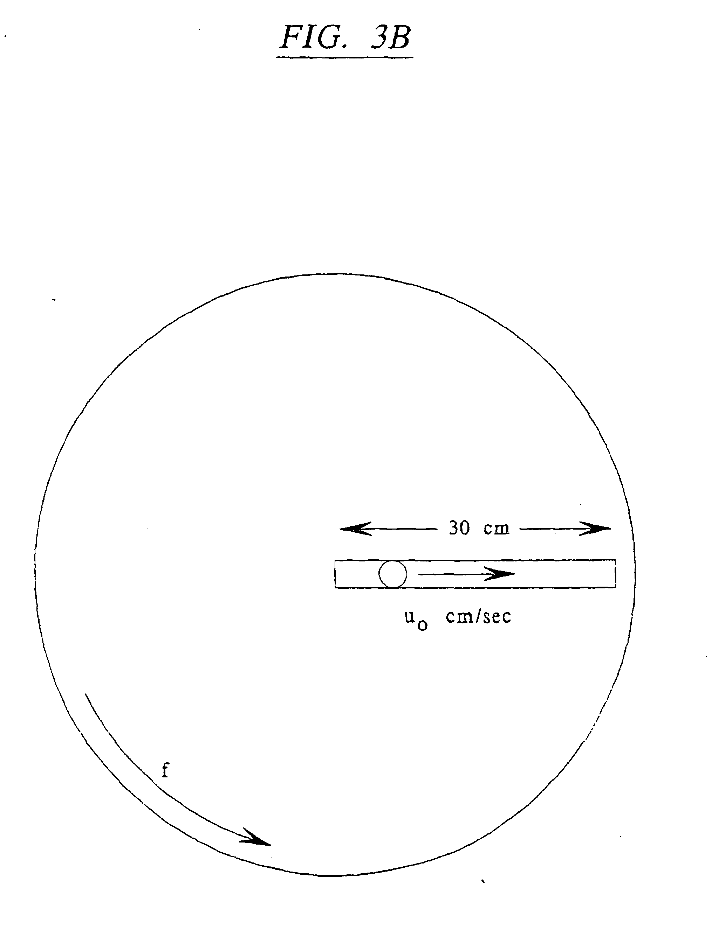

Figure 3A shows the radial velocity of droplets having volume of 1,10 and 100μL droplets moving in an empty, 30cm long tube with a diameter of 1mm, calculated from Equations 12 and 13. The tube is aligned to extend along the radius of the disk from the center, and the disk is rotated at speeds of 100, 1,000 or 10,000 rpm. The arrangement of the tube on a rotating disk is shown in Figure 3B. These velocities may be used to calculate the transfer time for fluid droplets. For example, a 1μL droplet flows at approximately 20cm/sec when at a position 2cm from the center of a disk rotating at 1,000 rpm. Hence, the time to flow through a 1cm tube can be calculated to be about 0.05 seconds. (For tubes oriented non-radially at an angle of 45° to the direction of rotation, the velocity drops by a factor of 30%.)

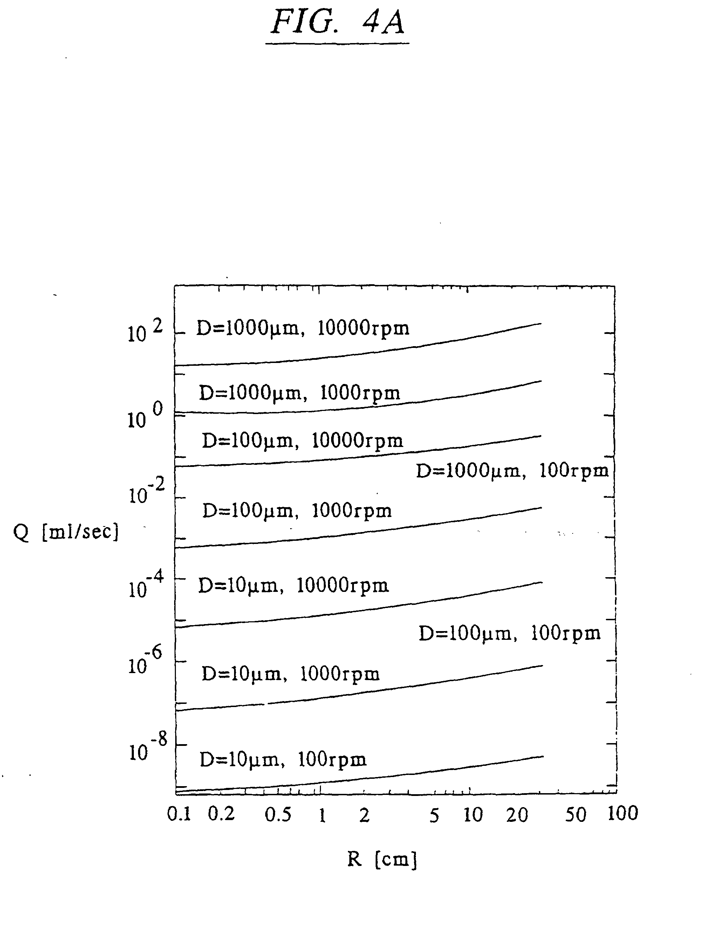

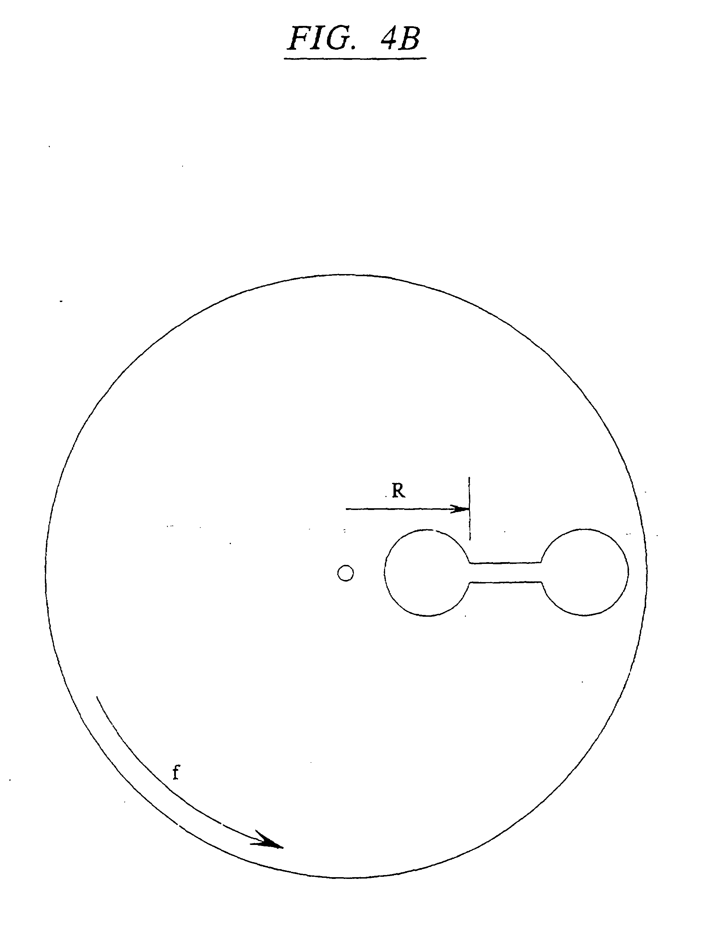

Figure 4A illustrates flow rates in a 5 cm fluid-filled tube of different diameters. The tubes are each placed on a rotating disk and connects two radially oriented reservoirs, shown in Figure 4B. According to Equation 14, flow rates are a function of radial position of the tube (which vary in this example from 2-30cm), the tube diameter (10μm, 100μm, or 1,000μm), and rotation frequency (100, 1,000 or 10,000 rpm). (As above, for tubes with a non-radial orientation of 45°, the velocity drops by a factor of 30%). Droplet velocities shown in Figure 3A were calculated by Equation 3 and flow rates determined using Equation 4.

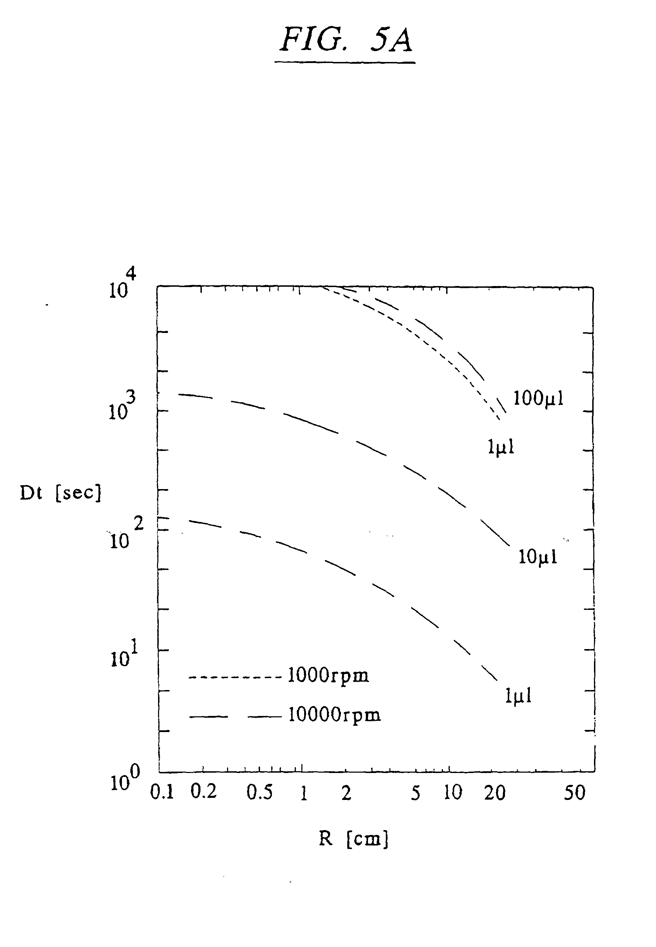

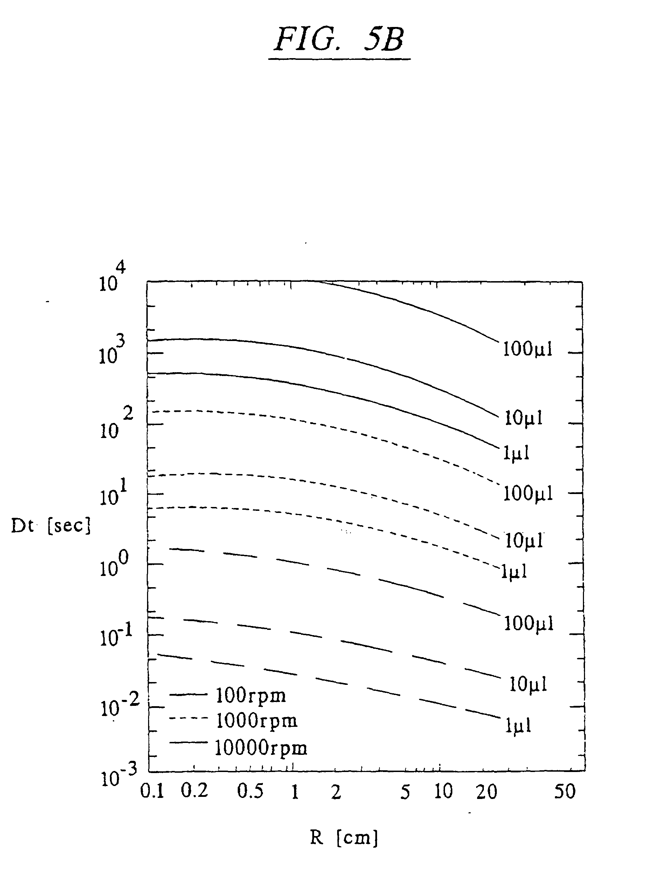

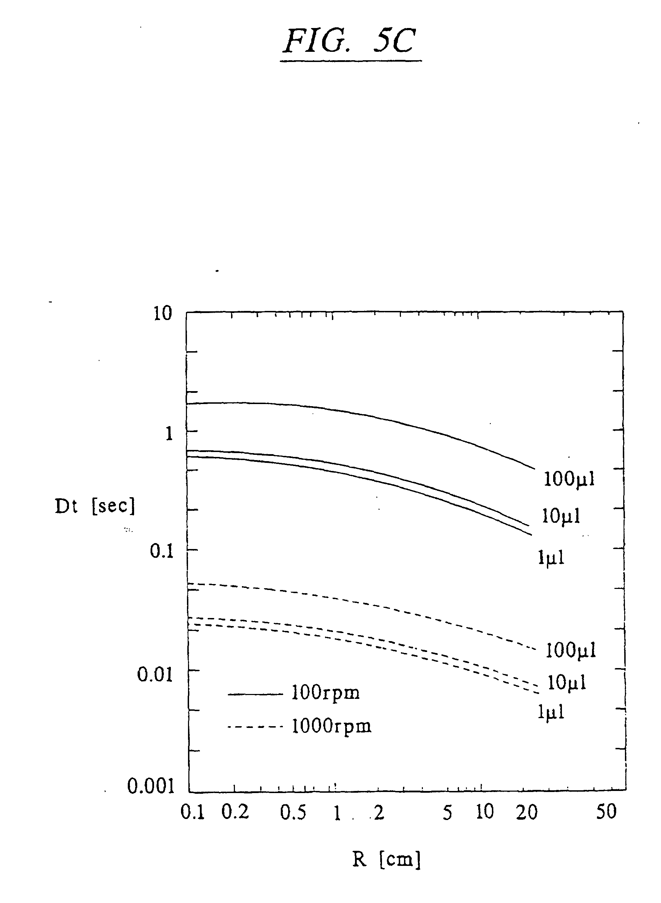

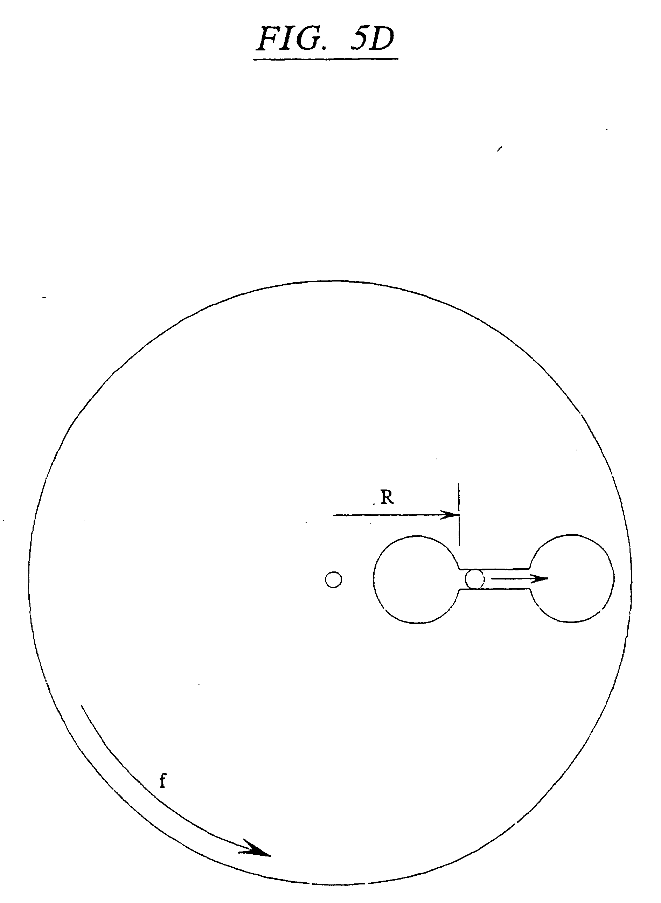

In Figures 5A, 5B and 5C, the time required to transfer 1, 10, and 100μL droplets, respectively, through a 5cm tube is shown. The tube connects two radially oriented reservoirs as illustrated in Figure 5D. Transfer times are a function of radial position of the tube (o-30cm), tube diameter (10μm, 100μm, or 1,000μm), and rotation frequency (100, 1,000 or 10,000 rpm). The curves shown in Figures 5A, 5B and 5C were calculated using Equation 15.

Taken together, these formulate and graphs describe the interrelationship of disk radii and rotation speeds, channel lengths and diameters, and fluid properties such as viscosity and density in determining fluid velocities and flow rates on the disk. The assumptions behind these derivations include viscous losses due to Poiseuille (non-turbulent) flow, with the addition of losses due to non-uniform flow of droplets and at tube inlet and outlet ports. These formulae and graphs provide lower limits for velocities and flow rates. Fluid velocities can range from less than 1 cm/sec to more than 1,000cm/sec, and fluid flow rates from less than 1pL/sec to tens of mL/sec for rotation rates ranging from 1 to 30,000 rpm. By combining channel diameters and positions on the disk, it is possible to carry out fluid transfer over a wide range of time scales, from milliseconds to hours and tens of hours for various processes.

## Disk Coatings and Composition

Microplatforms such as disks and the components comprising such platforms are advantageously provided having a variety of composition and surface coatings appropriate for a particular application among the wide range of applications disclosed herein. Disk composition will be a function of structural requirements, manufacturing processes, and reagent compatibility/chemical resistance properties. Specifically, disks are provided that are made from inorganic crystalline or amorphous materials, e.g. silicon, silica, quartz, metals, or from organic materials such as plastics, for example, poly(methyl methacrylate) (PMMA), acetonitrile-butadiene-styrene (ABS), polycarbonate, polyethylene, polystyrene, , polyolefins, polypropylene and metallocene. These may be used with unmodified or modified surfaces as described below.

One important structural consideration in the fabrication of the microsystems disks of the invention is mechanical failure due to stress during use. Failure mechanisms for disks rotated at high velocities include fracture, which can arise as the result of tensile loading, or due to cracking and crazing, as described on Hertzberg (1989, *Deformation and Fracture Mechanics of Engineerirrg Materials.* 3rd edition, Wiley & Sons: New York). These failures occur when the stress (defined as the load per unit area) due to rotation of the disk exceeds a critical value characteristic of the material used to make the disk. The "load" at any point in the disk is the force of tension due to rotation; *i.e.*, at a given radius on the disk, the overall load is the centripetal force necessary to keep the material at larger radii moving circularly; the load/area or stress is then this force divided by the total area of the disk ($2\pi r$ x the thickness of the disk). The critical value of stress at which a material will fail is termed the yield stress, and it depends on the cohesive energy binding the material together and the presence of defects in the material (such as crystalline defects in silicon or plastic substrate material). A defect-free material can be torn apart, whereas small defects will propagate through cracking or "crazing" (*i.e.,* plastic deformation and failure of a formerly glassy plastic). For example, the yield strength of commercial silicon permits a 30cm disk to be spun at 10,000 rpm without mechanical failure when the diameter of internal channels and chambers is less than approximately 80% of the total thickness of the disk. In disks made of plastics, stresses on the disk are reduced in general due to the lower density of the plastic (which reduces the load/unit area). However, the yield strengths are also smaller by about two orders of magnitude than in silicon (as described in greater detail in Luis & Yannis, 1992, *Computational Modeling of Polymers,* (Bureitz, ed.), Marcel Dekker: New York). One solution to this problem is provided either by spinning a plastic 30cm disk at a slower speed (such as 1,000 rpm), or increasing the size of the disk radius (such as using a 4cm plastic disk for applications requiring 10,000 rpm rotation speeds). Thus, material choice specific for a particular application is sufficient to accommodate disk composition-related constraints on disk functional properties and characteristics.

Disk material in contact with fluids must also be resistant to degradation by reagent solutions (such as acetonitrile, polyacrylamide, high- or low-pH buffers) under rotational stress, upon heating and cooling, and in response to illumination with high-intensity ultraviolet or visible light (occurring, *inter alia,* with the use of certain detection means as described below). In addition, the surfaces presented to reagents and reaction mixtures (such as microchannels, reservoirs and reaction chambers) must have desirable surface properties appropriate for each application. Silicon, silica, and quartz are especially robust materials as substrates for microplatform fabrication. Silicon and its oxides (essentially silica) are chemically attacked only by some peroxides (such as a mixture of hydrogen peroxide plus sulfuric acid), hydroxides (such as KOH), hydrofluoric acid (HF), either alone or in combination with alkali-based nitrates, and various perfluorinated solvents (like $SF_6$) *see* Iler, 1979, *The Chemistry of Silica,* Wiley & Sons: New York; *Properties of Silicon,* Xth ed., INSPEC:, London, 1988). Silicon-based substrates are chemically inert to aliphatic and aromatic hydrocarbons (such as tetrahydrofuran, toluene, and the like), and are substantially inert when exposed to water and neutral aqueous solutions.

A wide variety of polymer-based (plastics) substrates are suitable for fabricating microsystems platforms of the invention. The most chemically-resistant polymer, poly(tetrafluoroethylene; PTFE), is not melt-processible but may be easily machined. PTFE is virtually chemically inert and can be used in most applications utilizing strong acids, bases, alkalis, halogenated solvents, or other strong chemical reagents. Other fluoropolymers (such as FEP, PFA) are more easily processed than PTFE and retain most of PTFE's chemical resistance. More easily-processed materials may be chosen for selective resistance: for example, although polyimides are highly resistant to alcohols, alkalis, aliphatic hydrocarbons, and bases (*e.g.*, NaOH), their resistance to partially-halogenated solvents (*e.g.* dichlorobenzene) is poor. Poly (vinyl chloride) is strongly resistant to oxidizing acids and aliphatic hydrocarbons, while its resistance to aromatic compounds is poor. In addition, many materials that are not highly-resistant to concentrated applications of certain chemicals provide sufficient resistance to dilute solutions or provide sufficient resistance for single-use devices (e.g., polyamides and polyimides may be used with dilute solutions of certain acids such as acetic acid and hydrochloric acid). Most polymeric materials are resistant to water.

Specific chemical/polymer combinations include: formamide, lutidine, and acetonitrile with non-aromatic, non-polar polymers (polypropylene, polyethylene); dichloromethane with polycarbonates and aromatic polymers (polystyrene); ethanolamine and dimethyl sulfoxide with aliphatic and non-aromatic polymers (poly(methyl methacrylates), polyimides, polyamides). Fluoropolymers are resistant to all of the above chemical agents. Other solvents and reagents of interest, including pyridine, tetrazole, trichloracetic acid, iodine, acetic anhydride, N-methylpyrrolidine, N,N-diethylpropylethylamine and piperidine, are suitable for use with fluoropolymers and some solvent resistant polymers, such as PVC (*Encyclopedia of Polymer Science and Technology,* 2nd ed., v. 3, pp 421-430, X ed., John Wiley & Sons, New York, 1989). A small set of such materials provides sufficient flexibility for virtually any application.

The surface properties of these materials may be modified for specific applications. For example, appropriate surface-modification can either encourage or suppress cell and/or protein absorption. Surface modification can be achieved by silanization, ion implantation and chemical treatment with inert-gas plasmas (*i.e.,* gases through which electrical currents are passed to create ionization). A strong correlation has been established between water contact angle and cell adsorption, with hydrophilic surfaces showing significantly less cell adsorption than hydrophobic surfaces (*see* Ikada, 1994, *Biomaterials* 15: 725). Silicon, silica, and quartz present an inherently high-energy, hydrophilic surface. Alteration of surface properties is attained through hydroxylation (achieved by NaOH treatment at high temperatures) or silanization. Silanes and siloxanes are particularly appropriate for increasing the hydrophilicity of an otherwise hydrophobic surface. These compounds consist of one or several reactive head-groups which bond (chemically or through hydrogen-bonding) to a substrate, for example, a core region of alkane. These compounds also provide a route for more sophisticated alteration of surface properties (such as derivation with functional groups to obtain the surface properties of interest). A wide variety of such functionalities can be introduced at a surface, including vinyl, phenyl, methylene and methoxy groups, as well as surfaces providing mixed functionalities. These functional groups not only change gross properties like liquid contact angle, but provide sites for preferential adsorption of molecules, either *per se* or as a result of further conjugation of specific binding moieties such as peptides, antibodies or the like. Silation is most often accomplished through immersion. in aqueous solution at slightly-elevated temperatures. The chemical resistance of silane and siloxane coatings is determined by the nature of bonding within the chemisorbed molecule (Arkles, 1977, *Chemtech* 7: 125). It should be noted that such properties as hydrophobicity are maintained for significant periods when organosilanes are in contact with quite corrosive acids, implying that single-use or limited-use applications in these environments are possible.

Plastic-based disks can also be readily treated to achieve the required surface properties. Inert-gas or reactive-gas plasmas are commonly used to alter surface energies through the formation of surface complexes, for example, hydroxyl-rich surfaces for increased hydrophilicity, or perfluorinated surfaces for increased hydrophobicity. Surface graft polymerization is a technique used to graft polymers or oligomers with the desired surface properties to a substrate polymer chosen for its bulk processability and manufacturing properties, such as a plastic. Commercial methods for initiating graft polymerization include gamma radiation, laser radiation, thermal or mechanical processing, photochemical processes, plasma, and wet chemical processes (further discussed in *Encyclopedia of Polymer Science and Technology,* 2nd ed., (Supplement), Wiley & Sons: New York, 1989, pp 675-689). Chemical modification of polymer surfaces (and appropriate polymers) includes oxidations (polyethylenes), reductions (fluoropolymers), sulfonations, dehydrohalogenations (dehydrofluorination of poly (vinylidene fluoride), and hydrolyses. While the chemical nature of the surface is altered through chemical modification, mechanical properties, durability and chemical resistance are primarily a function of the substrate plastic. For example, surface grafting of poly(ethylene glycol) (PEG) onto polyethylene yields a surface that is both hydrophilic (unlike polyethylene) and resistant to water (PEG is itself soluble in water, while polyethylene is not). Finally, silation of organic polymer surfaces can also be performed, providing a wide variety of surface energy/chemistry combinations.

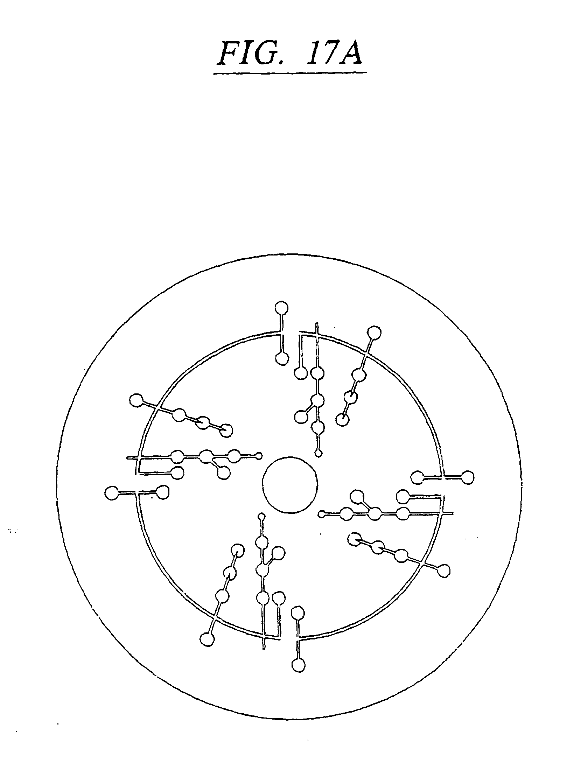

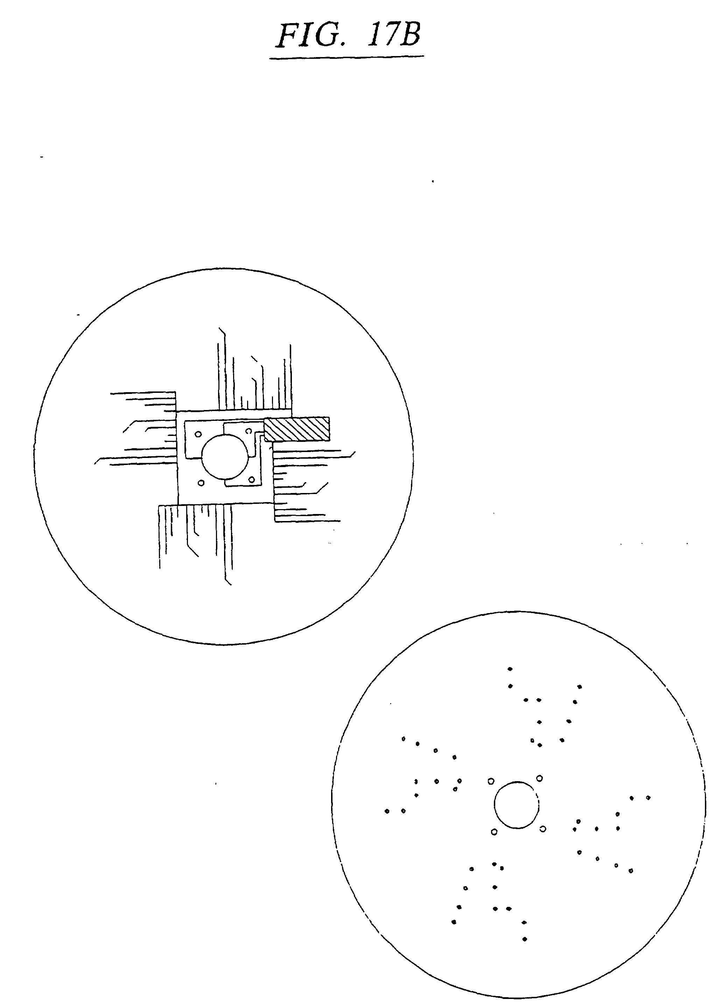

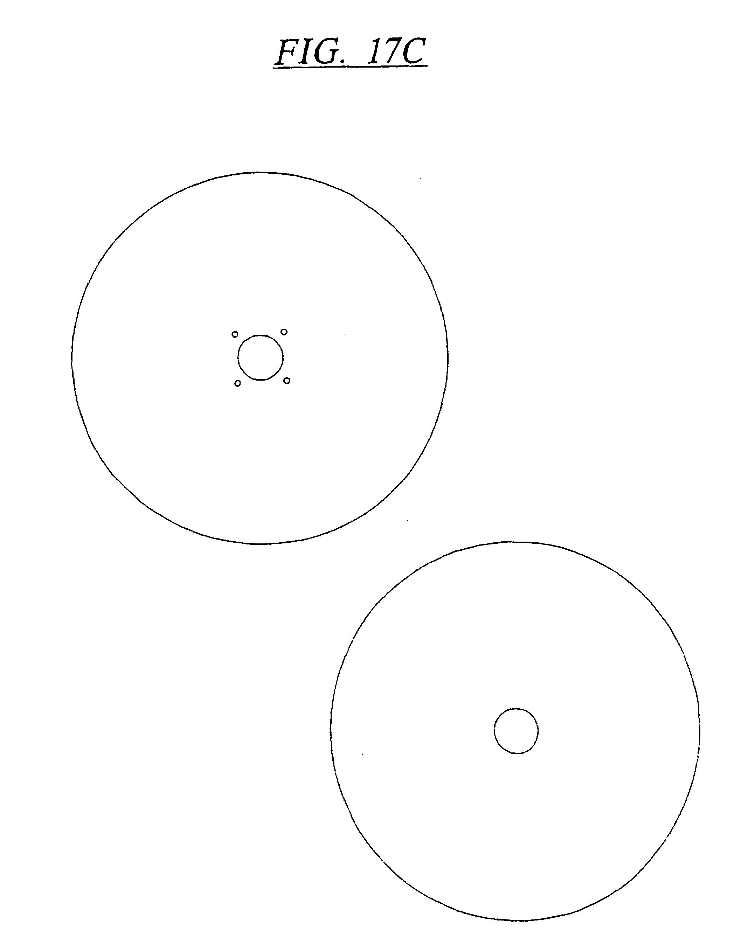

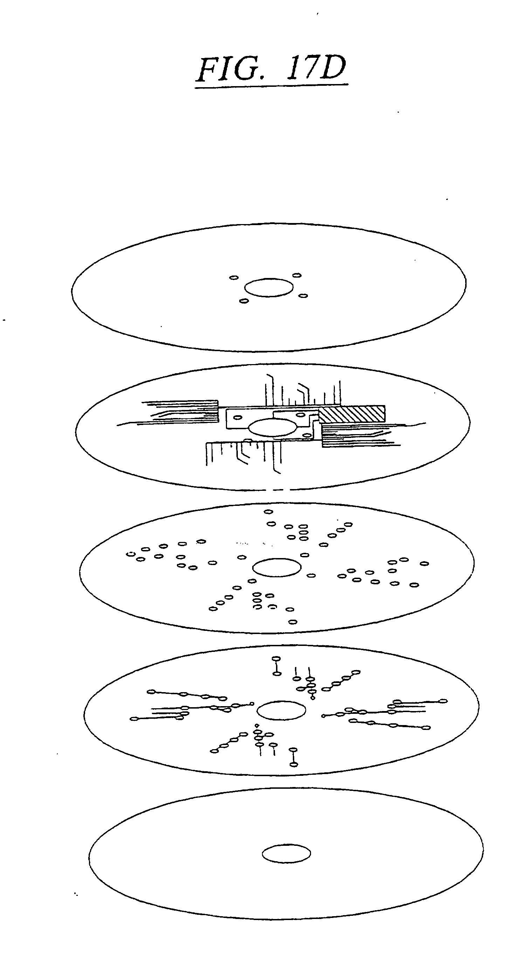

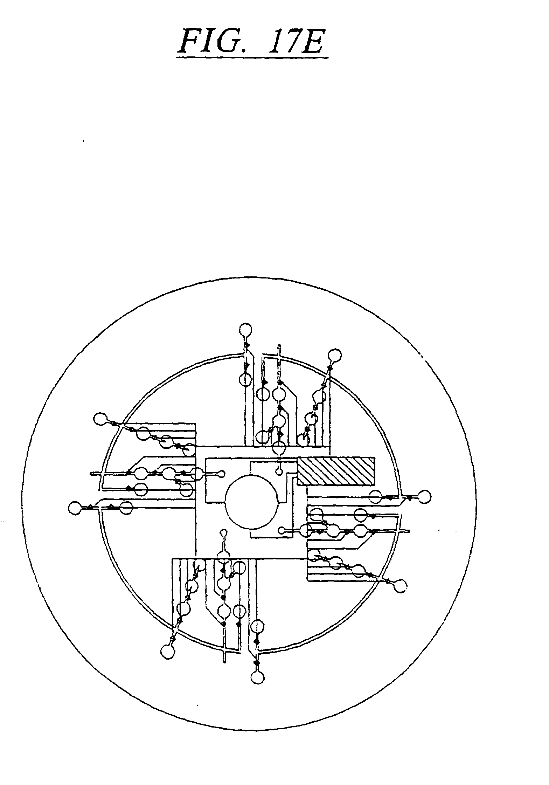

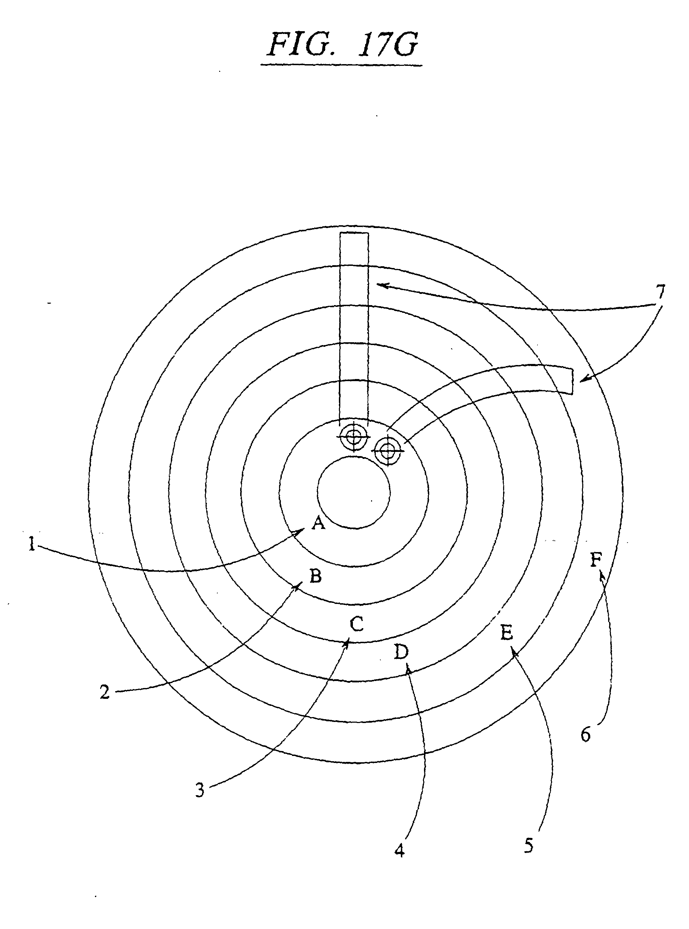

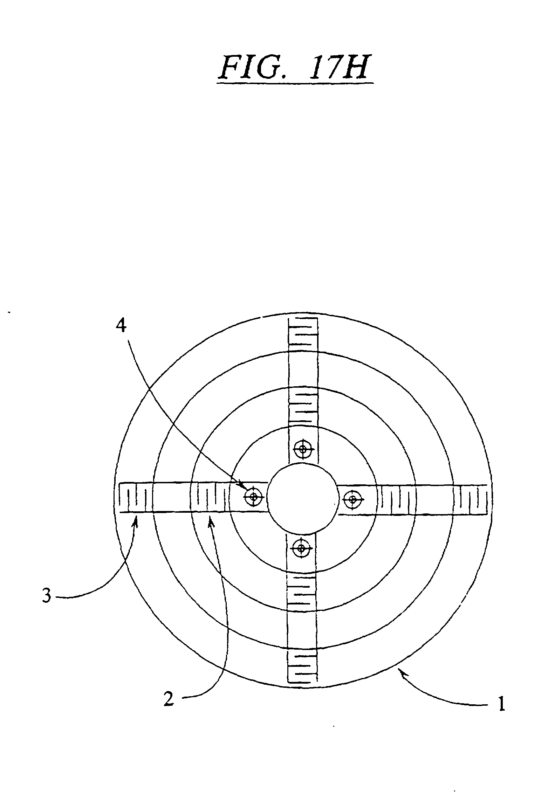

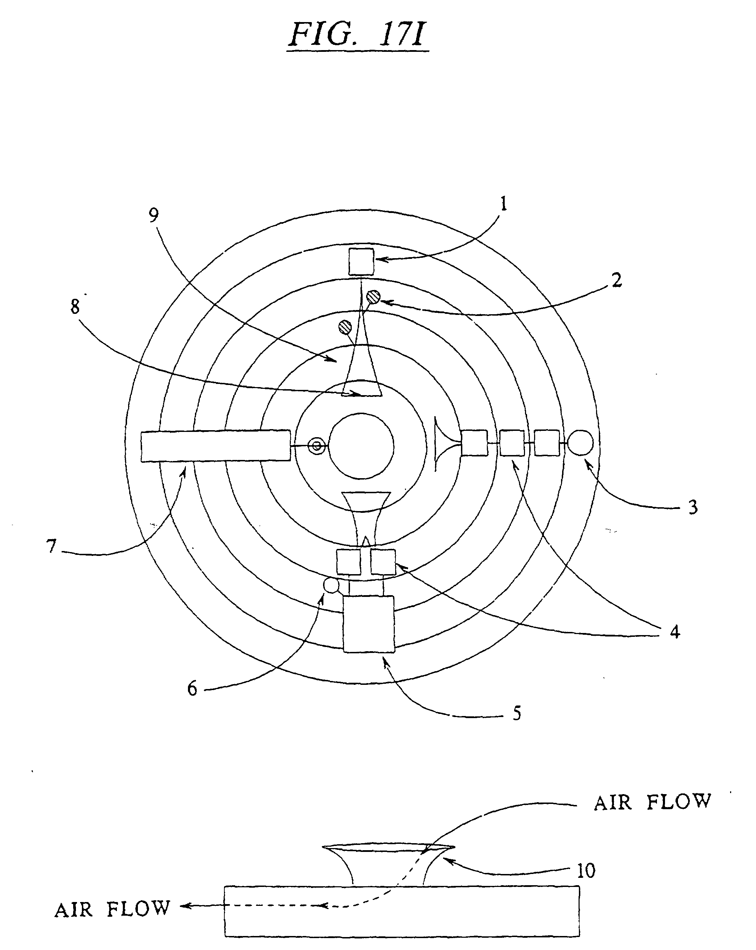

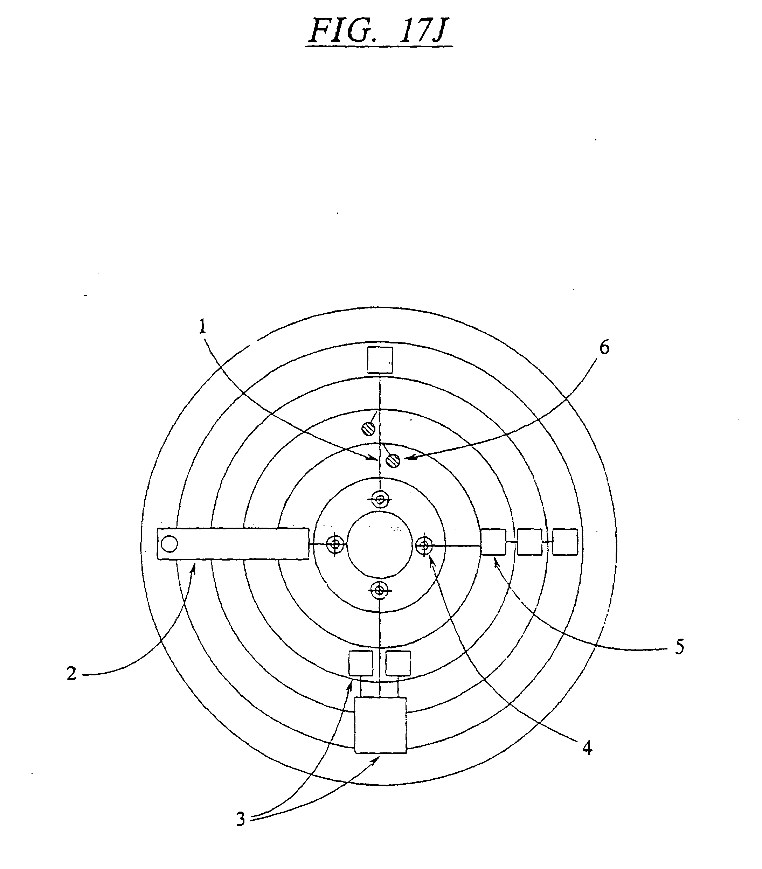

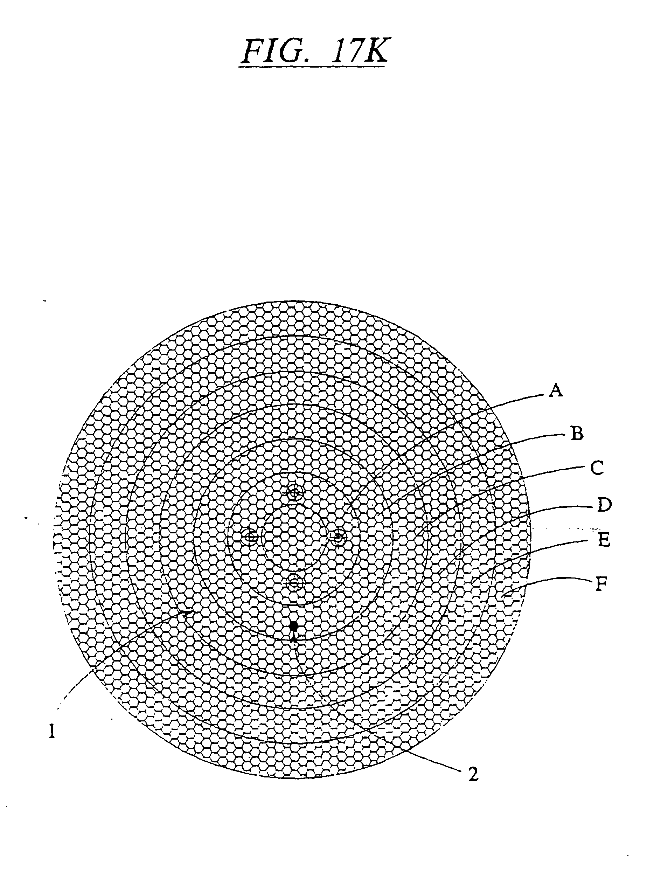

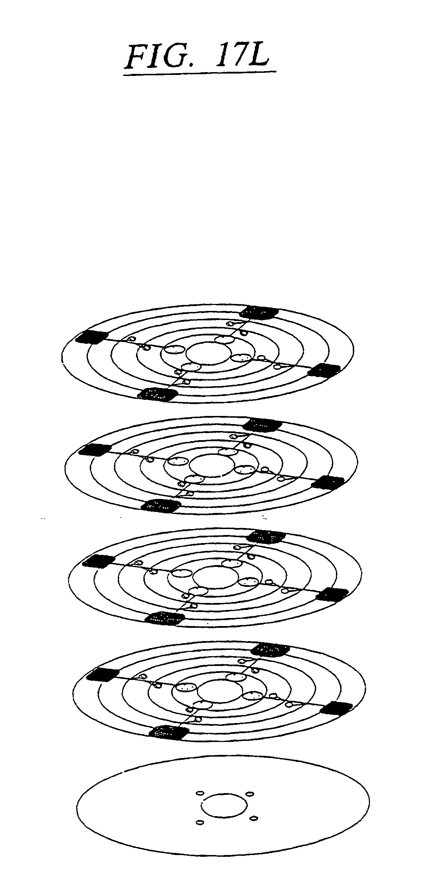

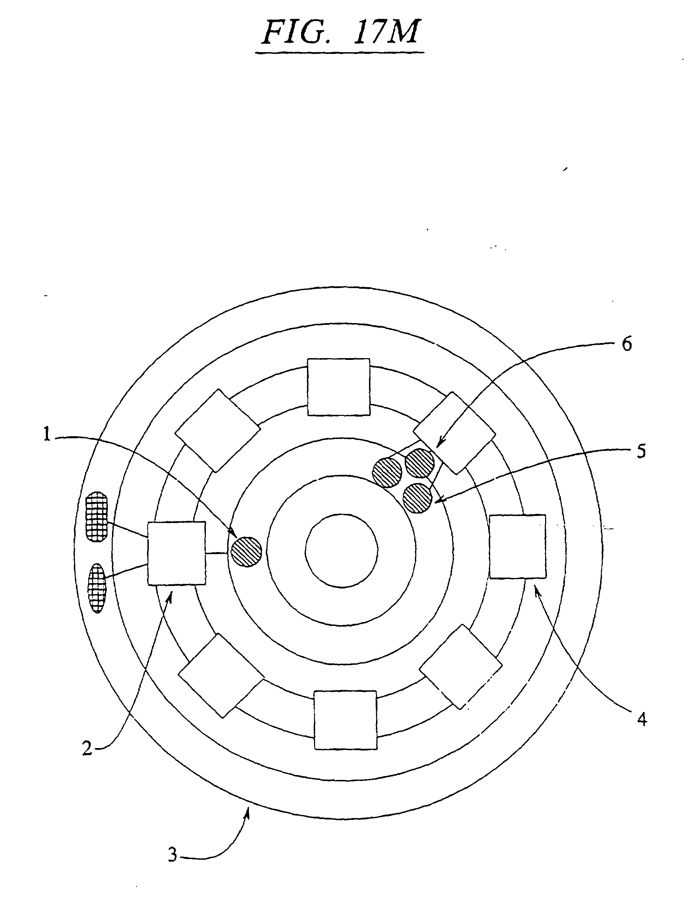

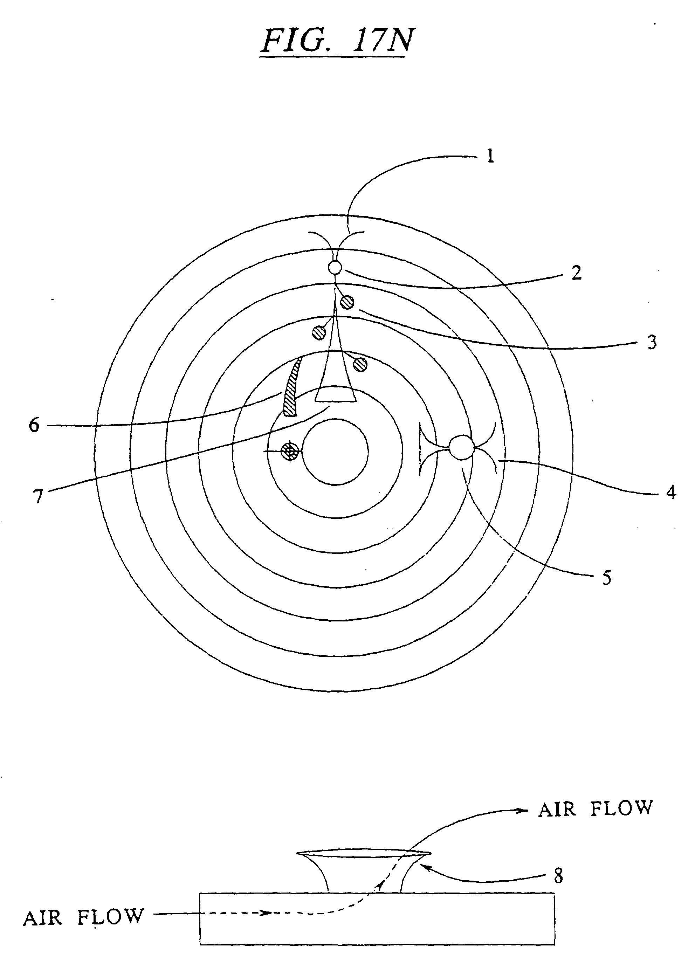

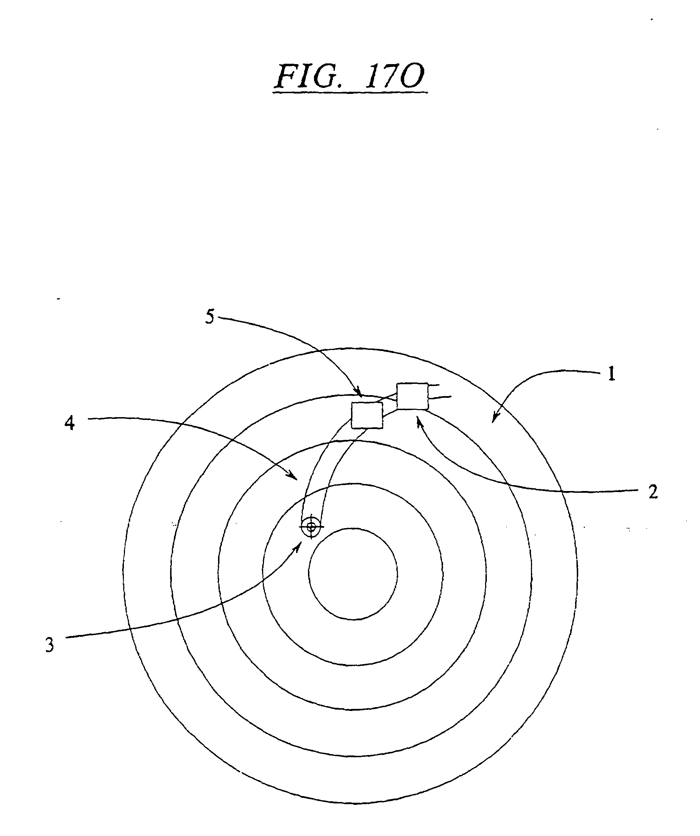

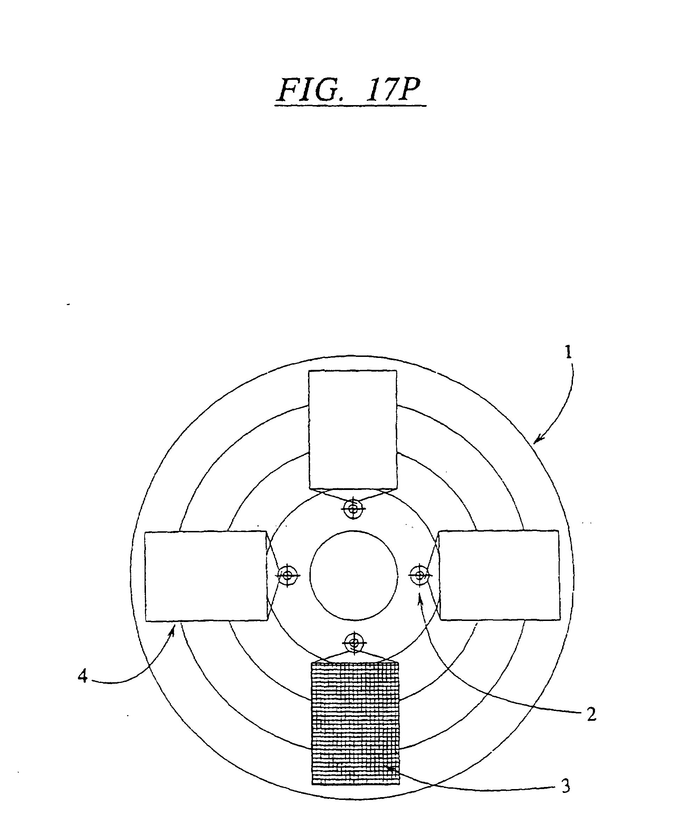

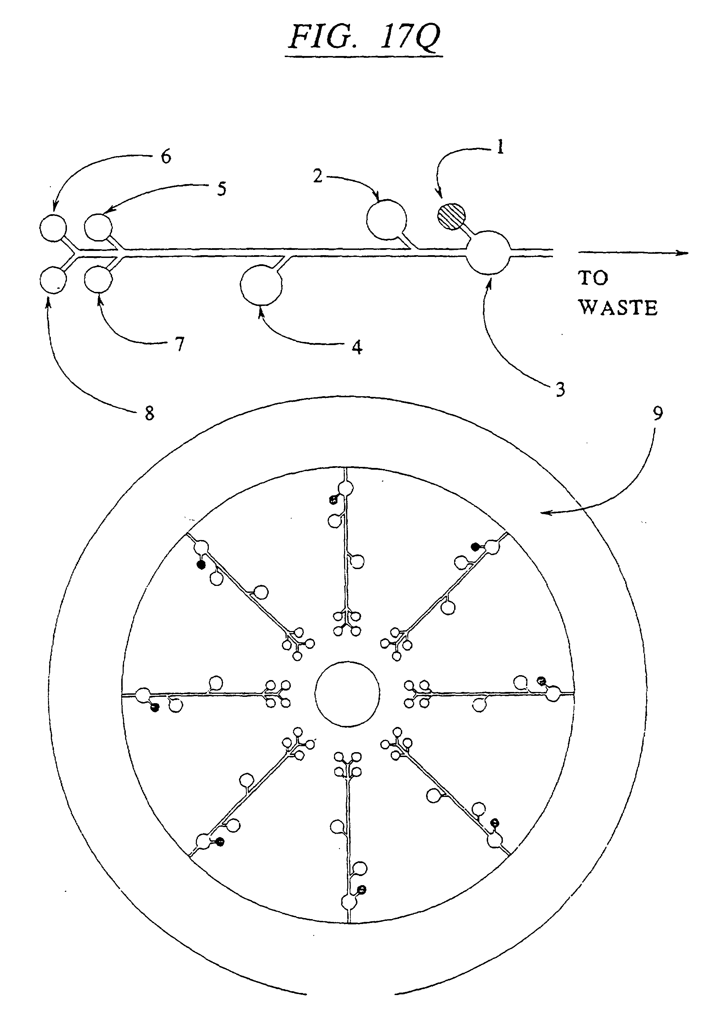

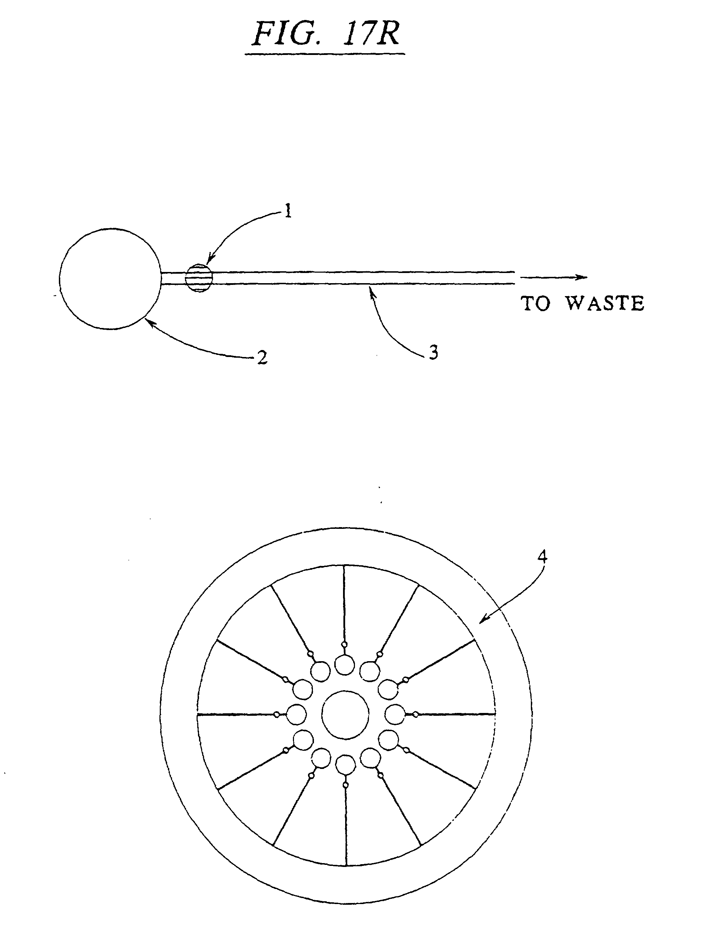

Embodiments comprising thin film disks are provided, comprising "layers" of microsystems disks stacked on a solid support, are useful for sequential assay with conservation of the disk and efficient and inexpensive use of the microsystem-comprising disks as consumables. An illustration of such a disk is shown in Figure 17L. Such disks are capable of being uniquely identified, for example, by imprinting a barcode directly on the disk.

Particular examples of disks fabricated for a variety of applications are provided below in the Examples.

## Disk-Related Devices and Elements

Microsystems platforms (microplatforms) of the invention are provided with a multiplicity of on-board components, either fabricated directly onto the disk, or placed on the disk as prefabricated modules. In addition to the integral components of the disk, certain devices and elements can be located external to the disk, optimally positioned on a device of the invention, or placed in contact with the disk.

1. Temperature control elements

Temperature control elements, particularly heating elements, include heat lamps, direct laser heaters, Peltier heat pumps, resistive heaters, ultrasonication heaters and microwave excitation heaters. Cooling elements include Peltier devices and heat sinks, radiative heat fins and other components to facilitate radiative heat loss. Thermal devices can be applied to the disk as a whole or in specific areas on the disk. The thermal elements can be fabricated directly onto the disk, or can be fabricated independently and integrated onto the disk. Thermal elements can also be positioned external to the disk. The temperature of any particular area on the disk is monitored by resistive temperature devices (RTD), thermistors, liquid crystal birefringence sensors or by infrared interrogation using IR-specific detectors. Temperature at any particular region of the disk can be regulated by feedback control systems. A micro-scale thermo-control system can be fabricated directly on the disk, fabricated on a microchip and integrated into the disk or controlled through a system positioned external to the disk.

2. Filters

Filters, sieving structures and other means can be provided for selectively retaining or facilitating passage of particulate matter, including cells, cell aggregates, protein aggregates, or other particulate matter comprising fluids applied to a microanalytical or microsynthetic disk of the invention. Such filtering means include microsieving structures that are fabricated directly into a fluid handling structure on the disk (*e.g.*, U.S. Patent 5,304,487; International Application, Publication No. WO93/22053; Wilding *et al.,* 1994, *Automat. Analyt. Tech.* 40: 43-47) or fabricated separately and assembled into the fluid handling structures. The sieving structures are provided with a range of size exclusion orifices and are optionally arranged sequentially so as to fractionate a sample based upon the sizes of the constituent parts of the sample.

Other types of filters include materials that selectively remove sample constituents based on electrostatic forces between the filter material and the sample constituents. The electrostatic composition of the sieving materials may be inherent to the material or bestowed upon it by virtue of a charge delivered to the material through an electronic circuit. The materials captured by the filter material can be irreversibly bound or can be selectively eluted for further processing by adjusting the composition and ionic strength of buffers or, in the case of an electronically regulated material, by modulating the electronic state of the material.

In yet other embodiments of the filters of the microsystem platforms of this invention, specific components of a sample can be retained in a section, microchannel or reservoir of a disk of the invention by interaction with specific proteins, peptides, antibodies or fragments thereof derivatized to be retained within the surface of a component of the disk. Materials captured by such specific binding can be eluted from the surface of the disk and transferred to a collection reservoir by treatment with appropriately-chosen ionic strength buffers, using conventional methods developed for immunological or chromatographic techniques.

The invention also provides compartments defined by sections of a microchannel or by a chamber or reservoir wherein the inlet and outlet ports of the chamber are delimited by a filtering apparatus. In certain embodiments, the chamber thus defined contains a reagent such as a bead and particularly a bead coated with a compound such as an antibody having an affinity for a contaminant, unused reagent, reaction side-product or other compound unwanted in a final product. In the use of disks comprising such a filter-limited chamber, a fluid containing a mixture of wanted and unwanted compounds is moved through the filter chamber by centripetal force of the rotating disk. The unwanted compounds are thus bound by the affinity material, and the desired compounds flushed free of the chamber by fluid flow motivated by centripetal force. Alternatively, the desired compound may be retained in such a filter-limited chamber, and the unwanted compounds flushed away. In these embodiments, egress from the chamber, for example by the opening of a valve, is provided.

3. Mixers

A variety of mixing elements are advantageously included in embodiments of the microsystems disks of the invention that require mixing of components in a reaction chamber upon addition from a reagent reservoir. Static mixers can be incorporated into fluid handling structures of the disk by applying a textured surface to the microchannels or chambers composing the mixer. Two or more channels can be joined at a position on the disk and their components mixed together by hydrodynamic activity imparted upon them by the textured surface of the mixing channel or chamber and the action of centripetal force imparted by the rotating disk. Mixing can also be accomplished by rapidly changing the direction of rotation and by physically agitating the disk by systems external to the disk.

In other embodiments, flex plate-wave (FPW) devices (*see* White, 1991, U.S. Patent No. 5,006,749, *ibid.*) can be used to effect mixing of fluids on a disk of the invention. FPW devices utilize aluminum and piezoelectric zinc oxide transducers placed at either end of a very thin membrane. The transducers launch and detect acoustic plate waves that are propagated along the membrane. The stiffness and mass per unit area of the membrane determine the velocity of plate wave. When connected with an amplifier, the waves form a delay-line oscillation that is proportional to the acoustic wave velocity. Structures based on the FPW phenomena have been used to sense pressure, acceleration, organic chemical vapors, the adsorption of proteins, the density and viscosity of liquids as well as to mix liquids together. FPW devices can be integrated onto the disk or can be positioned in proximity to the disk to effect mixing of fluid components in particular reaction chambers on the disk.

4. Valving Mechanisms

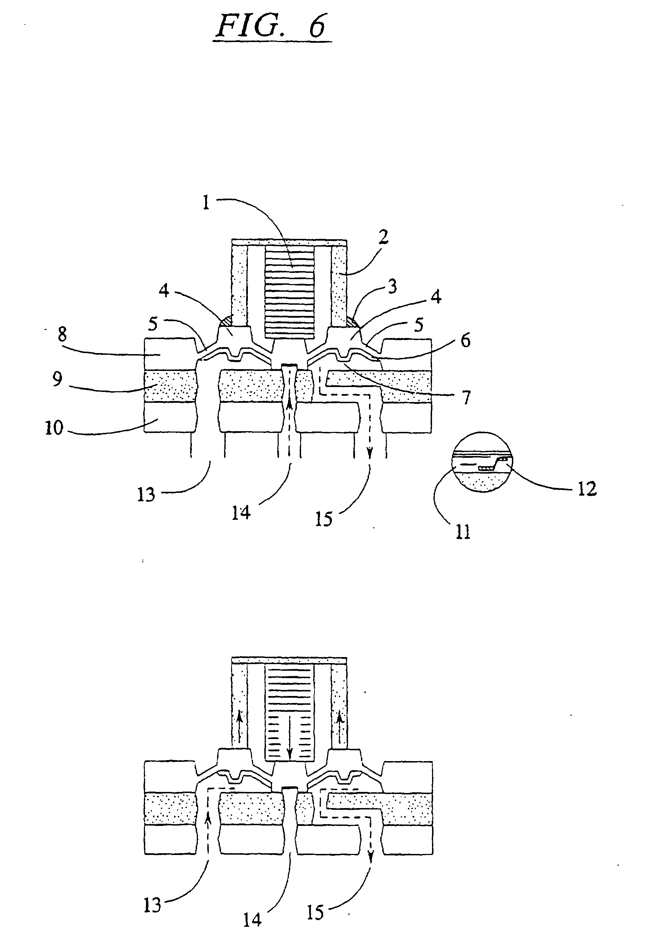

Control of fluid movement and transfer on the disk typically includes the use of valving mechanisms (microvalves) to permit or prevent fluid movement between components. Examples of such microvalves include a piezo activator comprising a glass plate sandwiched between two silicon wafers, as described by Nakagawa *et al.* (1990, Proc. IEEE Workshop of Micro Electro Mechanical Systems, Napa Valley, CA pp. 89); a schematic diagram of such a valve is shown in Figure 6. In this embodiment, a lower wafer and glass plate can have one or two inlets and one outlet channel etched in them. An upper wafer can have a circular center platform and a concentric platform surrounding it. The base of piezoelectric stack can be placed onto the center platform and its top connected to the concentric platform by means of circular bridge. The center of a $SiO_2/SiN_4$ arch-like structure is connected to the piezo element. Valve seats are made of nickel or other sealing substance. In a three-way embodiment, fluid moves from the center inlet port to the outlet with no applied voltage. With a voltage applied the piezo element presses down on the arch center causing the ends to lift, blocking the center inlet and allowing fluid to flow from the peripheral inlet. In other, two-way embodiments, fluid flows with no applied voltage and is restrained upon the application of voltage. In another embodiment of a two-way valve, fluid is restrained in the absence of an applied voltage and is allowed to flow upon application of a voltage. In any of these embodiments the piezo stack can be perpendicular to the plane of rotation, oblique to the plane of rotation, or held within the plane of rotation.

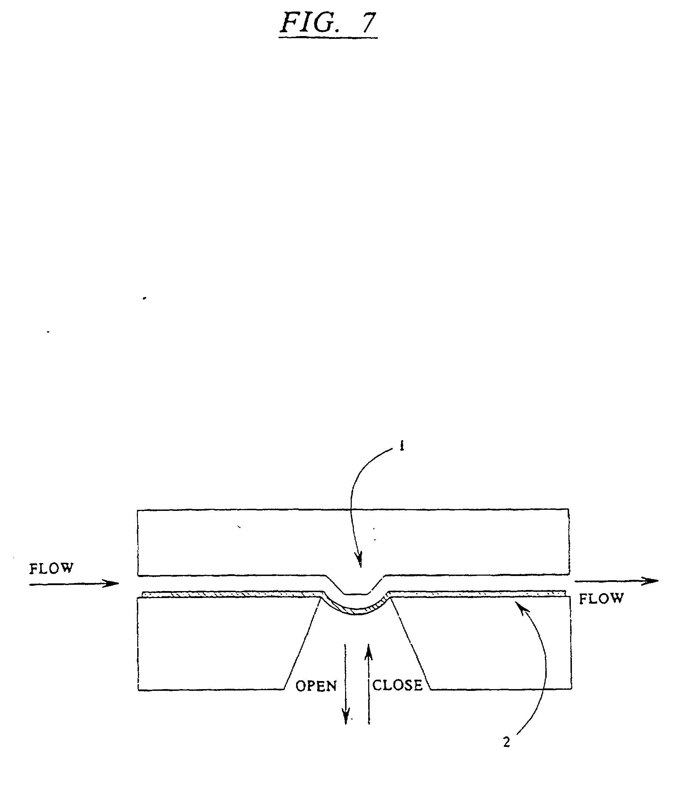

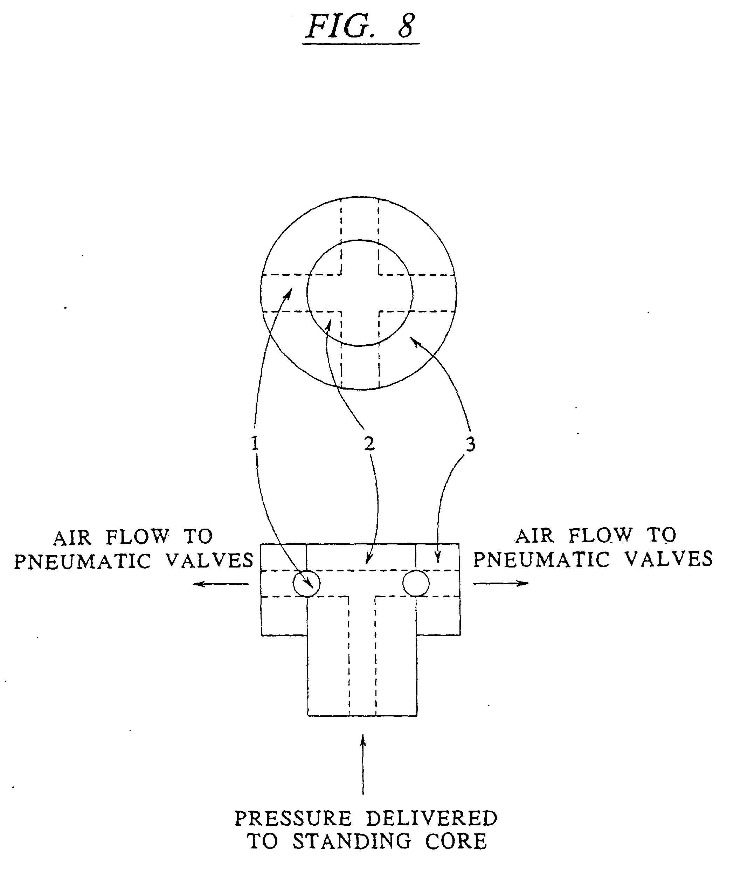

In another embodiment, fluid control is effected using a pneumatical ly-actuated microvalve wherein a fluid channel is etched in one layer of material that has a raised valve seat at the point of control (a schematic diagram of this type of valve is shown in Figure 7). Into another layer, a corresponding hole is drilled, preferably by a laser to achieve a hole with a sufficiently small diameter, thereby providing pneumatic access. Onto that second structure a layer of silicone rubber or other flexible material is spun-deposited. These structures are then bonded together. Fluid movement is interrupted by the application of air pressure which presses the flexible membrane down onto the raised valve seat. This type of valve has been described by Veider *et al.* (1995, Eurosensors IX, pp. 284-286, Stockholm, Sweden, June 25-29). Measurements made by Veider *et al.* have shown that a similar valve closes completely with the application of 30 KPa of pressure over the fluid inlet pressure. This value corresponds to 207 psig, and can be adjusted by changing the diameter of the pneumatic orifice and the thickness of the membrane layer. Pneumatic pressure is applied to the disk to activate such valves as shown schematically in Figure 8.

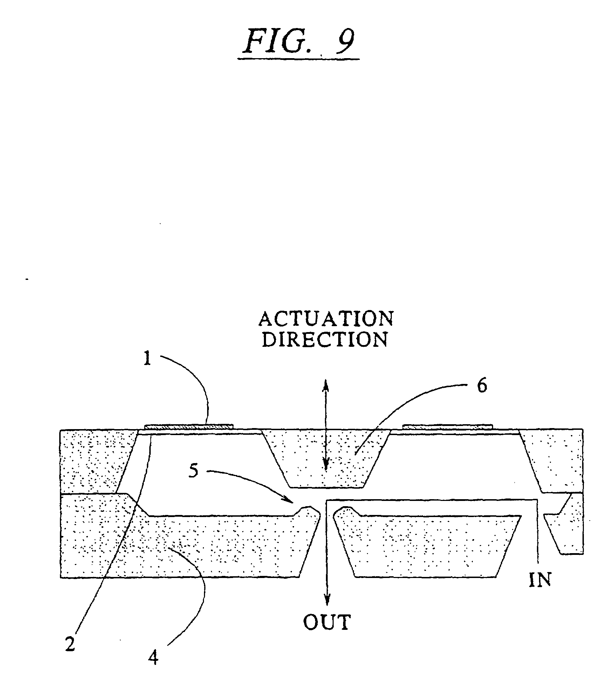

Pneumatic actuation can also be embodied by a micromachined gas valve that utilizes a bimetallic actuator mechanism, as shown in Figure 9. The valve consists of a diaphragm actuator that mates to the valve body. The actuator can contain integral resistive elements that heat upon application of a voltage, causing a deflection in the diaphragm. This deflection causes a central structure in the actuator to impinge upon the valve opening, thus regulating the flow of fluid through the opening. These valves allow proportional control based on voltage input, typically 0 - 15 V DC. These types of valve are commercially available (Redwood Microsystems, Menlo Park, CA; ICSensors, Milpitas, CA).

Embodiments of pneumatically actuated membrane valves can include integration of both components on a single disk or can comprise two disks aligned so that the pneumatic outlets of one disk align with the second disk to impinge upon the pneumatic actuation orifice of the other disk. In either embodiment a source of pneumatic pressure can be delivered to the disk via concentric rings of material such a Teflon®. In this embodiment, a standing core and a revolving element are contiguous to the disk. Pneumatic pressure is delivered through the interior of the standing core and directed by channels to the outer edge of the standing core. Suitably placed channels are machined into the revolving element and impinge upon the channels in the standing core and direct the pneumatic pressure to the gas valves.

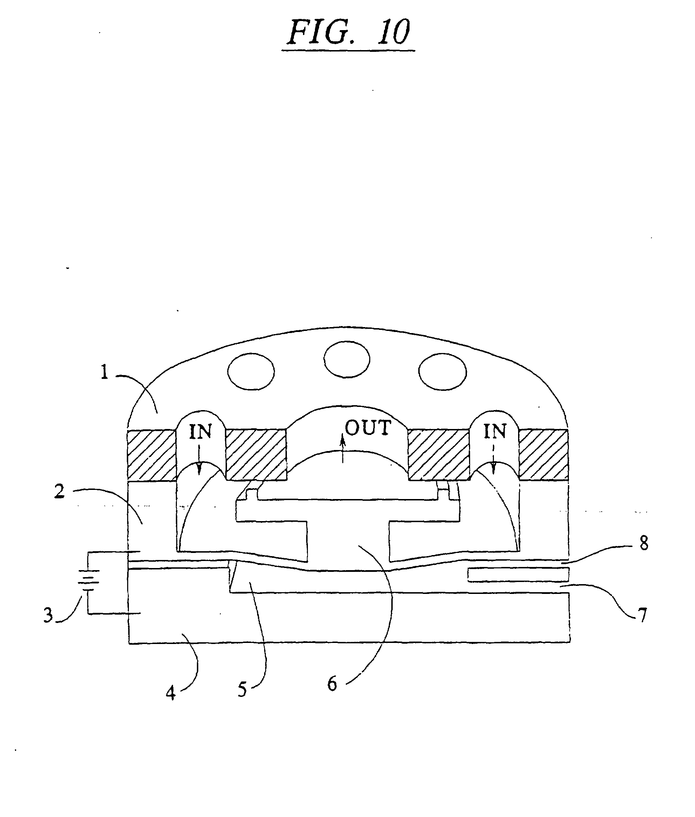

Another valve embodiment is a pressure-balanced microvalve, shown in Figure 10. This type of valve is constructed of three layers of material, comprising two layers of silicon separated by a thin layer of electrically-insulating oxide (*i.e.*, silicon dioxide). A glass layer is bonded onto the top of the valve and advantageously contains inlet and outlet ports. A center plunger fashioned in the middle silicon layer is deflected into a gap contained on the lower silicon layer by application of a voltage between the silicon layers. Alternatively, the plunger is deflected by providing a pneumatic pressure drop into a gap in the lower layer. Irreversible jamming of micromachined parts may be prevented by the application of a thin layer of Cr/Pt to the glass structure. As an electrostatically driven device, this type of valve has many advantages, including that it may be wired directly in the fabrication of the disk. In this embodiment the actuator is a finely tuned device that requires minimal input energy in order to open the valve even at relatively high pressures. These types of valves have been disclosed by Huff *et al.* (1994, 7th International Conference on Solid-State Sensors and Actuators, pp. 98-101).

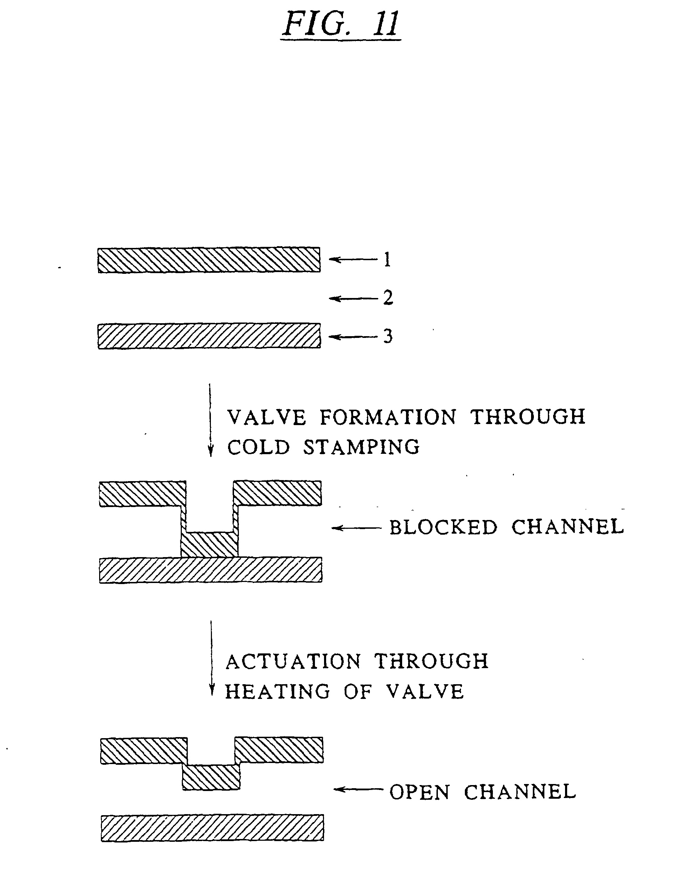

Another type of single-use valve, termed a polymeric relaxation valve, compatible with the disk and fluidic devices in general, is disclosed herein and shown in Figure 11. This valve is based on the relaxation of non-equilibrium polymeric structures. This phenomenon is observed when polymers are stretched at temperatures below their glass transition temperature ($T_g$), resulting in a non-equilibrium structure. Upon heating above the $T_g$, the polymer chains relax and contraction is observed as the structure equilibrates. A common example of this phenomenon is contraction of polyolefin (used in heat shrink tubing or wrap), the polyolefin structure of which is stable at room temperature. Upon heating to 135°C, however, the structure contracts. Examples of PR valve polymers include but are not limited to polyolefins, polystyrenes, polyurethanes, poly(vinyl chloride) and certain fluoropolymers.

One way to manufacture a PR valve is to place a polymer sheet or laminate over a channel requiring the valve (as shown in Figure 11). A cylindrical valve is then cold-stamped in such a way as to block the microchannel. The valve is actuated by the application of localized heat, for example, by a laser or by contact with a resistive heating element. The valve then contracts and fluid flow is enabled.

A further type of microvalve useful in the disks of the invention is a single use valve, illustrated herein by a capillary microvalve. This type of microvalve is based on the use of rotationally-induced fluid pressure to overcome capillary forces. Fluids which completely or partially wet the material of the microchannels (or reservoirs, reaction chambers, detection chambers, etc.) which contain them experience a resistance to flow when moving from a microchannel of narrow cross-section to one of larger cross-section, while those fluids which do not wet these materials resist flowing from microchannels (or reservoirs, reaction chambers, detection chambers, etc.) of large cross-section to those with smaller cross-section. This capillary pressure varies inversely with the sizes of the two microchannels (or reservoirs, reaction chambers, detection chambers, etc., or combinations thereof), the surface tension of the fluid, and the contact angle of the fluid on the material of the microchannels (or reservoirs, reaction chambers, detection chambers, etc.). Generally, the details of the cross-sectional shape are not important, but the dependence on cross-sectional dimension results in microchannels of dimension less than 500μm exhibit significant capillary pressure. By varying the intersection shapes, materials and cross-sectional areas of the components of the microsystems platform of the invention, "valves" are fashioned that require the application of a particular pressure on the fluid to induce fluid flow. This pressure is applied in the disks of the invention by rotation of the disk (which has been shown above to vary with the square of the rotational frequency, with the radial position and with the extent of the fluid in the radial direction). By varying capillary valve cross-sectional dimensions as well as the position and extent along the radial direction of the fluid handling components of the microsystem platforms of the invention, capillary valves are formed to release fluid flow in a rotation-dependent manner, using rotation rates of from 100rpm to several thousand rpm. This arrangement allows complex, multistep fluid processes to be carried out using a pre-determined, monotonic increase in rotational rate.

Control of the microvalves of the disks provided by the invention is achieved either using on-disk controller elements, device-specific controllers, or a combination thereof.

6. Control Systems

Integrated electronic processing systems (generally termed "controllers" herein) that include microprocessors and I/O devices can be fabricated directly onto the disk, can be fabricated separately and assembled into or onto the disk, or can be placed completely off the disk, most advantageously as a component of the micromanipulation device. The controllers can be used to control the rotation drive motor (both speed, duration and direction), system temperature, optics, data acquisition, analysis and storage, and to monitor the state of systems integral to the disk. Examples of rotational controllers are those using rotation sensors adjacent to the motor itself for determining rotation rate, and motor controller chips (*e.g.*, Motorola MC33035) for driving direction and speed of such motors. Such sensors and chips are generally used in a closed-loop configuration, using the sensor data to control rotation of the disk to a rotational set-point. Similarly, the rotational data from these sensors can be converted from a digital train of pulses into an analog voltage using frequency-to-voltage conversion chips (*e.g.*, Texas Instruments Model LM2917). In this case, the analog signal then provides feedback to control an analog voltage set-point corresponding to the desired rotation rate. Controllers may also use the data encoded in the disk's data-carrying surface in a manner similar to that used in commercially-available compact disk (CD) players. In these embodiments, the digital data read by the laser is used to control rotation rate through a phase-locked loop. The rotation rate information inherent in the frequency of data bits read may be converted to an analog voltage, as described above.

The controllers can also include communication components that allow access to external databases and modems for remote data transfer. Specifically, controllers can be integrated into optical read systems in order to retrieve information contained on the disk, and to write information generated by the analytic systems on the disk to optical data storage sections integral to the disk. In these embodiments it will be understood that both read and write functions are performed on the surface of the disk opposite to the surface comprising the microsystems components disclosed herein..

Information (*i.e.*, both instructions and data, collectively termed "informatics") pertaining to the control of any particular microanalytic system on the disk can be stored on the disk itself or externally, most advantageously by the microprocessor and/or memory of the disk device of the invention, or in a computer connected to the device. The information is used by the controller to control the timing and open/closed state of microvalves on the disk, to determine optimal disk rotational velocity, to control heating and cooling elements on the disk, to monitor detection systems, to integrate data generated by the disk and to implement logic structures based on the data collected.

7. Power Supply

The electrical requirements of systems contained on a disk can be delivered to the disk through brushes that impinge upon connections integral to the disk. Alternatively, an electrical connection can be made through the contact point between the microplatform and the rotational spindle or hub connecting the disk to the rotational motivating means. A battery can be integrated into the disk to provide an on-board electrical supply. Batteries can also be used to power the device used to manipulate the disk. Batteries used with the invention can be rechargeable such as a cadmium or lithium ion cell, or conventional lead-acid or alkaline cell.

Power delivered to the disk can be AC or DC. While electrical requirements are determined by the particular assay system embodied on the disk, voltage can range from microvolts through megavolts, more preferably millivolts through kilovolts. Current can range from microamps to amperes. Electrical supply can be for component operation or can be used to control and direct on-disk electronics.

Alternatively, inductive current can be generated on the disk by virtue of its rotation, wherein current is provided by an induction loop or by electrical brushes. Such current can be used to power devices on the disk.

8. Detectors and Sensors

Detection systems for use on the microsystem platforms of the invention include spectroscopic, electrochemical, physical, light scattering, radioactive, and mass spectroscopic detectors. Spectroscopic methods using these detectors encompass electronic spectroscopy (ultraviolet and visible light absorbance, luminescence, and refractive index), vibrational spectroscopy (IR and Raman), and x-ray spectroscopies (x-ray fluorescence and conventional x-ray analysis using micromachined field emitters, such as those developed by the NASA Jet Propulsion Lab, Pasadena, CA).

General classes of detection and representative examples of each for use with the microsystem platforms of the invention are described below. The classes are based on sensor type (light-based and electrochemical). In addition, the detection implementation systems utilizing the detectors of the invention can be external to the platform, adjacent to it or integral to the disk platform.

a. Spectroscopic Methods:

1. Fluorescence

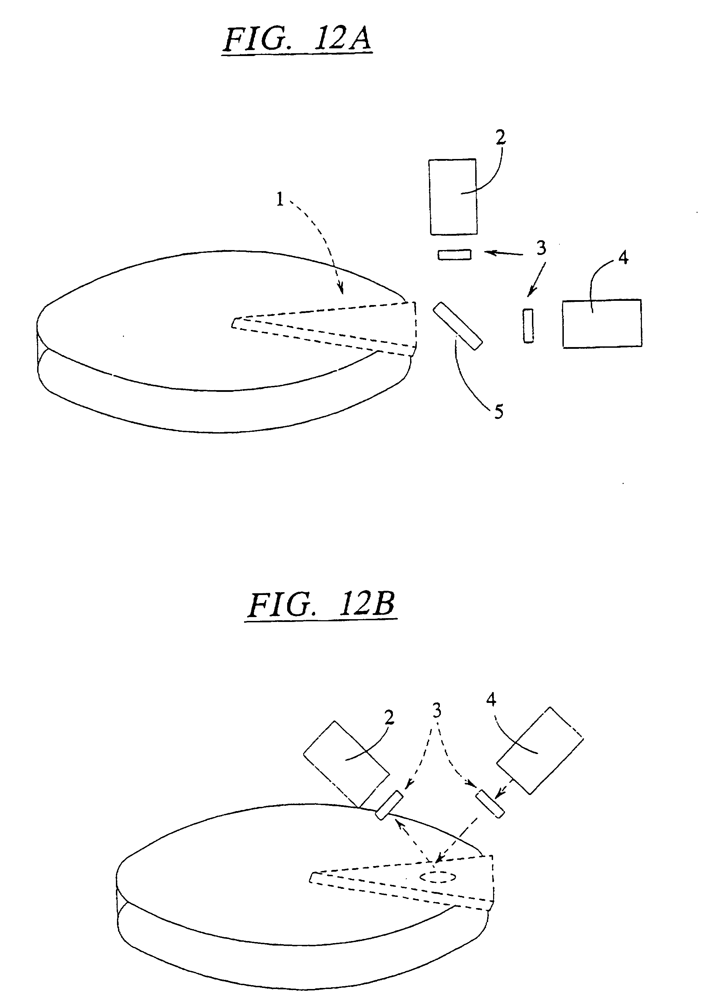

Fluorescence detector systems developed for macroscopic uses are known in the prior art and are adapted for use with the microsystem platforms of this invention. Figure 12A and 12B illustrate two representative fluorescence configurations. In Figure 12A, an excitation source such as a laser is focused on an optically-transparent section of the disk. Light from any analytically-useful portion of the electromagnetic spectrum can be coupled with a disk material that is specifically transparent to light of a particular wavelength, permitting spectral properties of the light to be determined by the product or reagent occupying the reservoir interrogated by illumination with light. Alternatively, the selection of light at a particular wavelength can be paired with a material having geometries and refractive index properties resulting in total internal reflection of the illuminating light. This enables either detection of material on the surface of the disk through evanescent light propagation, or multiple reflections through the sample itself, which increases the path length considerably.

Configurations appropriate for evanescent wave systems are shown in Figure 12A (*see* Glass *et al.,* 1987, *Appl. Optics* 26: 2181-2187). Fluorescence is coupled back into a waveguide on the disk, thereby increasing the efficiency of detection. In these embodiments, the optical component preceding the detector can include a dispersive element to permit spectral resolution. Fluorescence excitation can also be increased through multiple reflections from surfaces in the device whenever noise does not scale with path length in the same way as with signal.

Another type of fluorescence detection configuration is shown in Figure 12B. Light of both the fluorescence excitation wavelength and the emitted light wavelength are guided through one face of the device. An angle of 90 degrees is used to separate the excitation and collection optical trains. It is also possible to use other angles, including 0 degrees, whereby the excitation and emitted light travels colinearly. As long as the source light can be distinguished from the fluorescence signal, any optical geometry can be used. Optical windows suitable for spectroscopic measurement and transparent to the wavelengths used are included at appropriate positions (*i.e.*, in "read" reservoir embodiments of detecting chambers) on the disk. The use of this type of fluorescence in macroscopic systems has been disclosed by Haab *et al.* (1995, *Anal. Chem.* 67: 3253-3260).

2. Absorbance Detection

Absorbance measurements can be used to detect any analyte that changes the intensity of transmitted light by specifically absorbing energy (direct absorbance) or by changing the absorbance of another component in the system (indirect absorbance). Optical path geometry is designed to ensure that the absorbance detector is focused on a light path receiving the maximum amount of transmitted light from the illuminated sample. Both the light source and the detector can be positioned external to the disk, adjacent to the disk and moved in synchrony with it, or integral to the disk itself. The sample chamber on the disk can constitute a cuvette that is illuminated and transmitted light detected in a single pass or in multiple passes, particularly when used with a stroboscopic light signal that illuminates the detection chamber t a frequency equal to the frequency of rotation or multiples thereof. Alternatively, the sample chamber can be a planar waveguide, wherein the analyte interacts on the face of the waveguide and light absorbance is the result of attenuated total internal reflection (*i.e.*, the analyte reduces the intensity source light if the analyte is sequestered at the surface of the sample chamber, using, for example, specific binding to a compound embedded or

**16**

attached to the chamber surface; *see* Dessy, 1989, *Anal. Chem.* 61: 2191).

Indirect absorbance can be used with the same optical design. For indirect absorbance measurements, the analyte does not absorb the source light; instead, a drop in absorbance of a secondary material is measured as the anatyte displaces it in the sample chamber. Increased transmittance therefore corresponds to analyte concentration.

3. Vibrational spectroscopy

Vibration spectroscopic detection means are also provided to generate data from a detecting chamber or "read" section of a microplatform of the invention. Infrared (IF) optical design is analogous to the design parameters disclosed above with regard to absorbance spectroscopy in the UV and visible range of the electromagnetic spectrum, with the components optimized instead for infrared frequencies. For such optimization, all materials in the optical path must transmit IR light. Configuration of the optical components to provide Raman light scattering information are similar to those disclosed in Figures 12A and 12B above for fluorescent measurements. However, due to the illumination time needed to generate sufficient signal, the rotation rate of the disk must be slowed, or in some instances, stopped. Depending on the use, static IR or Raman scattering analysis is most advantageously performed off-line in a separate IR or Raman instrument adapted for analysis of the disks of the invention.

4. Light scattering

Turbidity can also be measured on the disk. Optics are configured as with absorbance measurements. In this analysis, the intensity of the transmitted light is related to the concentration of the light-scattered particles in a sample. An example of an application of this type of detection method is a particle agglutination assay. Larger particles sediment in a rotating disk more rapidly than smaller particles, and the turbidity of a solution in the sample chamber before and after spinning the disk can be related to the size of the particles in the chamber. If small particles are induced to aggregate only in the presence of an analyte, then turbidity measurements can be used to specifically detect the presence of an analyte in the sample chamber. For example, small particles can be coated with an antibody to an analyte, resulting in aggregation of the particles in the presence of the analyte as antibody from more than one particle binds to the analyte. When the disk is spun after this interaction occurs, sample chambers containing analyte will be less turbid that sample chambers not containing analyte. This system can be calibrated with standard amounts of analyte to provide a gauge of analyte concentration related to the turbidity of the sample under a set of standardized conditions.

Other types of light scattering detection methods are provided for use with the microsystems platforms and devices of the invention. Monochromatic light from a light source, advantageously a laser light source, is directed across the cross-sectional area of a flow channel on the disk. Light scattered by particles in a sample, such as cells, is collected at several angles over the illuminated portion of the channel (*see* Rosenzweig *et al.,* 1994, *Anal. Chem.* 66: 1771-1776). Data reduction is optimally programmed directly into the device based on standards such as appropriately-sized beads to relate the signal into interpretable results. Using a calibrated set of such beads, fine discrimination between particles of different sizes can be obtained. Another application for this system is flow cytometry, cell counting, cell sorting and cellular biological analysis and testing, including chemotherapeutic sensitivity and toxicology.

b. Electrochemical Detection Methods

Electrochemical detection requires contact between the sensor element and the sample, or between sensor elements and a material such as a gas in equilibrium with the sample. In the case of direct contact between sample and detector, the electrode system is built directly onto the disk, attached to the disk before rotation or moved into contact with the disk after it has stopped rotating. Detectors constructed using a gas vapor to encode information about the sample can be made with the detector external to the disk provided the gas vapor is configured to contact both the sample chamber and the detector. Electrochemical detectors interfaced with the disk include potentiometric, voltammetric and amperimetric devices, and can include any electrochemical transducer compatible with the materials used to construct the microsystem disk.

1. Electric Potential Measurement

One type of electrochemical detection means useful with the microsystems platforms of the invention is an electrical potential measurement system. Such a system provides a means for characterizing interfacial properties of solutions passed over differently activated flow channels in the instrument. In view of the temperature-controlled nature of the microplatforms of the invention, streaming potentials can also be measured on this device (*see* Reijenga *et al.,* 1983, *J. Chromatogr.* 260: 241). To produce streaming potentials, the voltage potential difference between two platinum leads in contact with a solution at the inner and outer portions of the disk is measured in comparison with a reference electrode. As fluid flows under controlled centripetal motion through the channel, a streaming potential develops in response to fluid interactions with the disk surfaces in a moving field.

Alternatively, a platinum electrode is used to generate electroluminescent ions *(see* Blackburn *et al.,* 37: 1534-9). Chemiluminescence is then detected using one of the optical detectors described above, depending on the wavelength of the chemiluminescent signal. Voltametric components are also useful in microsynthetic platforms of the invention to produce reactive intermediates or products.

2. Electrochemical Sensors

Electrochemical sensors are also advantageously incorporated into the disk. In one embodiment, an electrochemical detector is provided that uses a redox cycling reaction (*see* Aoki *et al., Rev. Polarogr.* 36: 67). This embodiment utilizes an interdigitated microarray electrode within a micromachined chamber containing a species of interest. The potential of one electrode is set at the oxidized potential of the species of interest and the potential of the other electrode is set at the reduction potential of the species of interest. This is accomplished using a dual channel potentiostat, allowing the oxidized and reduced (*i.e.*, redox) chemical state of the sample to be determined, or the chamber may be preset for a particular species. A volume of fluid containing a substance of interest is directed to the chamber. The electrochemically reversible species is then oxidized and reduced by cyclically energizing the electrodes. In this embodiment a molecule is detected by an apparent increase in the redox current. Since non-reversible species do not contribute signal after the first cycle, their overall contribution to the final signal is suppressed. Data analysis software is used to suppress signal due to non-reversible species.

In another embodiment, a multichannel electrochemical detector is provided comprising up to 16 lines of an electrode fabricated in a chamber by photolithography with dimensions resulting in each line being 100μm wide with 50 μm between lines. (*see* Aoki *et al.,* 1992, *Anal. Chem.* 62: 2206). In this embodiment, a volume of fluid containing a substance of interest is directed to the chamber. Within the chamber each electrode is set at a different potential so that 16 separate channels of electrochemical measurement may be made. Additionally, each electrode potential can be swept stepwise by a function generator. This protocol yields information pertaining to redox potential as well as redox current of the substances. This type of analysis also allows identification of molecules *via* voltammogram.

c. Physical Methods

Physical detection methods are also provided for use with the disks of the invention. For example, the disk can be used as a viscometer. Microchannels containing fluid to be tested advantageously contain a bead inserted on the disk. The motion of the bead through the fluid is analyzed and converted into viscosity data based on standards developed and stored in microprocessor memory. (*see* Linliu *et al.,* 1994, *Rev.Sci. Instrum.* 65: 3824-28).

Another embodiment is a capacitive pressure sensor *(see* Esashi *et al.,* 1992, *Proc. Micro Electro Mechanical Systems* 11: 43). In this embodiment, silicon and glass substrates are anodically bonded with hermetically sealed reference cavities. Pressure may be detected by the capacitance change between the silicon diaphragm and an aluminum electrode formed on the glass. A capacitance-to-frequency converter output of a CMOS circuit can be integrated on the silicon substrate or contained in controlling electronics off the disk.

By judicious placement of pressure sensors, the pressure due to centrifugation can be determined at any position on the disk. In conjunction with the microchannel diameter information and the pattern of orientation of the channels on the disk, pressure data can be used to determine flow rates at a particular rotational speed. This information can then be used by the microprocessor to adjust disk rotational speed to control fluid movement on the disk.

Surface acoustic wave (SAW) devices are also provided as components of the microsystems platforms of the invention. These devices can be placed above the disk to detect head-space gases, or incorporated in the fluid channel on the instrument. When placed in the fluid system, the SAW is used to detect density changes in the solution, indicative of changing buffer, reagent or reactant composition (*see* Ballantine *et al.,* 1989, *Anal. Chem.* 61: 1989).

Volatile gases on the disk or trapped in the head-space surrounding the disk can be monitored in several ways. For example, a Clark electrode positioned in contact with either the solution of the gases above the disk may be used to detect oxygen content. (Collison *et al.,* 1990, *Anal. Chem.* 62: 1990).

d. Radioactive Detection Components

Microsystems platforms of the invention also can incorporate radioactivity detectors. Radioactive decay of an analyte or synthetic product on a disk of the invention can be detected using a CCD chip or similar single channel photodiode detector capable of integrating signal over time. Alternatively, radioactivity can be determined directly by placing a solid state detector in contact with a radioactive analyte. (*see* Lamture *et al.,* 1994, *Nucleic Acids Res.* 22:

2121-2125).

## Modular structures

Analytic systems provided as components of the platforms of the invention typically consist of combinations of controllers, detectors, buffer and reagent reservoirs, chambers, microchannels, microvalves, heaters, filters, mixers, sensors, and other components. Components that constitute an analytic system on the disk can be composed of one or more of the following: complete integral systems fabricated entirely on the disk; complete integral systems fabricated as a component and assembled into or onto the disk; a subset of components fabricated directly onto the disk and interfaced with a subset of components that are fabricated as a component and assembled into or onto the disk; components that interface with the disk externally through a synchronously spinning disk; and components that interface with the spinning disk from a position that remains stationary in relation to the disk (*e.g.*, the rotational spindle).

## Methods and Uses

Because of its flexibility, the invention offers a myriad of possible applications and embodiments. Certain features will be common to most embodiments, however. These features include sample collection; sample application to disk, incorporating tests of adequacy at the time of sample application; a variety of specific assays performed on the disk; data collection, processing and analysis; data transmission and storage, either to memory, to a section of the disk, or to a remote station using communications software; data output to the user (including printing and screen display); and sample disk disposal (including, if necessary, disk sterilization).

Sample or analyte is collected using means appropriate for the particular sample. Blood, for example, is collected in vacuum tubes in a hospital or laboratory setting, and using a lancet for home or consumer use. Urine can be collected into a sterile container and applied to the disk using conventional liquid-transfer technology. Saliva is preferably applied to the disk diluted with a small volume of a solution of distilled water, mild detergent and sugar flavoring. This solution can be provided as a mouthwash/gargle for detecting antigens, biological secretions and microorganisms. Alternatively, a small sack made of a fishnet polymer material containing the detergent formulation and a chewable resin can be chewed by a user to promote salivation, and then removed from the mouth and saliva recovered and applied conventionally. Amniotic fluid and cerebrospinal fluid are, of necessity, collected using accepted medical techniques by qualified personnel.

Environmental and industrial samples are collected from ground water or factory effluent into containers produced to avoid leaching contaminants in the sample. Soil samples are collected and mixed with a solvent designed to dissolve the analyte of interest. Industrial applications, such as pyrogen screening, are accomplished using specially-designed sample ports.

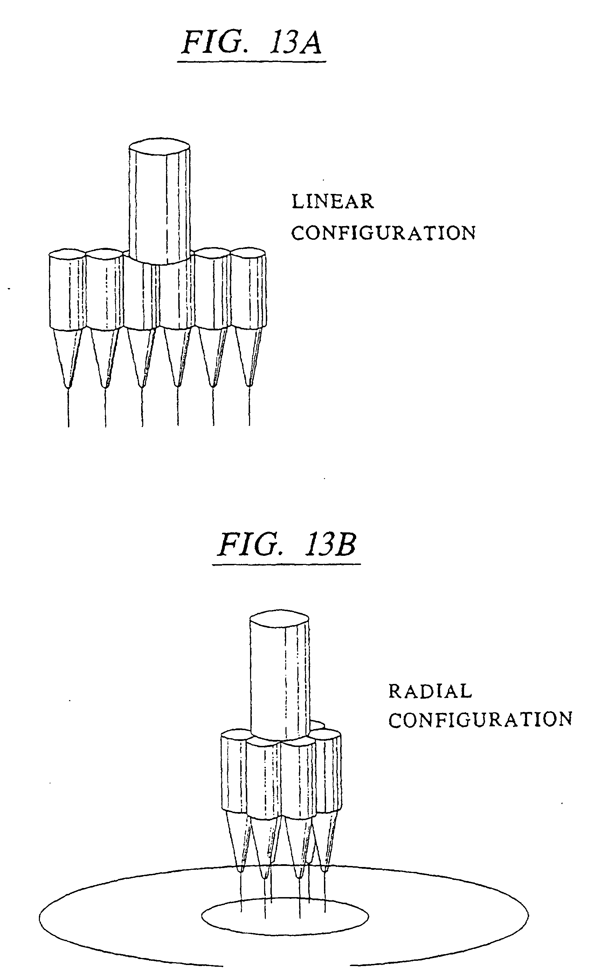

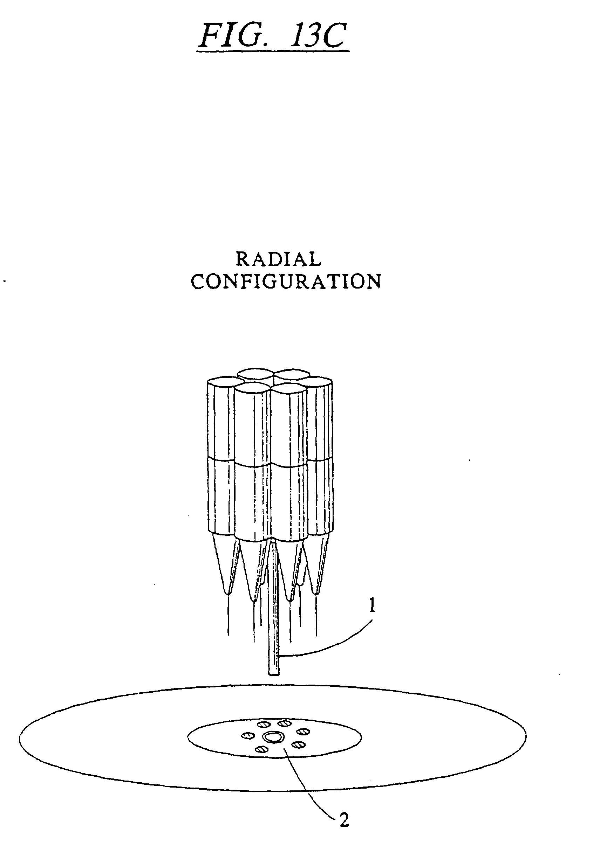

Sample or analyte is loaded onto the disk by the user. Sample is optimally loaded onto the disk at a position proximal to the center of rotation, thereby permitting the greatest amount of centripetal force to be applied to the sample, and providing the most extensive path across the surface of the disk, to maximize the number, length or arrangement of fluid-handling components available to interact with the sample. Multiple samples can be applied to the disk using a multiple loading device as shown in Figures 13A through 13C. In this embodiment of a multiple loading device, a multiplicity of pipette barrels are equally spaced and arranged radially. The pipettes are spaced to provide that the tips of the pipettes fit into access ports on the surface of the disk. The tips can be simple pins that hold a characteristic volume of sample by virtue of a combination of surface properties and fluid characteristics. Alternatively, the tips can be conventional hollow tubes, such as capillary or plastic conical tips, and the fluid manipulated manually in response to positive or negative pressure, as with a manual or automatic pipetting device. The loader can be operated manually or by robotic systems. The barrels can also be arrayed in a flexible arrangement, permitting the tips to address a linear array in one configuration and a radial array in another. In each embodiment, the loader comprises an alignment device to ensure reproducible placement of the loading tips on the disks of the invention.

Loaders are designed specifically for the substances being investigated. Examples include medical uses (where the samples include blood, body fluids including amniotic fluid, cerebrospinal, pleural, pericardial, peritoneal, seminal and synovial fluid, in addition to blood, sweat, saliva, urine and tears, and tissue samples, and excreta), and environmental and industrial substances (including atmospheric gases, water and aqueous solutions, industrial chemicals, and soils). Loading devices are also advantageously compatible with standard blood-handling equipment, such a vacuum tubes fitted with septa, and access sample therein by piercing the septa. Loading devices are also compatible with seat collection devices and means, such as lancets, for obtaining a small blood sample. A disk may also have integral lancets and rubber seals in order to sample blood directly.

Dynamic as well as static loading of the disk is envisioned as being within the scope of the invention (*see* Burtis *et al.,* 1974, *Clin. Chem.* 20: 932-941).

As the invention comprises the combination of a microsystems platform as described above and a micromanipulation device for manipulating this platform to impart centripetal force on fluids on the platform to effect movement, arrangement of components can be chosen to be positioned on the disk, on the device, or both. Mechanical, electronic, optico-electronic, magnetic, magneto-optic, and other devices may be contained within the disk or on disk surface. Some on-disk devices have been described above in detail; additionally, the disk may contain electronic circuitry, including microprocessors for coordination of disk functions, and devices for communication with the disk manipulation device or other devices. The disk optimally comprises detectors and sensors, or components of these devices and energy sources for various detection schemes (such as electric power supplies for electrochemical systems, electromagnetic radiation sources for spectroscopic systems), or materials, such as optically-transparent materials, that facilitate operation of and data generation using such detectors and sensors; actuators, including mechanical, electrical, and electromagnetic devices for controlling fluid movement on the disk, including valves, channels, and other fluid compartments; communications and data handling devices, mediating communications between the disk and the player/reader device, using electromagnetic (laser, infra-red, radiofrequency, microwave), electrical, or other means; circuitry designed for controlling procedures and processes on the disk, including systems diagnostics, assays protocols and analysis of assay data, These are provided in the form of ASICs or ROM which are programmed only at the point-of-manufacture; FPGA's, EPROM, flash memory (UV-erasable EPROM), or programmable IC arrays, or similar arrays programmable by the user through the platform manipulation device or other device. Also included in the components of the invention are CPU and microprocessor units and associated RAM operating with an assembler language or high-level language programmable through disk communications, and components for mediating communication with other devices, including facsimile/modem communications with remote display or data analysis systems.

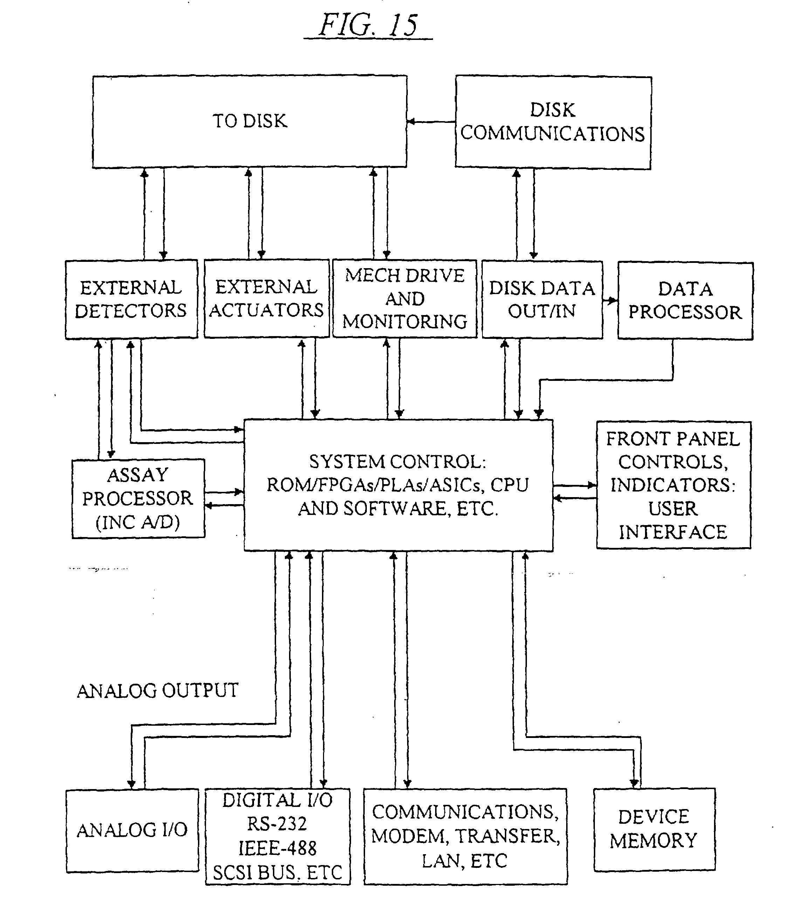

Off-disk devices comprise the microplatform micromanipulating device itself and other devices which can access information, write information, or initiate processes on the disk. Figure 15 illustrates the categories of devices and sub-devices which are part of the micromanipulation device , and indicates how there components interact. "Interaction" is used herein to mean the exchange of "data" between the disk and device, or among the components of the device itself. The relationship between these components is here described, followed by detailed examples of the components.

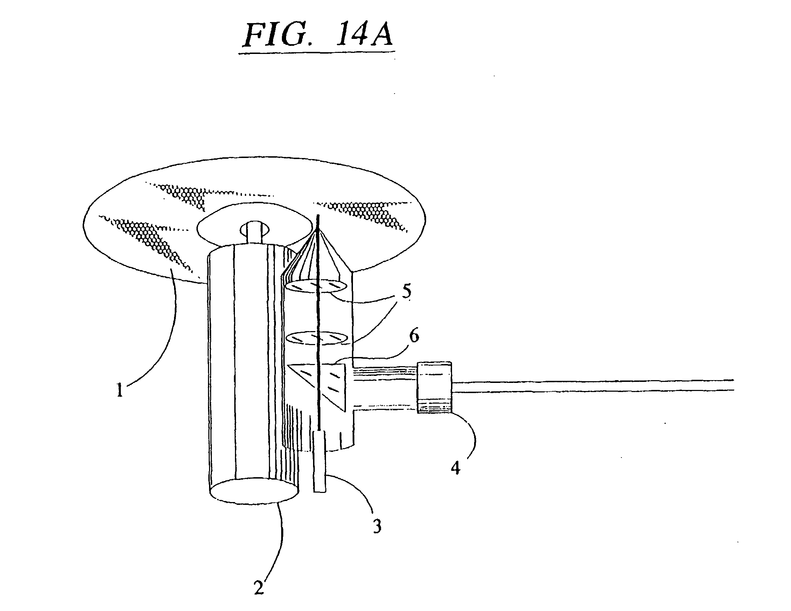

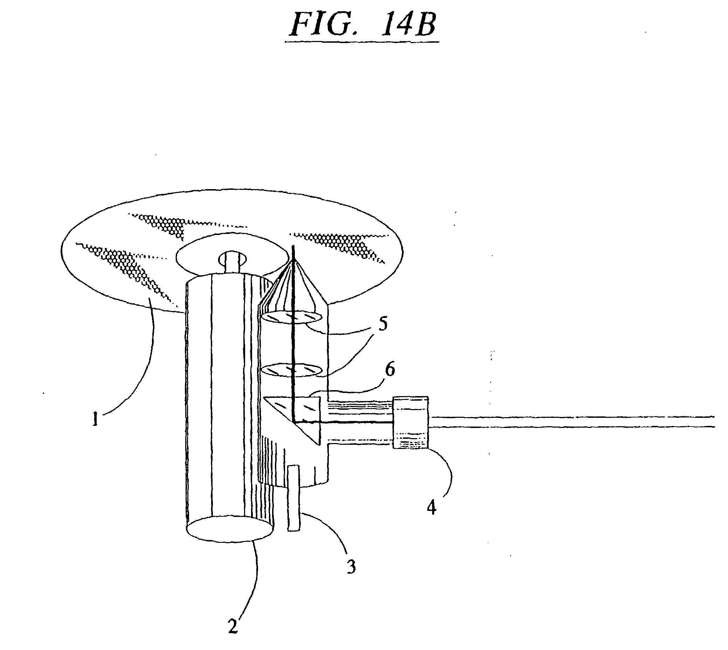

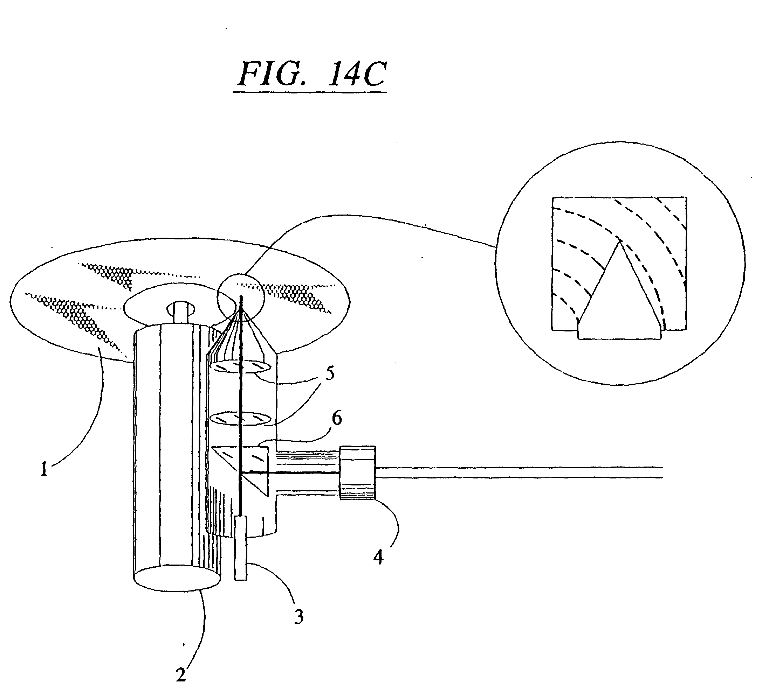

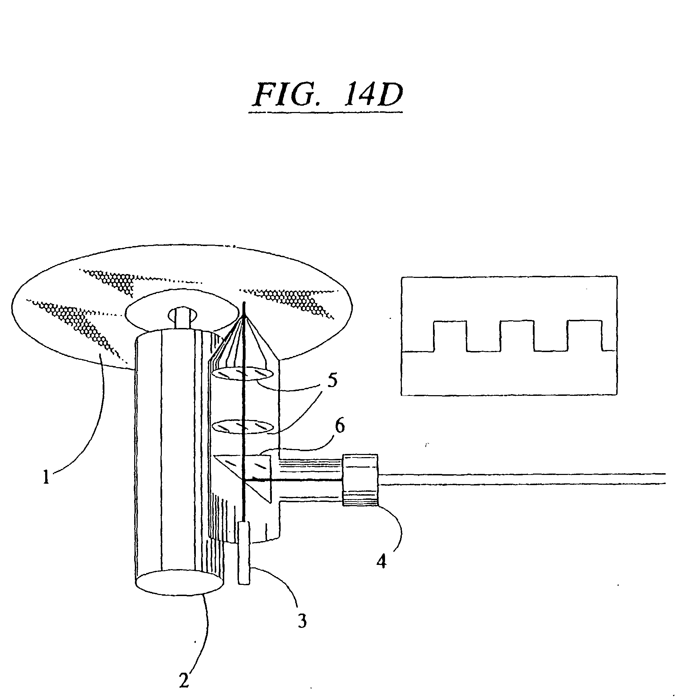

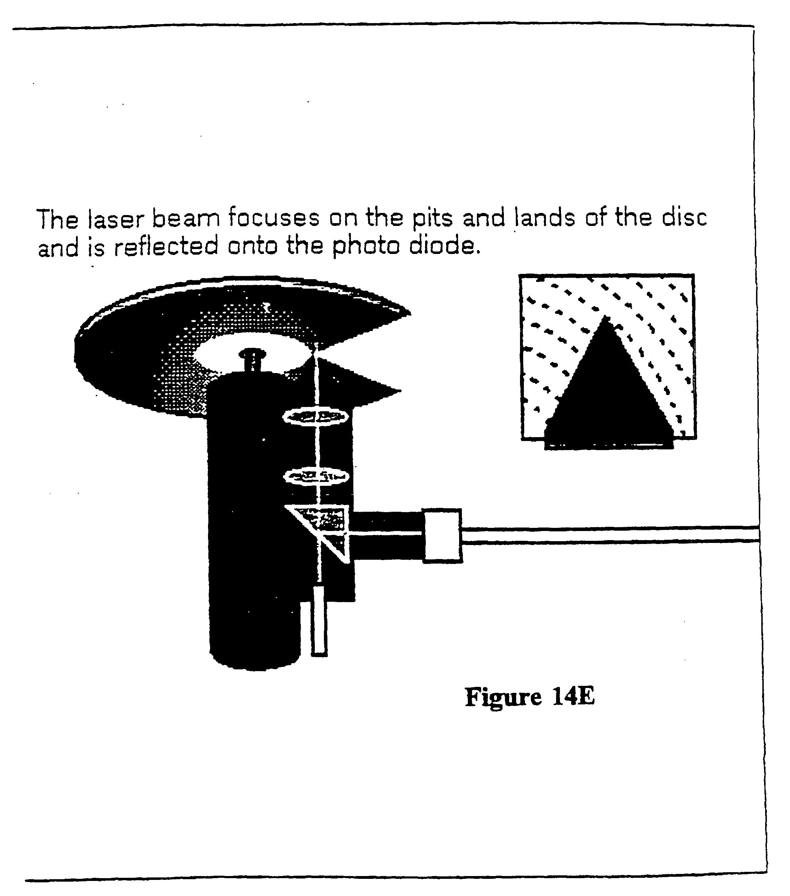

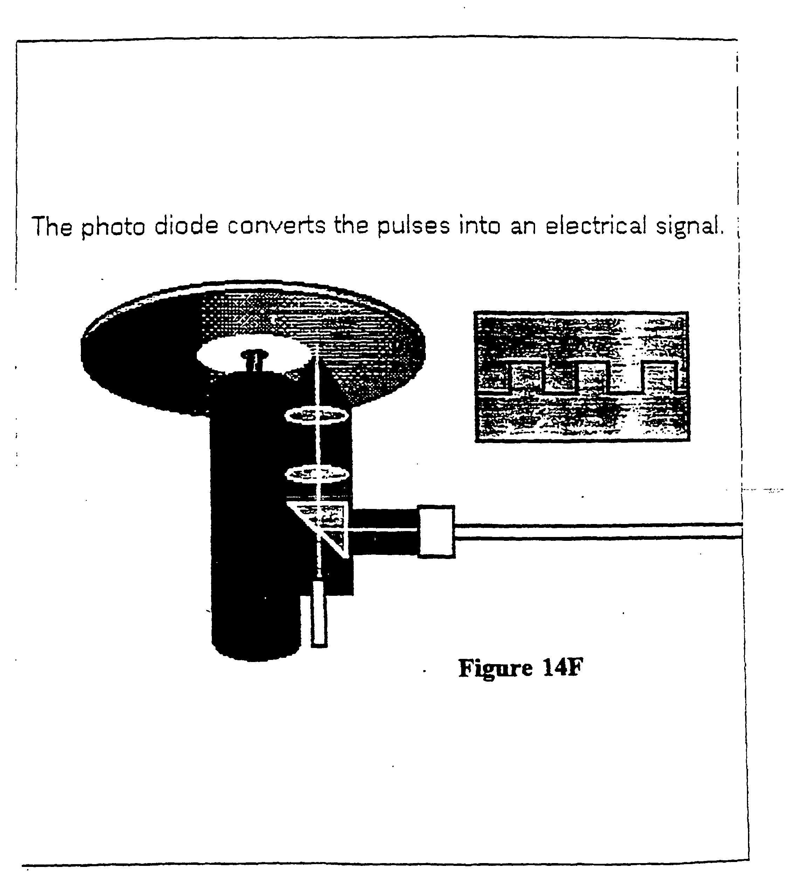

These include the mechanical drive and circuitry for rotation monitoring and control, overall system control, data read/write devices, external detectors and actuators for use with the disk, dedicated data and assay processors for processing encoded data and assay data, a central processor unit, a user interface, and means for communicating to the disk, the user, and other devices. Mechanical drive and associated circuits include devices to control and monitor precisely the rotation rate and angular position of the disk, and devices to select and mount multiple-disks from a cassette, turntable, or other multiple-disk storage unit. System control units provide overall device control, either pre-programmed or accessible to the user-interface. Disk data read/write devices are provided for reading encoded information from a disk or other medium. Optimally, write-to-disk capabilities are included, permitting a section of the disk to contain analytical data generated from assays performed on the disk. This option is not advantageous in uses of the disk where the disks are contaminated with biological or other hazards, absent means (such as sterilization) for neutralizing the hazard. The device can also include external actuators comprising optical magneto-optic, magnetic and electrical components to actuate microvalves and initiate processes on the disk, as well as external detectors. and sensors or components of detectors and sensors that operate in concert with other components on the disk, including analytic and diagnostic devices. Cerain of these aspects of the disk micromanipulating device are illustrated in Figures 14A through 14F.

Disk data processors are also advantageously incorporated into the devices of the invention which enable processing and manipulation of encoded disk data. These components include software used by the micromanipulator CPU, programmable circuits (such as FPGAs, PLAs) and dedicated chipsets (such as ASICs). Also provided are assay processors for processing data arising from events and assays performed on the disk and detected by external detectors or communicated from on disk components. The device also advantageously comprises a central processing unit or computer which will allow processing of disk data and assay results or data-analysis (through pre-programming); additionally, conventional computer capabilities (word-processing, graphics production, etc.) can be provided.

A user interface, including keypads, light-pens, monitors, indicators, flat-panel displays, interface through communications options to host-devices or peripheral devices, and printers, plotters, and graphics devices are provided as components of the microplatform micromanipulating devices of the invention. Communication and telecommunications are provided through standard hard-wired interfaces (such as RS-232, IEE-488M, SCSI bus), infra-red and optical communications, short-or long-range telecommunications ("cellular" telecommunications radio-frequency), and internal or external modem for manual or automated telephone communications.

Disk information comprises both software written to the disk to facilitate operation of the microsystem assays constructed thereupon, and assay data generated during use of the microsystem by the user. Disk information includes material written to the disk (as optically encoded data) and information inherent to the disk (*e.g.*, the current status of a valve, which can be accessed through magnetic pickup or through the reflective properties of the coating material at the valve-position). Data written to the disk may include but is not limited to the audio/video/test and machine format information (*e.g.*, binary, binhex, assembler language). This data includes system control data used for initiation of control programs to spin the disk, or perform assays, information on disk configuration, disk identity, uses, analysis protocols and programming, protocols descriptions, diagnostic programs and test results, point-of-use information, analysis results data, and background information. Acquired data information can be stored as analog or digital and can be raw data, processed data or a combination of both.

System control data include synchronization data to enable the micromanipulation device to function at the correct angular velocity/velocities and accelerations and data relating to physical parameters of disk. Disk configuration and compatibility data include data regarding the type of disk (configuration of on-disk devices, valves, and reagent, reaction and detection chambers) used to determine the applicability of desired testing protocols; this data provides a functional identity of the type of disk and capabilities of the disk. It can be also form part of an interactive feedback system for checking microsystem platform components prior to initiation of an assay on the disk. Disk identify and serial numbers are provided encoded on each disk to enable exact identification of a disk by fabrication date, disk type and uses, which data are encoded by the manufacturer, and user information, which is written to the disk by the user. Also included in disk data is a history of procedures performed with the disk by the user. Also included in the disk data is a history of procedures performed with the disk, typically written for both machine recognition (*i.e.*, how many and which assays remain unused or ready for use), as well as information written by the user.

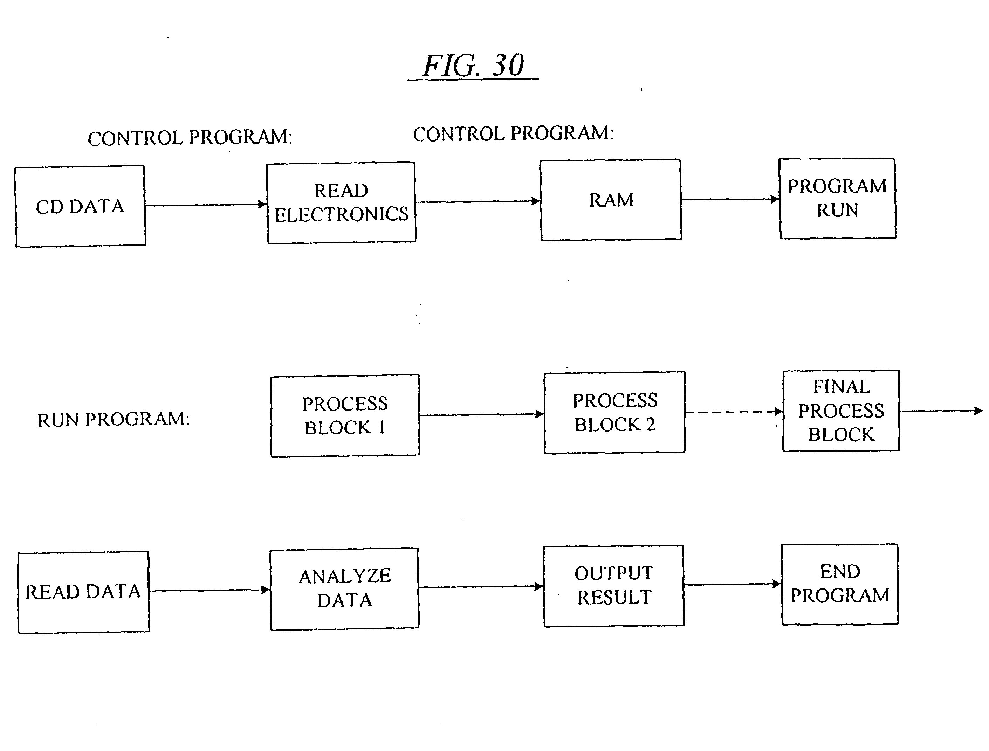

Figures 30-32 display the action of software encoded on the disk used for controlling the device driving the disk. Figure 30 displays the process flow. The control program, encoded as data on the disk, is read through conventional means, for example by the laser of an optical storage medium reader (such as a compact disc or "Laservision" disc player) and decoded in the conventional way for loading into the random access memory (RAM) of the micromanipulation device. This program is then executed. In some applications, execution of the program to completion will be automatic and without active interaction with the user. In other applications the user will be presented with a variety of options (typically, as a menu) for running the program. As an example, user choices, such as whether to run an exhaustive or limited set of diagnostics, test procedures, analyses, or other disk functions, or to determine the extent of detail and the method of reporting test results are provided through the user interfaces.

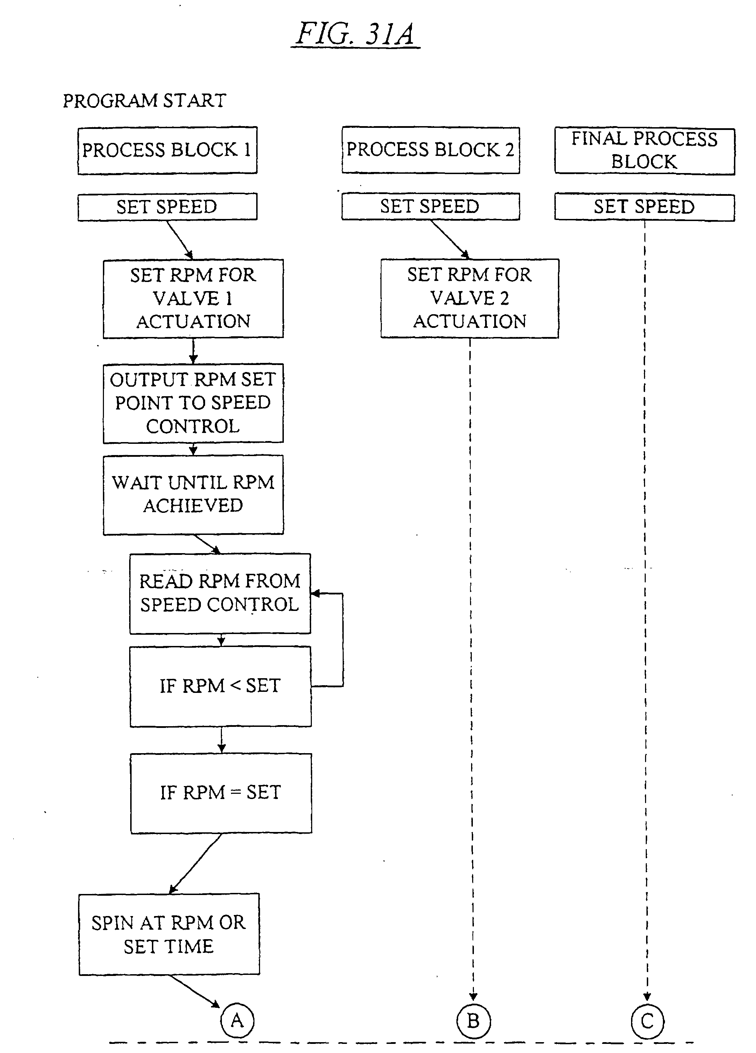

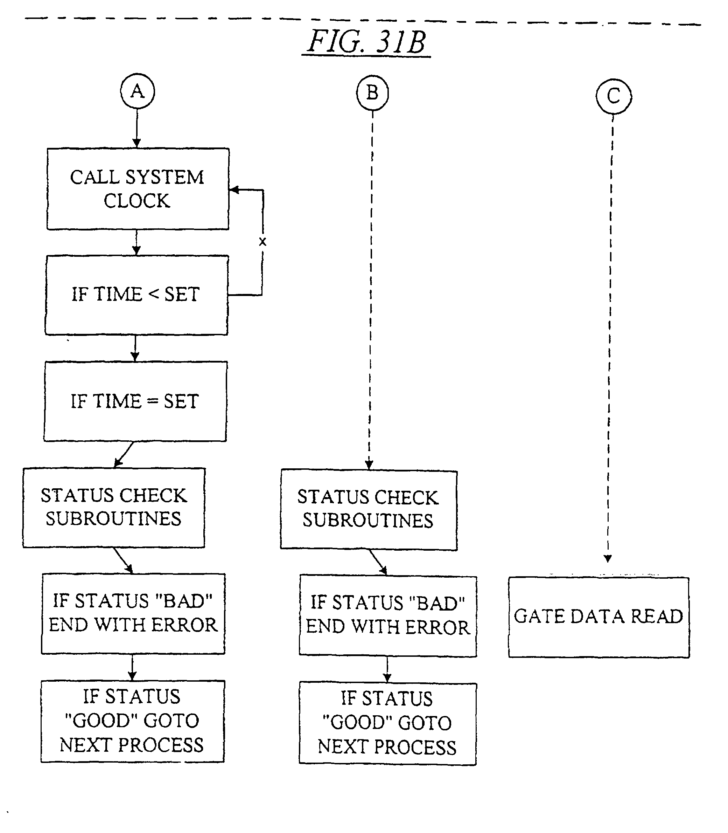

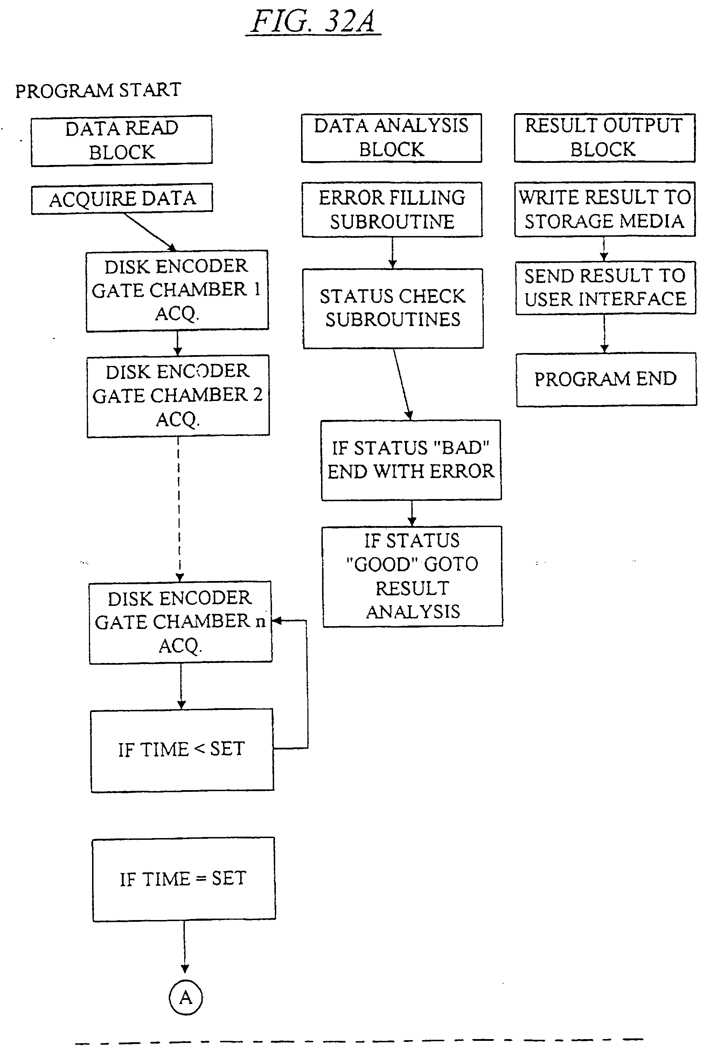

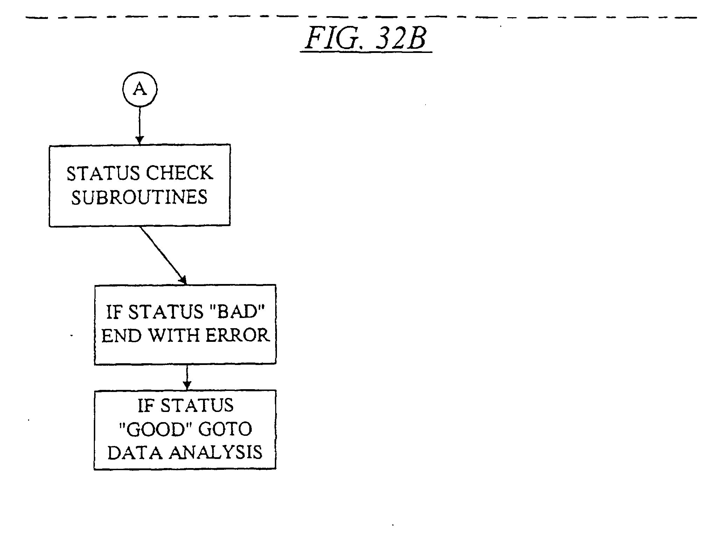

Figures 31 and 32 show one specific set of programmed steps for performing assays using the capillary microvalves disclosed above; other arrangements of steps within the program will be apparent to one of ordinary skill and readily integrated, for example, for sending signals to activate microvalves and other actuators. The program disclosed here consists of blocks in which different rotation rates are set for varying amounts of time, allowing for capillary valving, mixing, and incu.bation; mixing program blocks, which (for example) put the spindle motor through an oscillatory acceleration and deceleration, are possible but not shown. These program blocks consist of outputting commands to various electronic devices (motor, detectors, etc.) and reading data from devices, yielding a measure of device and process status. Provisions are shown in the program for halting the program if the status is "bad" (such as motor cannot reach appropriate speed, door to device cannot close, no power detected in light source for spectroscopic measurements.). This condition can lead to a program halt (as shown) or send the program back to the user for further instructions via the interface.

The program shown here additionally incorporates data acquisition, data analysis, and data output blocks. The particular acquisition process here involves using an encoded signal on the disk—for example, an optical signal associated with a detection chamber passing the detector—to gate acquisition of data. In this way, data is acquired for a specific time when detection chambers are in proximity to the detector. It is also possible to continuously take data and use features in that data—for example, the shape of the signal as a function of time, which might look like a square wave for an array of windows on an otherwise opaque disk—to determine what parts of the data are useful for analysis. Data analysis could include non-linear least-squares fitting, linear regression of data as function of time, or end-point analysis (data at an end-point time for a reaction), as well as other methods. Data output may be in the form of "yes/no" answers to the user interface, numeric data, and storage to internal or external storage media.

All component parts of this program need not be contained on the disk. For example, the program can be resident in the computer and designed to read the disk itself to obtain the rotation velocity profiles necessary for using the disk. All other aspects of the program—such as when and how to read and analyze data—can be part of a dedicated program or read from other media.

Analysis/test protocol data are descriptions of tests and analyses which can be performed with a disk. These data can be a simple as a title given, the disk, or can contain a detailed description of disk use, data analysis and handling, including test protocols and data analysis protocols. Analysis/test protocol programming is provided that can be used as systems-specified subroutines in more general software schemes, or can be fed into programmable logic so that the device can perform the desired analyses. Analysis/protocol descriptions are provided, as audio, video, text or other descriptions of analytic processes performed on disk, including background information, conditions for valid use, precautions, and other aspects.

Encryption and verification data/programming is provided to ensure the security of the programming and data generated in the analyses performed by the disk. Encryption/de-encryption routines are used to restricted access to data contained on the disk. Such routines also used in medical diagnostic applications.

System self-diagnostics are also provided. System diagnostics include diagnostic test results on detector function, status of reagent chambers, valves, heating elements, and other components, stored in disk-memory or written to the disk by a separate device used at the time of diagnostics.

Point-of-use information is encoded on the disk at its point-of-use (e.g. sample loading) in the form of video, audio, or text images, including, for example location, time and personnel. Also included in point of use information is test result data, recorded by the disk itself or by a disk player/reader at the time these procedures were performed.

Certain data are inherent to the disk and are accessible through the micromanipulation device. These include sample adequacy test data, which records the presence or absence of samples or reagents at appropriate reservoirs and other fluid handling regions of the disk, and can be accessed through external detectors and sensors. Valve status is also recorded, including the record of the change in valve status during a procedure performed in the disk. Valve status is determined, for example, by using magnetic pickups in the device applied to magnetic valve mechanisms; status can also be visible through optical windows on the disk. The presence of radioactive, chemical or biological contaminants on the external surface of the disk can be recorded upon detection by sensors comprising the device, optimally resulting in a warning message delivered to a user interface such as a display or print-out.

Disk data and information are stored using a variety of media, including both the recording medium of the disk material (i.e., reflective properties of an optically-read disk, most preferably a read/write CD-ROM) and by the device itself using electronic components. Information is encoded using conventional or modified technologies used for computer information storage. Video, audio, and text information is digitized using methods developed by the digital video, audio, and computer industries. Analog signals arising from test procedures, such as a signal observed in a photodiode detector or photomultiplier tube, are converted through analog-to-digital conversion regimes or may be supplied in raw or amplified form through external jacks for processing off-disk or off-device. Various embodiments of the disk manipulation device of the invention include the capacity to both read and write data to the disk or to use read-only data from any of these media types. Encryption and authentication codes can be used for security purposes. Disk data storage media include optical media, utilizing reflecting/non-reflecting flats and pits on a surface, using technology adapted from audio CD, CD-ROM, and "Laserdisc" technology, and barcodes. Magnetooptic and magnetic media are also within the scope of this aspect of the invention, using conventional computer magnetic storage media. Electronic data storage means are also provided, using the status of internal arrays of electronic components (FPGAs, PLAs, EPROM, ROM, ASICs, IC networks) for information handling. Chemical recording means, including simple chromatographic staining of a detector section or chamber of the device, is also disclosed to provide a simple visual record of a test result. This simple chemical recording means provides an avenue to at-home diagnostic without the need for an expensive device more sophisticated in capabilities than required to determine an assay amenable to simply the presence or absence of chemical markers.

## Software and Communications

Software providing the information and instruction set for microsystem performance, quality control, data acquisition, handling and processing , and communications is included within the scope of this invention. For the purposes of this invention, such software is referred to as "machine language instructions." Control and analysis software is advantageously provided in high-level languages such as C/C ++, Visual Basic, FORTRAN or Pascal. Drivers are provided for interface boards (either internal to the device or to a host computer interfaced with the device) which translates instructions on the host computer's bus into micromanipulator commands. Additionally, drivers for experiment-control software such as LabView may be created, again using conventional, industry-standard interface protocols. These applications are most preferably capable of being run on a number of popular computer platforms, including UNIX/Linux, X-windows, Macintosh, and SGI. Control and analysis can also be performed using dedicated chipsets and circuitry, ROM, and EPROM. For example, test validity can be insured (at least in part) through the use of ROM-based test procedures, in which all programming is performed at the point-of-manufacture without possibility of end-user corruption. Separate application software can also be developed so that data from a disk-player can be analyzed on non-controller platforms, using available applications (such as Excel, Clarisworks, SigmaPlot, Oracle, Sybase, etc.).

Because some applications of the disk technology disclosed herein involve important questions related to human health, disk diagnostic software must be able to analyze diagnostics of the disk, its contents (samples, reagents, devices), the player, and analysis software to ensure result validity. Types of information used by this diagnostic software include sample adequacy and flow, verification of disk format and software/test procedure compatibility, on-and off-disk software tests, quality control monitoring of disk manufacture (for example, channel placement and alignment), viability, positioning and functionality of on-disk and off-disk sensors and detectors, diagnostics of player communications and microprocessor, microprocessor/CPU, and power stability.

Diagnostics of mechanical and electronic components are performed in ways familiar to those proficient in the art. Software self-diagnostics are achieved using checklist/verification of software routines and subroutines to detect incompatibility with system hardware (from either the micromanipulation device or the disk) or with other components of system software.

Sample-related disk diagnostics include assays of flow, sample adequacy, and reagent adequacy, type and quality for the assay to be performed. Device-related disk diagnostics include checks of detector/sensor function, electronic components self-test, valve control, and thermal control tests. Software diagnostics provide self-testing of software components encoded in the disk or in the device, corruption safeguards, read-only and read-write tests. Disk format is also checked using disk diagnostics, ensuring that the disk format and assay type are properly read and are in agreement with the protocol held in the device memory.

On-disk software includes read-only software, available as ROM, specifically CD-ROM, for diagnostics, assay control and data analysis. Read -only software is designed for specific procedures and processes which cannot be altered and insure proper usage of the disk and fail-safe against corruption by the user. Software may also be embodied within the encoding medium (optical, magnetic, etc.) or an alternate medium (such as barcodes). Re-programmable software (such as FPGAs, PLAs, EPROMs, or IC arrays) can be re-programmed by the disk micromanipulation device or devices designed for this purpose. Similar types of software are alternatively provided on-device. In either case, a user-interface through keyboard, touchpad and/or display components of the device is provided.

Applications software is provided in read-only or re-programmable software formats. Included in this component of the fluidics micromanipulation apparatus of the invention is software that can be read from standard computer data storage medium. Examples include medical or analytic diagnostic programs reliant on integrated databases which are contained within disk or device memory, or that can be accessed from networked workstations, or access on-line services, such as a newsletter and news services, and software for the production and analysis of images, including pattern recognition, and statistical analysis software.

Integration of control and applications software can be made through the use of either a unique operating system developed for the disk and micromanipulator of the invention, or by adaptation of existing OS. Optimally, the OS uses authoring software to combine text, graphics, video and audio into an easy to use, "point and click" system. Such as OS could also provide an object-oriented environment or facsimile thereof (*e.g.*, LabView-based systems) for customizing programming by sophisticated users, as well as providing for the development of additional software by the disk reader/player manufacturer or independent software developers.

The OS can also be chosen to allow design of disks and disk-based assays. Mechanical design, including simulation of rotational dynamics and stability and fluid flow simulation are advantageously encompassed in a disk design software package.

Communications aspects of the invention include hardware and software embodiments relating to data input and output from a user or to remote control and analysis sites. Hard-wired communications features include high-speed data-, video- or image-transmission and communication through local busses (*e.g.*, a VGA bus for video signals) and conventional hard-wired interfaces (*e.g.*, RS-232, IEEE-488, SCSI bus), Ethernet connections, Appletalk, and various local area networks (LANs). Telecommunications devices include cellular transceivers for short-range communications, radio-frequency and micro-wave transceivers for long-range communications, and internal or external modem for manual or automated telephone communications. Video in/out ports, analog out-lines for data transmission, input jacks for input of analog signals from other instruments, and optical and infra-red communications ports are also provided for communications with peripheral instruments.

**Configurations of the Micromanipulation Apparatus for Certain Applications**

The micromanipulation device includes various combinations of hardware and software as described above. Figure 15 is an illustration of the general combination of communication, device, detection, and control instrumentation in a device. Certain applications may not have certain features, for example, portable units may not have graphical user interfaces. The micromanipulation device can be a "stand-alone" device, or a peripheral instrument to a larger assemblage of devices including, for example, computers, printers, and image-processing equipment, or a host for peripheral elements such as control pads, data entry/read-out units (such as Newton-type devices or equivalent), or an integrated system. The device in all embodiments comprises hardware to rotate the disk at both steady and variable rates and systems for monitoring rotation rate. The device can also include devices to initiate sample and disk diagnostics, perform "external" tests and detection as described herein, initiate sample and disk diagnostics, perform "external" tests and detection as described herein, initiate analyses on-disk through specific actuators such as valves, read disk-inherent information and information encoded in the disk or other data/information storage media information, and in some applications write information to the disk.

Additional elements in the device, including system control, data processors, array of assay processors, external detectors, external actuators, assay out and data out lines, communications, and software, are device-and/or application-specific.

For example, in a "point-of-use" portable or home-use application, sample loading is followed by initiation of the player's program. System control can be provided by front-panel controls and indicators which can access a variety of programs stored in the disk or the device. These "hard-wired" programs utilize controller circuitry to read or read/ write operations from or to disk or memory, and/or perform tests using external devices. The device can be designed for performance of a single procedure, or can be pre-programmed to perform a set of procedures or multiple embodiments of the same procedure using a single disk. Device actuation is optimally obtained with the pressing of a single button. These processor(s) and data processors(s) of this type of device comprise circuitry and chipware designed to process analysis data (assay processor) and encoded data (data processor). Information from these processors can be available for output to the user on a front-panel or video display and can also be used internally to ensure correct operating conditions for the assay. This internal information processing can include the results of systems diagnostic tests to insure disk identity and test type compatibility; the presence of reagent and sample as determined through light absorption through a detector port scanning reagent and sample reservoirs; the presence of contamination detected before testing begins, and the results of self-diagnostics on external detectors and actuators. These results are used by the system controller to determine whether the requested test can be performed.

After loading and activation, analysis results can be stored internally in electronic memory or encoded upon the disk. The results of these analyses and procedures are then routed to the front-panel display (flat-panel LCD, etc.) using appropriate video drivers. Processed assay data can also be routed to one of many standard digital I/O systems including RS-232, RS-232C, IEEE-488, and other systems familiar-from digital I/O and interface systems. Similarly, encoded disk data can be routed to the audio/visual display. Raw analog signals can also be switched to one or more external jacks for off-device storage or processing.

An embodiment of the least technically sophisticated device is a portable unit no larger than a portable audio CD player consisting of disk-drive, controllers and selectors for programmable or pre-programmed angular acceleration/deceleration profiles for a limited number of procedures. Such a device is advantageous for on-site toxic-chemical/ contamination testing. Analyte to be tested is introduced to the disk, which is inserted into the player and the appropriate program chosen. Analysis results are stored on the disk, to be later read-out by a larger player/reader unit, and/or displayed immediately to the user. Results can also be stored as the inherent state of an indicator (positive/negative status of litmus paper in different cuvettes, for example), with no other data collection or analysis performed by the device. This data would be accessed by a larger player/reader or by other means outside the field-work environment. Information about the location, time, and other conditions of sample collection are entered through the user interface.

Another embodiment is a stand-alone device with active communications capabilities and greater functionality. An exemplary application for such a device is as a home blood-assay unit. This device is used by an individual placing a drop of blood on the disk, inserting the disk, and initiating the assay, preferably simply by pressing a single button. One or more analytical procedures are then performed. Assay data is transferred to software which performs the requisite analysis, either on-disk or within the device. The device can also be permanently or temporarily attached to the home-telephone line and automatically transmit either raw or reduced data to a computer at the central location which is used to analyze the data transmitted, compare the data with accepted standards and/or previous data from the same patient, make a permanent record as part of a patient's device a confirmation of receipt of the data, perhaps the data analysis, and advice or suggested/recommended course of action (such as contacting the physician).

A desk-top peripheral/host application station constitutes a device as described above with the ability to accept instructions from and respond to a host computer over one of many possible data-protocols. The system is capable of acting as host or can transmit data to peripherals or other networked devices and workstations. Remote accessing of pre-programmed functions, function re-prograrnming, and real-time control capabilities are also provided.

Yet another embodiment of this application is a centralized or bedside player/reader device with associated software located as a nurses' station in a hospital. As tests are performed on disks, the information is relayed to a physician by telephone, facsimile or pager *via* short-range transceiver. Patient identity can be entered at the time of sample collection by the use of bar codes and light pens attached to the device, providing the advantage of positive patient/sample identification.

The device can also be provided having the above-capabilities and functionalities and in addition having an interface with an integrated computer having high-resolution graphics, image-processing and other features. The computer provides control of the device for performing the functions described above for the peripheral system, while physical integration greatly increases data-transmission rates. Additionally, the integrated system is provided with extensive analysis software and background databases and information. Disk-storage cassettes of carousels are also an advantageous feature of such system. An integrated system of this type is useful in a large, analytical laboratory setting.

A self-contained system is useful for applications in isolated environments. Examples include devices used in remote or hostile setting, such as air, water and soil testing devices used in the Arctic for environmental purposes, or for use on the battlefield for toxic chemical detection.

The microsystem platforms provided by the invention are also useful for preparing samples for other analytical instruments, such as mass-spectrometers, gas chromatographs , high pressure liquid chromatographs, liquid chromatographs, capillary electrophoresis, inductively-coupled plasma spectroscopy, and X-ray absorption fine-structure. In some application, the final product is removed from the disk to be analyzed.

Samples can be pre-concentrated and purified on the device by incorporating aqueous two-phase separation systems. This can be done, for example, by mixing two phases which separate from each other based on thermodynamic differences like polyethylene glycol (PEG) and dextrans; biopolymers are usefully separated using this method. Alternatively, environmental tests such as colorimetric analysis can be enhanced by incorporating cloud-point separations to concentrate and enhance optical signals. In addition, small scale counter-current chromatography can be performed on the device (*see*, Foucault, 1991, *Anal.* Chem. 63: PAGE). Centripetal force on the disk can be used to force different density fluids to flow against each other, resulting in separation of components along a density gradient to develop the chromatogram.

**Applications and Uses**

The microsystem platforms and micromanipulating devices that make up the fluidics micromanipulation apparatus of the invention have a wide variety of microsynthetic and microanalytic applications, due to the flexibility of the design, wherein fluids are motivated on the platform by centripetal force that arises when the platform is rotated. What follows is a short, representative sample of the types of applications encompasses within the scope of the instant invention that is neither exhaustive or intended to be limiting of all of the embodiments of this invention.

The invention is advantageously used for microanalysis in research, especially biological research applications. Such microanalyses include immunoassay, *in vitro* amplification routines, including polymerase chain reaction, ligase chain reaction and magnetic chain reaction. Molecular and microbiological assays, including restriction enzyme digestion of DNA and DNA fragment size separation/fractionation can also be accomplished using the microsystem disks of the invention. Microsynthetic manipulations, such as DNA fragment ligation, replacement synthesis, radiolabeling and fluorescent or antigenic labeling can also be performed using the disks of the invention. Nucleic acid sequencing, using a variety of synthetic protocols using enzymatic replacement synthesis of DNA, can be performed, and resolution and analysis of the resulting nested set of single-stranded DNA fragments can be separated on the disk, identified and arranged into a sequence using resident software modified from such software currently available for macroscopic, automated DNA sequencing machines. Other applications include pH measurement, filtration and ultralfiltration, chromatography, including affinity chromatography and reverse-phase chromatography, electrophoresis, microbiological applications including microculture and identification of pathogens, flow cytometry, immunoassay and other heretofore conventional laboratory procedures performed at a macroscopic scale.

An illustrative example is immunoassay. While there exist a multiplicity of experimental methodologies for detecting antigen/antibody interactions that are in research and clinical use at the present time, the most robust immunoassay protocols involve "sandwich"-type assays. In such assays, an immobilized antibody is presented to a sample to be tested for the antigenic analyte specific for the immobilized antibody. A second antibody, specific for a different epitope of the same antigen is subsequently bound, making a "sandwich" of the antigen between the two bound antibodies. In such assays, the second antibody is linked to a detectable moiety, such as a radiolabel or fluorescent label, or an enzymatic or catalytic functionality. For example, horseradish peroxidase or alkaline phosphatase are used to produce a color change in a substrate, the intensity of which is related to the amount of the second antibody bound in the sandwich,

An example of a disk adapted for performing such an immunoassay is shown in Figure 17Q. In this embodiment, the secondary antibody is linked to alkaline phosphate (AP). The presence and amount of AP activity is determined by monitoring the conversion of one of the following exemplary substrates by the enzyme colorimetrically: $\beta$-naphthyl phosphate converts to an insoluble azo dye in the presence of a diazonium salt; 5-bromo-4-chloro-3-indolyl phosphate is converted to 5,5'-dibromo-4-,4'-dichloro indigo in the presence of cupric sulfate; or 4-methylumbelliferyl phosphate is converted to 4-methylurnbelliferone, which emits light at 450nm.

In one exemplary embodiment, the reaction chamber comprises an antibody specific for an antigen, where the antibody is immobilized by adsorption of the antibody to the reaction chamber. Contiguous with the reaction chamber is advantageously placed a reagent reservoir containing a second antibody, this antibody being linked to an enzyme such as alkaline phosphate. Sample, which may contain an antigen of interest that is specifically recognized by the above antibodies, is loaded at an inlet port. The disk is spun to first introduce the sample into the reaction chamber containing immobilized antibody, followed by introduction of the second antibody into the reaction chamber after a time sufficient to saturate the immobilized antibody with antigen to the extent the antigen is present in the sample. Alternatively, the sample may be contacted with the second antibody, allowed to interact, then introduced into the reaction chamber. Incubation of the sample with antibody is performed without spinning for about 1 minute. After each incubation, washing buffer from a buffer reservoir is spun into the reaction chamber in order to remove unbound antibody. For alkaline phosphatase assays, solutions of 2mg/mL *o*-dianisidine in water, 1mg/mL $\beta$-naphthyl phosphate in 50mM boric acid/50mM KCI (pH 9.2) buffer and 100 mM magnesium chloride are delivered to the reaction chamber in the appropriate amounts. The extent of enzyme-linked, secondary antibody binding is evaluated by detection of a purple precipitate using a photodiode or CCD camera.

A disk configured for immunoassay applications is shown in Figure 17R for illustration.

In an alternative embodiment of the immunological assays of the invention, the invention provides a means for identifying and quantitating the presence and number of particular cells or cell types in fluids, most preferably biological fluids such as blood, urine, amniotic fluid, semen and milk. In these embodiments of the invention, the microsystems platform comprises a chamber or solid surface on the disk that is prepared to selectively bind the particular cell or cell type. After attachment of the cells to the surface, non-specific binding cells and other components are removed by fluid flow (washing) or centrifugal force (comprising the inertial flow of fluid in response to the centripetal acceleration of the disk). The cells of interest that remain attached to the microplatform surface or chamber are then detected and quantified using means including but not limited to microscopic, spectroscopic, fluorescent, chemiluminescent, or light-scattering means. The invention also provides such cells attached to a specific surface for toxicity monitoring, such as metabolic monitoring to determine the efficacy of bioactive drugs or other treatments. Ordered arrays of such surface are provided in certain embodiments to facilitate a complete determination of the purity and sterility of certain biological samples, and for cell cytometric and cytometry applications.

The surface or chamber of the disk for specific binding of the particular cells or cell types of interest is prepared to provide specific binding sites therefor. Typically, an antibody, preferably a monoclonal antibody, is attached to the surface or chamber, wherein the antibody is specific for a cell surface antigen expressed on the cell or cell type of interest. Alteratively, a ligand specific for a cell surface receptor expressed on the particular cell or cell type of interest is used to provide a specific attachment site. Arrays of specifically prepared surfaces or chambers are provided on certain embodiments of the disk. Surfaces and chamber are provided, for example, by contacting the surface with a solution of an appropriate antibody. In the practice of these preparation methods, contact of the surface with the antibody is followed by contacting the surface with a non-specific blocking protein, such as bovine serum albumin. Antibodies and blocking proteins can be contacted with the surface or chamber using a piezoelectrically driven point head (such as are used in ink-jet printing applications) which can be advantageously used for this purpose. Alternatively, screen printing, or spraying the antibody solution on the chamber or surface using an airbrush can be employed. These methods are preferred in preparing surfaces and chambers in the 0.1 - 10mm scale. In additional alternatives, microlithographic and microstamping techniques can be used to prepare the surface or chamber.

In the practice of the invention, a biological or other fluid sample containing the particular cell or cell type of interest is applied to the prepared surface or chamber and allowed in contact with the prepared surface or chamber for a time sufficient to allow specific binding of the cells or cell types to the surface. As contact with the surface may be inhibited by cell settling properties in the volume of the fluid, chambers and surfaces having minimized height transversely through the microsystem platform are preferred.

Non-specific cell binding is minimized or eliminated from the chamber or surface by washing the surface or chamber with a fluid amount sufficient to remove such non-specific binding. Washing is accomplished by simple bulk flow of fluid over the surface or chamber, or by centrifugation.

After washing, cells that remain attached to the surface or chamber are detected and counted. In a preferred embodiment, detection and counting is achieved using fluorescence microscopy. In the practice of the invention, specific dyes can be used to provide a fluorescence signal for any live cells remaining on the disk. The dye can be added directly to the surface or chamber, for example using a membrane-permeant dye, such as acetoxy-methyl ester dyes. Alternatively, specific antibodies can be linked to such dyes. Dyes can be added to the biological fluid comprising the cells prior to introduction onto the microsystem platform, or such dyes can be contacted with the cells *in situ* on the disk. The presence of the cells is detected using a fluorescence detector comprising a light source, a source filter, a dichroic filter or mirror, an emission filter, and a detector such as a photomultiplier tube.

In another example, thin-layer chromatography is accomplished on a microplatform disk comprising 100 pm square cross-section channels radiating outward from the center of the disk. Each channel is filled with separation substrate, which typically contains a binder material (0.1-10%) such as starch, gypsum, polyacrylic acid salts and the like, to provide mechanical strength and stability. (The use of such compounds in conventional TLC applications is discussed in Poole *et al.,* 1994 *Anal. Chem.* 66: 27A). Sorbents are also included in the materials comprising the separation channels, including for example cellulose, polyamide, polyethylene powders, aluminum oxide, diatomaceous earth, magnesium silicate, and silica gels. Such substrates can be modified for example with silanizing molecules, such as dimethyl-, ethyl;octa- and 3-aminopropyl-silanes. Preferentially the separation substrate contains sorbent-impregnated fiber glass or PFTE matrices.

Sample is loaded *via* a port located proximal to the center of rotation of the disk. Upon spinning the disk, a mobile phase is allowed to flow outward through the separation substrate, carrying sample components to the periphery of the disk at characteristic rates. The mobile phase can be chosen from a multiplicity of appropriate solvent systems. including hexane, methanol and dichloromethane. Choice of a particular solvent depends on the nature of the disk material, the separation substrate and the components of the sample to be separated. Similarly, the choice of visualization reagents used to detect separated sample components are specific for the substances separated. For example, ninhydrin is used to detect amino acids; antimony chloride is used plus potassium permanganate for hydrocarbons; sulfuric acid plus anisaldehyde for carbohydrates; and bromine for olefins. Imaging of separation channels after separation is achieved using a CCD camera. A disk configured for thin layer chromatography applications is shown in Figure 17R for illustration.

Medical applications using the microsystems of the invention are abundant, and robust. Various embodiments of the invention provide for at-home, bedside, hospital and portable devices for rapid analysis of blood components, blood gases, drug concentrations, metabolities and infectious agents. In at-home monitoring embodiments, the invention provides a simple, easy-to-use consumer friendly device requiring a patient to add a blood droplet, urine sample or saliva sample to a specific application region on the disk, insert the disk in the device and start the device by pushing a button. In a hospital setting, both bedside and clinical laboratory embodiments are provided, wherein the bedside embodiment is advantageously linked electronically to a central processing unit located, for example, at a nurses' station, and the clinical laboratory embodiment comprises a medical reference library for rapid, automated diagnostics of patient sample. The medical applications of the instant invention include blood testing (such as monitoring platelet counts in patients being treated with chemotherapeutic drugs); immunoassay for metabolites, drugs, and other biological and other chemical species; vaccine efficacy monitoring; myeloma or lupus erythematosus monitoring; determination of blood glucose and/or ketone body levels in patients with diabetes; automated cholesterol testing: automated blood drug concentration determination; toxicology; monitoring of electrolytes or other medically-relevant blood component at a patient's bedside; sepsis/endotoxin monitoring; allergy testing; and thrombus monitoring.

The invention also provides analytical instruments for environmental testing, industrial applications and regulation compliance. Portable, preferably hand-held embodiments, as well as more extensive embodiments, installed as part of an industrial quality control regime, are provided. Applications for these embodiments of the invention include analyte testing, particularly testing for industrial effluents and waste material, to be used for regulatory compliance; and quality control of industrial, most advantageously of human consumable items, particularly pharmaceuticals and specifically endotoxin determinations. Application for testing, mixing and evaluating perfumes and other complex mixtures are also within the scope of the invention.

The invention also provides chemical reaction and synthesis modeling, wherein a reaction scheme or industrial production regime can be tested and evaluated in miniaturized simulations. The invention provides for cost-effective prototyping of potential research, medical and industrial chemical reaction schemes, which can be scaled to macroscopic levels after analysis and optimization using the microsystems platforms of this invention.

A variety of other applications are provided, including microsynthetic methods and forensic applications.

The following Examples are intended to further illustrate certain preferred embodiments of the invention and are not limiting in nature.

## EXAMPLE 1

### Fabrication of Microplatform Disks for Chemical Analysis, Synthesis and Applications

Microplatform disks of the invention are fabricated from thermoplastics such as teflon, polyethylene, polypropylene, methylmethacrylates and polycarbonates, among others, due to their ease of molding, stamping and milling. Alternatively, the disks can be made of silica, glass, quartz or inert metal. A fluid handling system is built by sequential application of one or more of these materials laid down in stepwise fashion onto the thermoplastic substrate. Figures 17A through 17E are a schematic representation of a disk adapted for performing DNA sequencing. Disks of the invention are fabricated with an injection molded, optically-clear base layer having optical pits in the manner of a conventional compact disk (CD). The disk is a round, polycarbonate disk 120mm in diameter and 100 pm thick. The optical pits provide means for encoding instrument control programming, user interface information, graphics and sound specific to the application and driver configuration. The driver configuration depends on whether the micromanipulation device is a hand-held, benchtop or floor model, and also on the details of external communication and other specifics of the hardware configuration. This layer is then overlaid with a reflective surface, with appropriate windows for external detectors, specifically optical detectors, being left clear on the disk. Other layers of polycarbonate of varying thickness are laid down on the disk in the form of channels, reservoirs, reaction chambers and other structures, including provisions on the disk for valves and other control elements. These layers can be pre-fabricated and cut with the appropriate geometries for a given application and assembled on the disk. Layers comprising materials other than polycarbonate can also be incorporated into the disk. The composition of the layers on the disk depend in large part on the specific application and the requirements of chemical compatibility with the reagents to be used with the disk. Electrical layers can be incorporated in disks requiring electric circuits, such as electrophoresis applications and electrically-controlled valves. Control devices, such as valves, integrated circuits, laser diodes, photodiodes and resistive networks that can form selective heating areas or flexible logic structures can be incorporated into appropriately wired recesses, either by direct fabrication of modular installation onto the disk. Reagents that can be stored dry can be introduced into appropriate open chambers by spraying into reservoirs using means similar to inkjet printing heads, and then dried on the disk. A top layer comprising access ports and air vents, ports or shafts is then applied. Liquid reagents are then injected into the appropriate reservoirs, followed by application of a protective cover layer comprising a thin plastic film.

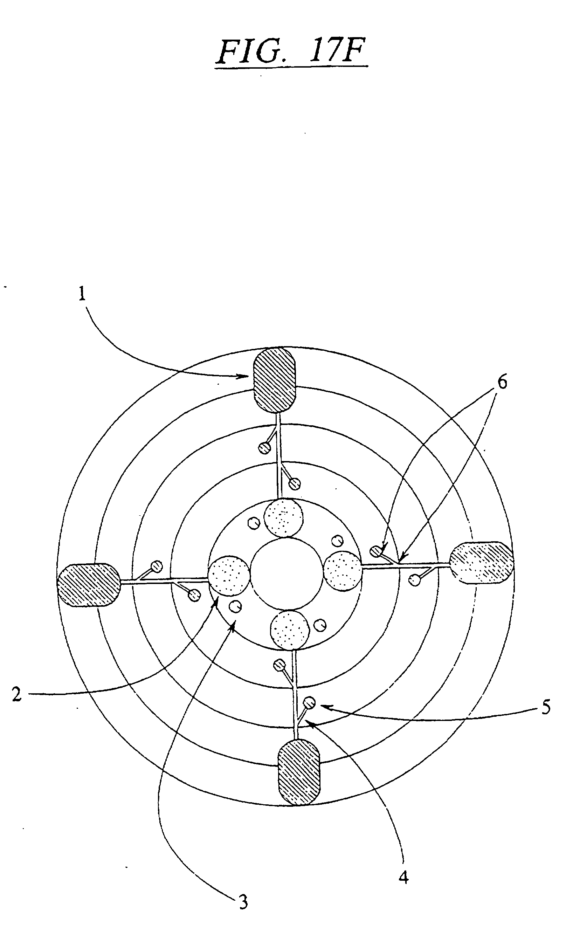

A variety of other disk configurations are disclosed in Figures 17F through 17P, adapted for particular applications as described in the Figure legends.

**EXAMPLE 2**

**Blood Composition Determination**

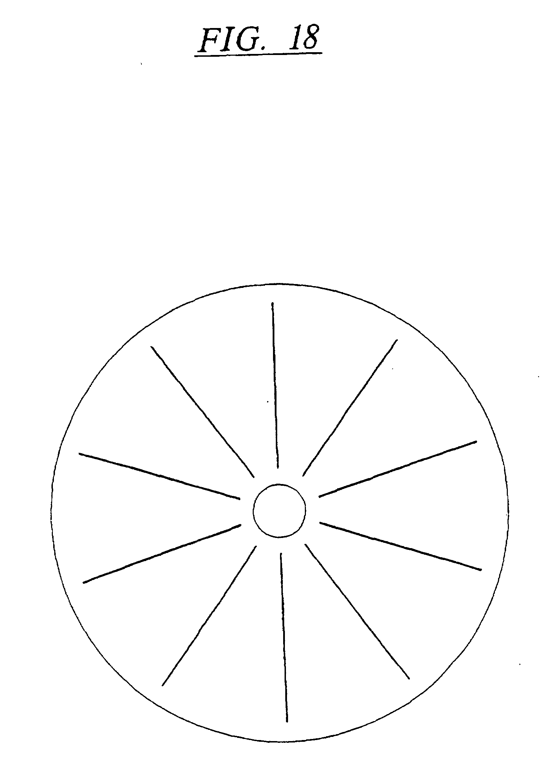

Blood composition can be determined via hematocrit analysis using an analytic microplatform disk prepared as described in Example 1 held within a device comprising a microchannel layer with a number of microchannels as shown in Figure 18. The microchannel layer is 100pm thick and treated with heparin to prevent coagulation during the assay. The blood sample to be analyzed is drawn by capillary action into a channel arranged perpendicular to the direction of rotation, as shown in Figure 18; a number of such channels may be arranged radially on the disk. When all samples to be tested have been drawn into the channels, the disk is spun at a speed of 8000 to 10,000 rpm to effect sedimentation of erythrocytes within the channel. Once centrifugation has been performed for an appropriate time (3 to 5 minutes), the hematocrit of each sample is determined simultaneously by stroboscopic interrogation of each of the channels using a conventional CD laser system in the device described above. When the laser passes the boundary of erythrocytes, the change in light scattering pattern detected by the photodiode detector is converted into a hematocrit value based on a standardized set of light scatter/hematocrit information stored in the internal processor and memory of the device. Alternatively, the raw information is relayed via a infrared port or hard-wired interface to a microprocessor for analysis. Such a central microprocessor is on site or in the alternative at a centralized location, such as a nursing station in a hospital or in a medical center connected to the hematocrit determining device by telephone or other dedicated connection. Hematocnt can be determined by untrained individuals (including patients) by the simple application of a blood droplet produced by lancet onto the disk, followed by the simple application of the device and automated hematocnt analysis and data processing on site or transmission to a central location of trained medical personnel. This embodiment of the invention provides for chronic monitoring of patients having hematopoietic proliferative disease (such as leukemia, lymphoma, myeloma, and anemias).

In addition, blood gas can be determined using the above device in combination with a disk having integrated electrodes embedded within the hematocrit channel, or having a separate channel devoted to blood gas determination on the hematocrit disk. Blood oxygenation ($PO_2$) is determined by a Clark-type electrode consisting of a thin Cr-Au cathode and an Ag-AgCl wire anode. The amount of carbon dioxide in the blood is determined by a Severing-type electrode using an ISFET (a type of field effect transistor) as a pH monitor. Blood pH is determined with the use of a $Sl_3N_4$ gate ISFET with a reference electrode consisting of a liquid junction and an Ag-AgCl wire electrode. Further examples of such analytical methods for determining blood gases, electrolyte concentration and other information advantageously performed using the hematocrit disk or alternate variations of this disk are described as modifications of the macroscopic-scale methods of Shoji & Esashi (1992, *Sensors and Actuators B* 8: 205).

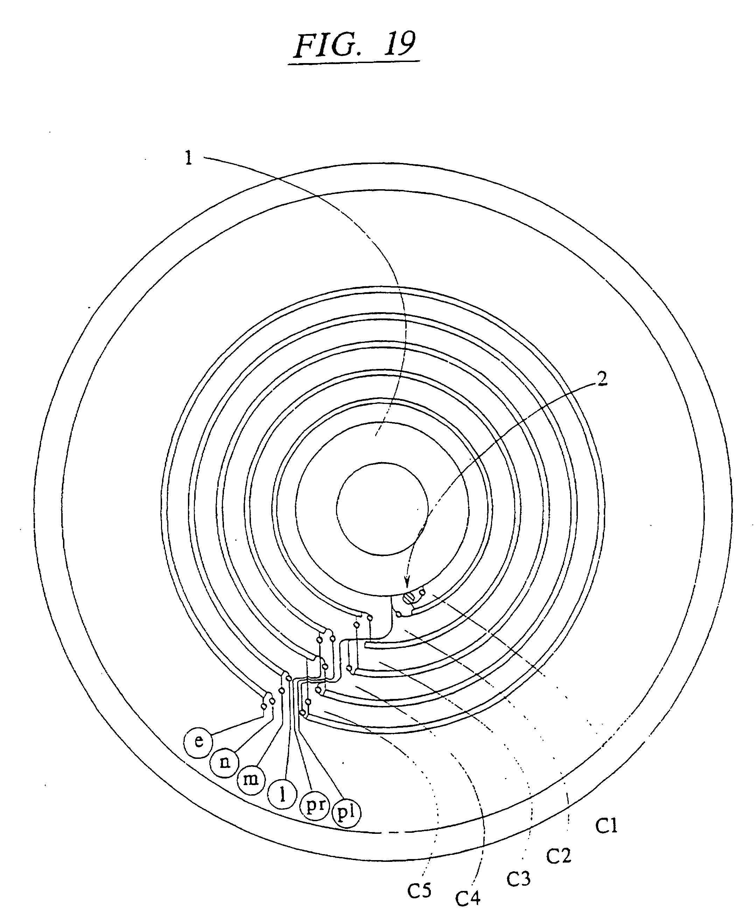

Blood analysis are also performed using split-flow thin cell (SPLITT) fractionation as described by Bor Fuh *et al.* (1995, *Biotechnol. Prog.* 11: 14-20). A schematic representation of a disk configured for SPLITT analysis is shown in Figure 19. This process can produce enriched fractions of proteins and lipoproteins, platelets, erythrocytes, lymphocytes, monocytes, and neutrophils. A non-contiguous circular channel is etched into the disk incorporating a thin wall at either end (Figure 19), the inlet stream splitter. Sample and carrier streams are introduced at opposite sides of one end, and the chamber is spun in that direction. Within the spinning chamber two distinct splitting planes are set up based on hydrodynamic forces, the inlet splitting stream (ISP) and the outlet splitting stream (OSP). The ISP is adjustable by regulating the ratio of the sample to the carrier streams. Depending on the method of sample input two distinct separation modes are possible, the equilibrium and transport modes.

In the equilibrium mode separation is based on the equilibrium of the components in relation to the applied centrifugal field. Separation is optimized by adjusting the outlet flow ratio. The enriched fraction can then be collected from either side of the outlet stream splitter. In the transport mode the components are introduced as a thin lamina above the ISP. Based on the difference in sedimentation coefficients components with a higher transport rate are selectively directed to the opposite sides of the outlet valves at the orifices. Variable flow valves are described elsewhere in this document. In another embodiment each SPLITT chamber may be dedicated to the separation type required of it, ISP or OSP, and the flow regulated by fixed flow-restriction orifices.

In order to fully fractionate blood into the above-identified. fractions, five separations, each yielding two fractions, are performed. One embodiment of the microsystems disk of the invention used for this type of fractionation is shown in Figure 19. Five concentric SPLITT cells are illustrated in this Figure, labeled C1, (close to the center of rotation) through C5 (toward the periphery). A blood sample is introduced into C1 and subjected to a transport mode separation by rotating the disk at the appropriate speed. Platelets and proteins (fraction 1) are fractionated toward the center of rotation and blood cells (fraction 2) move toward the periphery. Fraction 1 is routed to the inlet of C2 while fraction 2 is routed to C3 by the opening and closing of appropriately-positioned valves on the disk. The fractions are then subjected to transport and equilibrium mode separations respectively. Using these techniques, Fraction 1 results in platelets toward the center of rotation and proteins toward the periphery. Fraction 1 results in platelets toward the center of rotation and proteins toward the periphery. Fraction 2 yields fractions 3 and 4, consisting of lymphocytes and monocytes toward the center of rotation and erythrocytes and neutrophils toward the center of rotation and monocytes toward the periphery. Fraction 4 yields neutrophils toward the center of rotation and erythrocytes toward the periphery. Thus, fractionation of blood into five isolated components is achieved.

The activity of enzymes in the protein fraction can be determining using immobilized enzymes (Heineman, 1993, *App. Biochem. Biotech.* 41: 87-97). For example, blood-specific enzymes (such as glucose oxidase, alkaline phosphatase, and lactate oxidase) can be immobilized in polyvinyl alcohol (PVAL). Lactate oxidase is immobilized on platinized graphite electrodes by sandwiching a thin layer of enzyme between two layers of PVAL. The sensor responds to lactate by the electrochemical oxidation of hydrogen peroxide generated by the enzyme-catalyzed oxidation of lactate that diffuses into the network. The current produced is proportional to the concentration of peroxide, which in turn is proportional to the concentration of lactate. This sensor has been shown to be sensitive to lactate concentrations ranging from 1.7-26 $\mu$M.

Upon separation, each fraction is interrogated by detection systems to determine the relative components of the fractions. Alternatively, each fraction can be removed from the disk through an outlet port for further study off-device. For example, each fraction can be subjected to simple counting by passing the cells in a thin steam past two electrodes comprising a resistance monitor. As a cell passes through the electrodes a corresponding rise in resistance is monitored and counted. These data are then integrated relative to a standard set of particles distributed according to size to determine the relative number of each cell type in the original sample.

The fractions can be subjected to fluorescent antibody staining specific to each cell type. The cells are held in place by micromachined filters integral to the channels (U.S. Patent No. 5,304,487), stained and washed on the disk. The resulting labeled cells can then be quantified as a function of the degree of fluorescent staining associated with the cells.

## EXAMPLE 3

## DNA sizing and mutation detection

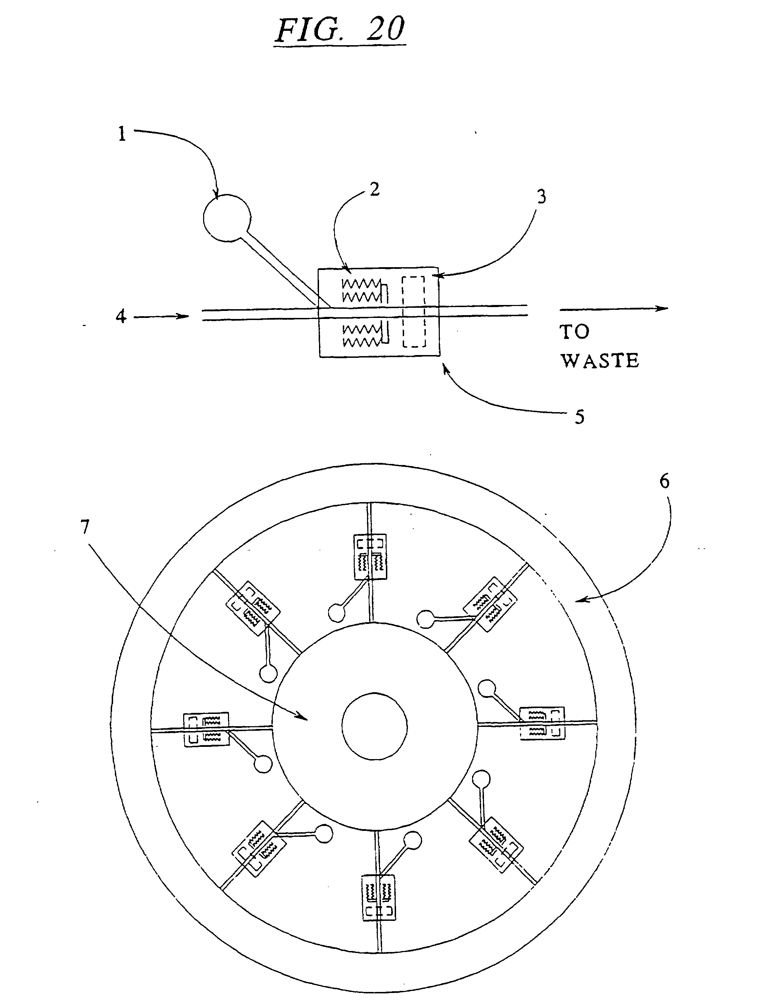

DNA sizing and detection of specific mutations in DNA at a particular site are carried out using double stranded melting analysis with a disk prepared according to Example 1 and illustrated in Figure 20. A DNA meltometer (as described in co-owned (International Application, Publication No. WO 95/25815) is advantageously incorporated into the structure of the disk of Example 1. The DNA meltometer technique takes advantage of the fact that the denaturing point of a DNA duplex is dependent upon the length, the base composition, and the degree of complimentary of the two strands in the duplex. A denaturing point may be determined in relation to some physical state of the molecule (such as temperature or the concentration of a denaturing chemical such as urea or formamide), and a set of standard conditions employed, the information derived from which can be stored in the microprocessor and/or memory of the device. In order to size any particular DNA duplex, one strand is immobilized on the disk by attaching it to a streptavidin coated bead. The bead is retained by a filter machined in to the channel (*see* U.S. Patent 5,304.487). Alternatively, the bead can be a paramagnetic bead retained in the channel by application of a magnetic filed using a permanent magnet incorporated into the disk of positioned in proximity to the channel. An electromagnet can be used. The electromagnet can be incorporated directly into the disk and actuated by application of 0.8volt DC at 500mA. The other strand is labeled, typically using a fluorescent dye or a radioactive isotope. Alternatively, the distinct optical properties of the DNA molecule itself (*i.e.,* hyperchromicity) are detected using unlabeled DNA molecules by monitoring absorbence at 260nm. Although this aspect of the method requires a more sophisticated device to generate and detect ultraviolet light, user preparation of the DNA is minimized and the cost of DNA preparation per sample greatly reduced. In the practice of the method of the invention, the immobilized, labeled duplex is placed on the disk and subjected to a flow stream of a buffered solution contained on the disk. During the development of the flow stream, the DNA is further subjected to a controlled denaturing gradient produced in the flow stream by the gradual addition of denaturant to the DNA. With an effective radius of 3.5" (85.9 mm) and a rotational speed of 600 rpm, a flow rate of 10$\mu$L/min can be generated in a channel 100$\mu$m in diameter. Four buffer reservoirs each containing 300$\mu$L can be incorporated into each quadrant of the disk (800$\mu$m deep extending from a position at a radius of 25mm to 50mm). At 10$\mu$L/min, this will allow a melting ramp of 30 min. Each duplex dissociates at a characteristic concentration of denaturant in the gradient, and can be identified in comparison with standards the denaturant profile information of which is stored in the microprocessor and/or memory of the device. Denaturation is detected by interrogation downstream of the melting chamber, using the appropriate detecting means (photooptical means for ultraviolet absorption or fluorescence detection, or radioisotope detectors (Geiger-Mueller counters) for DNA stands labeled with radioisotopes).

Exemplary of the uses the disks and devices of this aspect of the invention is the detection,, identification and size determination of DNA fragments produced by polymerase chain reaction or magnetic chain reaction (the latter disclosed in U.S. Patent No. 5,545,540 issued August 13, 1996 to Mian). Amplification is carried out using one primer labeled with a detectable label such as a fluorescent dye or radioisotope, and the other primer is covalently attached to a molecule that permits immobilization of the primer (e.g., biotin). After amplification (either off-disk or on the disk as described in more detail in Example 4 below), the labeled, biotinylated duplex DNA product fragment is attached to a solid support coated with streptavidin, for example, by movement of the amplification reaction mixture into a channel or compartment on the disk wherein the walls are coated with streptavidin, or by movement of the amplification mixture into a compartment on the disk containing a binding matrix such as Dynal M-280 Dynabeads (polystyrene coated paramagnetic particles of 2.8μm in diameter). Standardized size markers are included in the post-amplification compartment in order to provide a reference set of DNA fragments for comparison with the amplification product fragments. In this analysis, a number of different duplex DNA molecules from either a multiplex amplification reaction or a number of separate amplification reactions may be sized simultaneously, each fragment or set of fragments being distinguished from others by use of reaction- or fragment-specific detectable labels, or differences in some other physical property of the fragments. For amplifications performed off-disk, beads attached to the fragment are loaded into a channel on the disk capable of retaining the beads (such as size exclusion, "optical tweezers" or by magnetic attraction). In the latter embodiment, the magnetic retention means (permanent magnets or electromagnets) are either integral to the disk, held on second disk spinning synchronously with the first, or placed on the device so as to immobilize the DNA fragments in the appropriate compartment.

DNA size analysis is also performed essentially as described above, whereby the retained particles are subjected to a thermal denaturing gradient. For a thermal gradient used to denature the bound DNA fragments, a Peltier heat pump, direct laser heating or a resistive element is used to increase the temperature of the binding compartment through the denaturation range by the gradual addition of thermal energy. As above, a flow rate of 10μL/min can be generated in a channel 100μm in diameter, allowing a melting ramp of 30 min. The compartment is also subjected to a flow stream as described above to elute the denatured, labeled stands from the binding/melting chamber. Downstream from the binding/melting chamber are appropriate means for detecting DNA fragment denaturation, such as laser excitation at the resonant frequency of the dye label and photodiode detection. The strength and corresponding temperature of the raw absorbance or other signal is integrated by the microprocessor and the size of each DNA fragment determined by comparison to internal DNA size marker controls and DNA melting profiles and characteristics stored in the microprocessor and/or memory of the device.

DNA mutations are also detected by meltometer analysis. DNA fragments to be tested (including amplification-derived fragments and restrictions enzyme digestion or cloned fragments) are prepared and hybridized with a bound standard (typically wildtype) copy of the gene or gene fragment of interest. Hybridization is performed either on-device or using conventional DNA hybridization methods (as described in Hames & Higgins, Nucleic Acid Hybridization: A Practical Approach, Rickwood & Hames, eds., IRL Press: Oxford, 1985). Elution of the hybridized fragments is dependent on the degree of complimentary between the two species of DNA-strands (*i.e.*, wildtype and mutant). Hybridization analysis is performed using wildtype DNA that is prepared wherein one strand is covalently attached to a molecule that permits its immobilization. The non-covalently attached strand is then eluted by washing at a temperature much greater than the $T_m$ of the duplex (typically, the DNA is heated to >90°C, or to lower temperatures in the presence of denaturants such as formamide). Elution is monitored to determine the concentration of bound single-stranded product available for further hybridization; typically, the amount of DNA eluted is monitored, for example by ultraviolet light absorbance, and the bound DNA considered to be completely single stranded when no more DNA can be eluted. The wildtype DNA is prepared whereby only one of the strand making up the duplex is covalently attached to the immobilizing molecule, in order to require detectable labeling of only one (the complementary one) strand of the mutant DNA to be tested. Alternatively, either strand may be covalently attached, requiring both mutant strands to be detectably labeled. An advantage of double-labeling the mutant fragment even when only one wildtype strand is covalently attached to the immobilizing molecule, is that denaturation and elution of the non-complementary strand can be monitored during hybridization, and non-specific binding/hybridization of the mutant to wildtype DNA strands can be detected.

After hybridization is accomplished, the degree of complementarity of the strands is determined by a modification of the thermal or chemical denaturing protocols described above. Analysis of the resulting pattern of duplex melting is performed by comparison to a pattern of mismatched DNA duplex melting prepared either simultaneously or prior to experimental analysis and stored in the device microprocessor and/or memory using standard or expected single base or multiple mismatches. Such comparison form the basis for a determination of the rapid screening of individuals for a variety of characterized disease-associated genetic polymorphisms.

DNA mutations are also detected by meltometer analysis. In this embodiment, test DNA is immobilized on the disk and subjected to hybridization/denaturation analysis with a battery of precharacterized test probes. Using this method, DNA fragments are preferably prepared using *in vitro* amplification techniques, so that one strand is immobilizable due to covalent attachment of the binding molecule to one of the primers. Using this method, the DNA fragment to be tested is sequentially hybridized with and eluted by denaturation from a series of well-characterized DNA probes being detectably labeled. Alternatively (depending on the nature of the DNA mismatch expected for each probe), hybridization and denaturation are multiplexed, using probes detectably labeled with different detectable labels so that each probe can be identified. This method is useful for genetic screening as described above.

**EXAMPLE 4**

**DNA Amplification and Analysis**

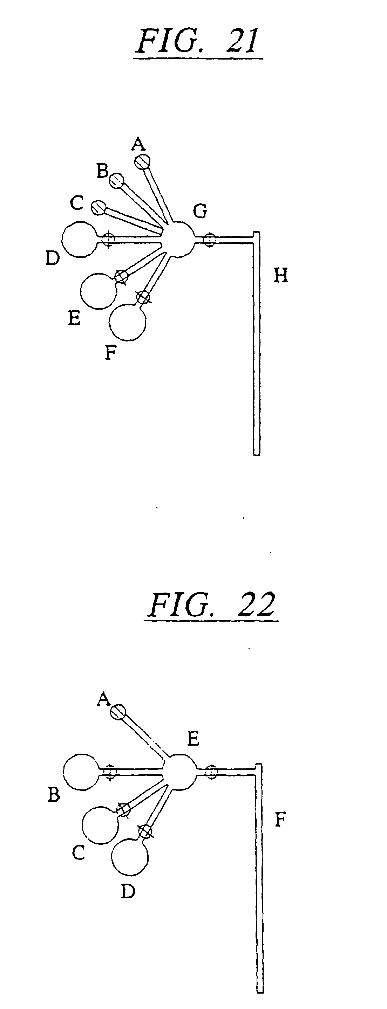

Fragments of DNA are amplified *in vitro* by polymerase chain reaction (PCR) or magnetic chain reaction and analyzed by capillary electrophoresis. Reagent mixing, primer annealing, extension and denaturation in an amplification cycle resulting amplification of a 500bp target fragment and its subsequent analysis are carried out using a device and disk as described in Example 1 above. A schematic diagram of the structure of the disk is shown in Figure 21.
The disk comprises at least three sample input ports A, B and C. Port A permits injection of 30 attomoles (about 100pg) linear bacteriophage lambda DNA. Port B and C allow input of 5 µL of a 20 µM solution of primer 1 and 2 respectively, having the sequence:

Primer 1:     5'-GATGAGTTCGTGTCCGTACAACTGG-3' (SEQ ID No.: 1)

and

Primer 2:     5'-GGTTATCGAAATCAGCCACAGCGCC-3' (SEQ ID No.: 2).

The disk also comprises three reagent reservoirs D, E and F in the Figure and containing 54 µL of distilled water; 10 µL of a solution of 100mM Tris-HCl (pH 8.3), 500mM KCl, 15mM MgCl$_2$, 0.1% gelatin and 1.25 µM of each dNTP; and 1µL of *Taq* DNA polymerase at a concentration of 5 Units/µL, respectively.
In addition, the disk comprises a reaction chamber G that is configured to facilitate mixing of these reagents using a flexural-plate-wave component (as described in U.S. Patent No. 5,006,749). Also included in the configuration of reaction chamber G are cooling and heating means via a Peltier component. These components can be integral to the disk or can be positioned in the device so as to provide heating and cooling specific for the reaction chamber. Disks are also provided that comprise a multiplicity of sets of the reaction components A through G.
Amplification is initiated by introducing sample DNA and primer into each set of ports A, B and C. When all samples and primers have been introduced into the ports, the disk is spun at a speed of 1 to 30,000 rpm to effect mixing of the reagents into reaction chambers G. Simultaneously, valves controlling reservoirs D, E and F are opened and the contents of these reservoirs are also forced into reaction chamber G. Mixing of sample DNAs, primers and reagents is facilitated by activation of the flexural-plate-wave component. DNA amplification takes place in the reaction chamber using the following thermocycling program. The reaction mixture is initially heated to 95°C for 3 minutes. The amplification cycle thereafter comprises the steps of: step 1, incubation at 95°C for 1 minute; step 2, cooling the chamber to 37°C for 1 minute; and step 3, heating the chamber to 72°C for 3 minutes. This amplification cycle is repeated for a total of 20 cycles, and the reaction completed by incubation at 72°C for 5 minutes.
Amplified DNA fragments are analyzed by transfer to capillary electrophoresis unit H by spinning the disk at a speed of 1 to 30,000rpm and opening a valve on reaction chamber G leading to capillary electrophoresis unit H, thereby effecting transfer of an amount of the reaction mixture to the electrophoresis unit. The amount of the reaction mixture, typically 10µL, is determined by a combination of the length of time the valve on reaction chamber G is open and the speed at which the disk is rotated. Capillary electrophoresis is accomplished as described below in Example 11, and fractionated DNA species detected using optical or other means as described above in Example 2. This method provides a unified amplification and analysis device advantageously used for performing PCR and other amplification reactions in a sample under conditions of limited sample.

**EXAMPLE 5**

**DNA Restriction and Digestion and Analysis**

Restriction enzyme digestion and restriction fragment analysis is performed using a disk and device as described above in Example 1. A double-stranded DNA fragment is digested with a restriction endonuclease and subsequently analyzed by capillary electrophoresis. Reagent mixing, DNA digestion and restriction fragment analysis are carried out on the disk. A schematic diagram of the structure of the disk is shown in Figure 22.

The disk comprises a sample input port A; three reagent reservoirs B, C and D; a reaction chamber E configured for mixing the reagents as described above in Example 5, and a capillary electrophoresis unit F. The reagent reservoirs contain: 1-2µL of a restriction enzyme, *e.g.* HindIII, at a concentration of 20 Units/µL in reservoir B; 4 µL of a solution of 100mM Tris-HCl (pH 7.9), 100mM $MgCl_2$ and 10mM dithiothreitol in reservoir C; and 30µL of distilled water in reservoir D. Disks are also provided that comprise a multiplicity of sets of the reaction components A through E.

Restriction enzyme digestion of the DNA is initiated by placing 4.5µL of a solution (typically, 10mM Tris-HCl, 1mM EDTA, pH 8) containing 4µg bacteriophage lambda DNA in sample input port A. The DNA sample and the reagents in reservoirs B, C and D are transferred to reaction chamber E by spinning the disk at a rotational speed of 1 to 30,000 rpm and opening valves controlling reservoirs B, C and D. The reaction is incubated at 37°C for 1h in reaction chamber E after mixing, the reaction chamber being heated by provision of a Peltier heating element either on the disk or positioned in the device so as to specifically heat the reaction chamber. After digestion, an amount of the digested DNA is transferred to electrophoresis unit F by spinning the disk at a speed of 1 to 30,000 rpm and opening a valve on reaction chamber E leading to capillary electrophoresis unit F, thereby effecting transfer of an amount of the reaction mixture to the electrophoresis unit. The amount of the reaction mixture, typically 10µL, is determined by a combination of the length of time the valve on reaction chamber E is open and the speed at which the disk is rotated. Capillary electrophoresis is accomplished as described below in Example 11, and fractionated DNA species detected using optical or other means as described above in Example 2.

**EXAMPLE 6**

**DNA Synthesis**

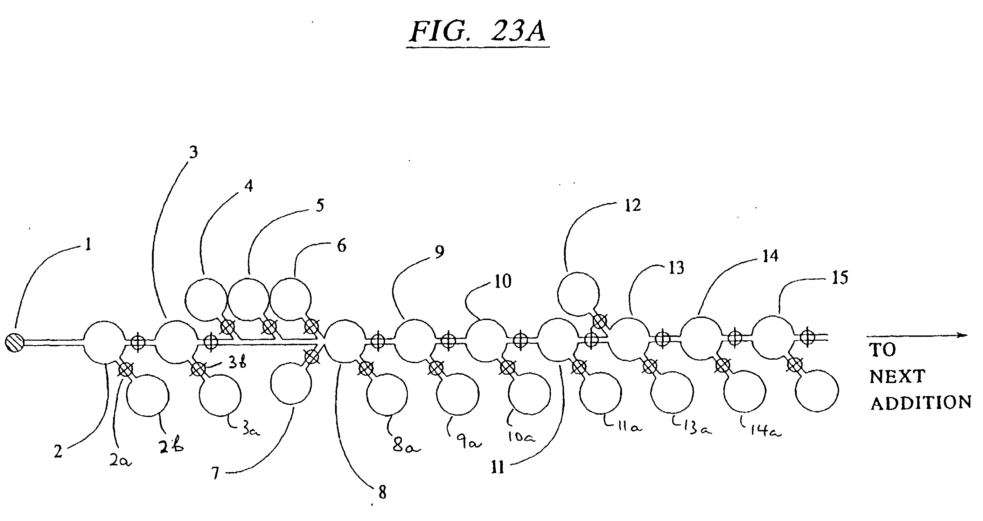

Oligonucleotide DNA synthesis is performed using a disk and device as described above in Example 1. Synthesis is achieved by the stepwise transport of controlled pore glass (CPG) through a series of reaction chambers containing reagents necessary for phosphoramidite DNA synthesis. Reagents and CPG are delivered sequentially to reaction chambers by single-use valves connecting the reaction chambers to each other and to reagent reservoirs. Each disk has a number of synthesis reaction chambers to produce oligonucleotides having a length similar to the length of oligonucleotides produced by commercially-available DNA synthesis instruments (*i.e.,* 100-150 bases). A schematic diagram of the structure of the disk is shown in Figure 23A.

A CPG bearing a first base of a sequence (thereby defining the 3' extent of the oligonucleotide) is loaded either by the user or by automated means into a sample input port 1. The CPG is then transferred into a reaction chamber 2 containing trichloroacetic acid (TCA) in acetonitrile ($CH_3CN$) by spinning the disk at a rotational speed of 1 to 30,000 rpm. Detritylation of the nucleotide is performed at room temperature for a defined time interval, typically 1 minute. The reagent is then decanted from the first reaction chamber 2 by opening a valve 2a with a bore too small to allow passage of the CPG but sufficient to drain the TCA-containing mixture into a decantation chamber 2b As the deprotection of the base by detritylation is known to produce a colored product (orange), the intensity of which is a measure of the extent of the reaction, optical means for determining the absorbance of this effluent are advantageously provided to be recorded on the device microprocessor/memory. After decanting the reaction mixture, the CPG are spun into a rinse chamber 3 containing $CH_3CN$, the chamber 3 optionally comprising a mixing means as described above. After rinsing, the $CH_3CN$ is decanted into a effluent reservoir 3a controlled by a size-selective valve 3b as above, and the CPG spun into a second reaction chamber 8. Mixed with the CPG in the second reaction chamber is a solution containing one of four phosphoramidite bases (G, A, T, or C) from one of chambers 4, 5, 6, or 7 corresponding to the next position in the oligonucleotide chain. The reaction mixture in the second reaction chamber 8 is mixed and allowed to react for a defined time interval, typically three minutes. The reaction mixture is then decanted as above into chamber 8a and the CPG spun into a rinse chamber 9 containing $CH_3CN$ and a mixing means. After rinsing, the $CH_3CN$ is decanted to an effluent reservoir 9a and the CPG is spun into a third reaction chamber 10 containing an oxidizing mixture of iodine, water, pyridine and tetrahydrofuran, where the reaction mixture is incubated for a defined time interval typically 1 minute. The reaction mixture is decanted to an effluent reservoir 10a and the CPG spun into a rinse chamber 11 containing $CH_3CN$. After rinsing, the $CH_3CN$ is decanted to an effluent reservoir 11a and the CPG spun into a fourth reaction chamber 13 along with a two-component "capping" reagent from reservoir 12. The capping reaction is performed for a defined time interval, typically 1 minute. After the reaction is complete, the reaction mixture is decanted to an effluent reservoir 13a as above and the CPG spun into a rinse chamber 14 containing $CH_3CN$. The $CH_3CN$ is then decanted to an effluent reservoir 14a and the CPG is spun into a fifth chamber 15 containing TCA, comprising the beginning of another cycle. The cycle is repeated by transit of the CPG through interconnected series of the four reaction chambers until the preprogrammed sequence is completely synthesized. The CPG is then spun into a reaction chamber containing concentrated ammonium hydroxide and heated at 60°C for a defined time interval, typically 6 hours, during which time the DNA molecule is deprotected and cleaved from the CPG support. The finished oligonucleotide is removed by the user or by automated means.

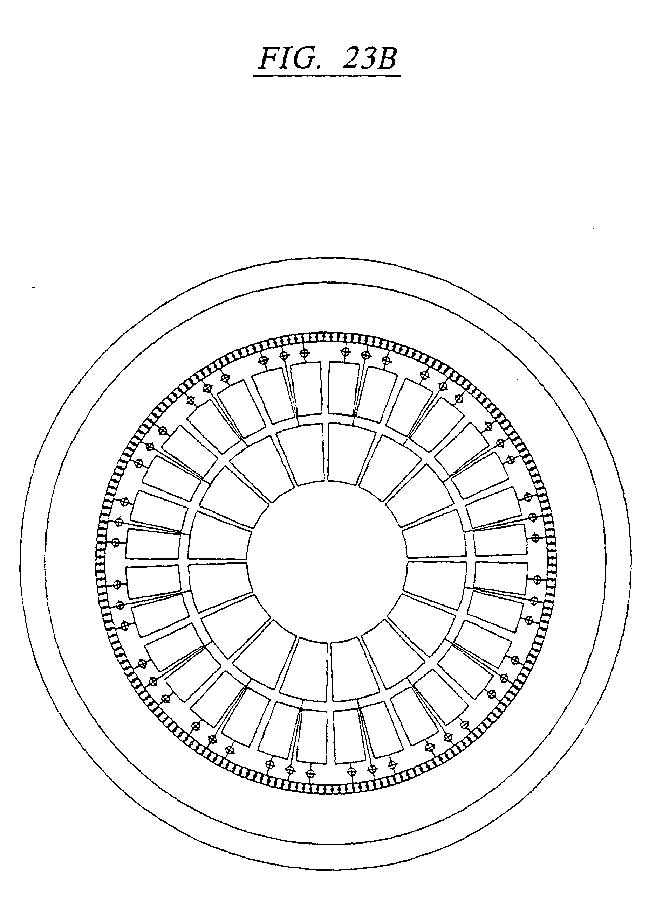

The disk provides a series of reaction chambers linked to each other and comprising four reaction and rinsing chambers per nucleotide to be added to the oligonucleotide chain. The disks can be loaded to produce a particular oligonucleotide, or each reaction chamber 2 can be in contact with reagent reservoirs containing each of the four nucleotide bases and linked to the reaction chamber by an individually-controllable valve. In this embodiment, activation of the appropriate valve at each step in the cycle is controlled by a signal from the device. Disks comprising a multiplicity of these synthetic arrays; permitting simultaneous synthesis of a plurality of oligonucleotides, are also provided. A schematic diagram of a disk configured for multiple oligonucleotide synthesis is shown in Figure 23B.

DNA synthesis can also be performed upon preloaded CPG contained in reaction chambers toward the periphery of the disk and reagents delivered by the use of multiuse two-way valves, as schematically diagramed in Figure 23A. In these disks, reaction chambers capable of containing 100nL, spaced 150μm on-center (measured from the center of one sphere to the center of the next sphere) in a disk of a 120mm diameter, as many as 1250 reaction chambers can be manufactured. Reagent reservoirs containing sufficient volumes to supply the reagent chambers on the disk are prefilled with the four phophoramidites, $CH_3CN$, TCA, oxidizer and capping reagents. Trityl-bearing CPG or linkers bound directly to the reaction chambers are similarly preloaded onto the disk. Microliter volumes of reagents are sufficient for each reaction. TCA is spun into each first reaction chamber and allowed to react for a defined length of time, typically one minute, then spun to a effluent (waste) chamber on the periphery of the disk. The $CH_3CN$ rinse is spun into each reaction chamber and then to waste. By selective valve actuation, the A, C, G, or T phosphoramidite is spun to the reaction chambers requiring that base and reacted for a defined time interval, typically three minutes, and the spun to waste. A $CH_3CN$ rinse is spun to each reaction chamber and after, to the waste chamber. The oxidizer mixture is spun into each reaction chamber, reacted for a defined time interval, typically one minute, then to waste. Another $CH_3CN$ rinse is spun to each reaction chamber and then to waste. The two-component capping reagent is spun to each reaction chamber and reacted for a defined time interval, typically one minute, then to waste. For each cycle, the final $CH_3CN$ rinse is then spun to each reaction chamber and then to the waste chamber. The cycle is repeated for a preprogrammed number of cycles until each oligonucleotide is completely synthesized. Concentrated ammonium hydroxide is then spun to each of the reaction chambers and reacted for a defined length of time, typically 6 hours, and reacted at 60°C to deprotect and cleave the completed DNA from its support. The DNA can then be removed by manual or automated means. Conversely, the linkage of the oligonucleotide to the CPG support is chosen to be resistant to the action of ammonium hydroxide, so that the deprotected oligonucleotide remains in the reaction chambers bound to CPG.

Peptide synthesis disks are also provided, whereby the arrangement of reagent reservoirs and reaction chambers as described above is adapted for the synthetic reactions comprising a peptide synthesis regime.

## EXAMPLE 7

### Enzymatic DNA Sequencing

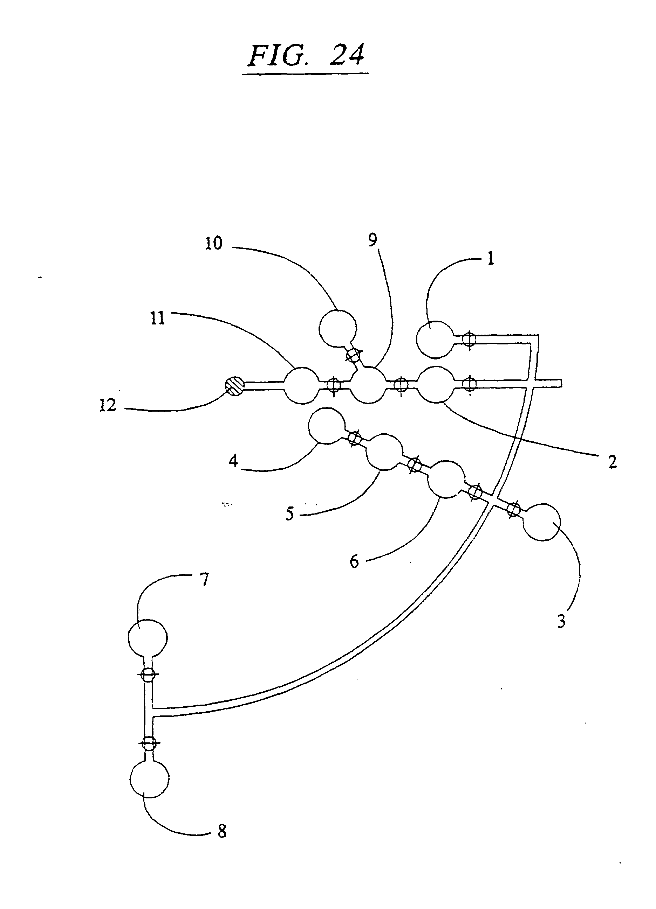

The nucleotide sequence of a DNA fragment is determined by the Sanger enzymatic sequencing method using a disk prepared as described in Example 1 above (see Figure 24). Template DNA (200pg in 250mL) and 100 femtomoles of an appropriate primer are pipetted manually or by an automated process into a sample input port. The DNA is then transferred into a mixing chamber containing terminator solution (*i.e.,* a solution comprising a dideoxy form of nucleotides G, A, T or C) by spinning the disk at a rotational speed of 1 to 30,000 rpm. Terminator solution typically comprises 100nL of a solution containing 5 picomoles of each deoxynucleotide, 0.5 picomoles of one dideoxynucleotide covalently linked to a fluorescent label, 90mM Tris-HCl-(pH 7.5), 45mM $MgCl_2$ and 110mM NaCl. The contents of the mixing chamber are transferred into a reaction chamber containing 0.1 units of 17 DNA polymerase (or, alternatively, 0.1 Units of *Taq* polymerase) and 20nL 0.1M dithiothreitol (DTT) by spinning the disk at a rotational speed of 1 to 30,000 rpm, yielding a reaction mixture in the reaction chamber having a final concentration of buffer components that is 26mM Tris- HCl (pH 7.5), 13mM MgCl 2, 32mM NaCl, and 6mM DTT. The reaction chamber is heated to 37°C (or, alternatively, to 65°C for Taq polymerase) by a resistive heating element integral to the disk, or alternatively, positioned within the device to specifically heat the reaction chamber, and incubated for a defined length of time, typically 1 minute. The reaction products are spun into an equal volume of 90% formamide/EDTA, heated to 90°C for 1 minute and spun to a capillary electrophoresis unit on the disk. The set of dideoxynucleotide-terminated DNA fragments comprising the reaction mixture is then separated by capillary electrophoresis and the sequence of fragments determined by laser-induced fluorescence detection as described above. Disks comprising a multiplicity of these synthetic arrays, permitting simultaneous synthesis of plurality of dideoxynucleotide-terminated oligonucleotides, are also provided. The deducted nucleotide sequence is determined from the pattern of fluorescence signals detected and the sequence is determined from the pattern of fluorescence signals detected and the sequence derived by the device microprocessor from these data.

**EXAMPLE 8**

**Liquid phase synthesis and analysis**

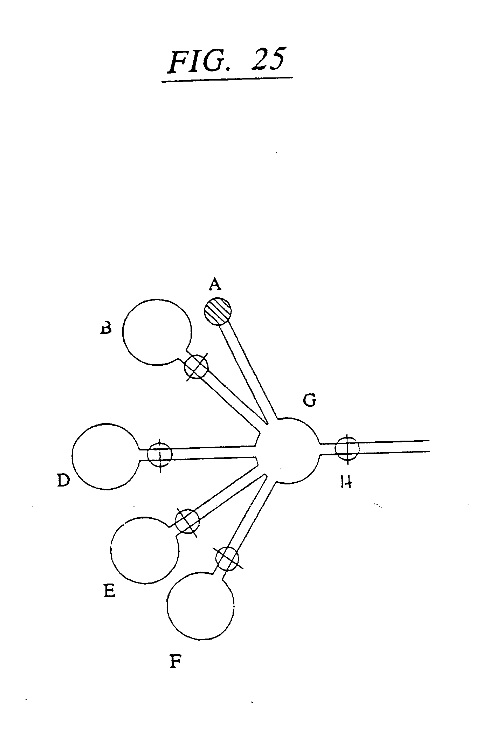

A variety of colorimetric chemical analyses are performed using a disk as described in Example 1. For example, a disk is provided (*see* Figure 25) for performing a solution assay to determine iron concentration in a test solution (such as an industrial effluent) using a standard colorimetric test. The device is fabricated with reagent reservoir containing 40μL 12N HCl, 100μL 10% hydroxylamine hydrochloride, 100μL 10% sodium citrate buffer (pH 4), and 50μL 0.02%, 1,10-phenanthroline. The reagent reservoirs are arranged as shown in Figure 25 so that these reagents are added to a reaction chamber sequentially by opening valves controlling flow from each reagent reservoir. Reagent transfer to the reaction chamber is achieved by spinning the disk of Example 1 at a rotational speed of 1 to 30,000 rpm, whereby the centripetal force motivates each reagent solution from its reservoir to the reaction chamber. As shown in Figure 25, sample is introduced through the sample port (A) and centripetally delivered to the reaction chamber (G). The valve to the reagent reservoir containing HCl (B) is opened and acid is added to the sample. The sample is incubated 10 minutes to dissolve all iron oxide present. Hydroxylamine hydrochloride (reservoir D) and citrate (reservoir E) are next added to the reaction mixture. The reaction mixture is incubated 20 minutes to ensure complete reduction of iron III to iron II. Next, 1,10-phenanthroline is transferred from reservoir F to complex the iron II and form a colored product. The solution is incubated 30 minutes at 30°C to complete color development. Photometric measurement at 520 nm is done after the incubation process in a "read" cell (not shown) connected to the reaction chamber through a valve (H).

**EXAMPLE 9**

**Solid phase (surface/colloid) synthesis/analysis**

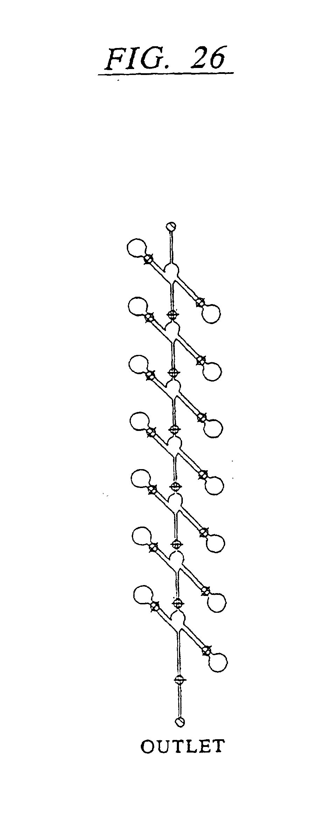

Oligonucleotide, single-stranded DNA or duplex DNA, is covalently linked to a reactive particle (such as a bead or magnetic particle or a chromatographic substrate) using a disk prepared as described in Example 1 and shown in Figure 26. In the illustrate embodiment, a 25μL aliquot of carboxy-activated magnetic particles (BioMag 4125, PerSeptive Diagnostics, Framingham, MA) is added to the disk through a sample introduction port. The particles are exchanged from the initial solution into 50μL 0.1 M imidazole (pH 6) by decanting the original solution through a valve to an effluent or waste reservoir, whereby the valve is configured to prevent loss of the magnetic particles from the reaction chamber. The imidazole solution is then added to the particle reaction chamber from an imidazole reservoir on the disk, transfer of imidazole being controlled by a valve. The motive force for both decanting the original magnetic particle solution and transferring imidazole from the imidazole reservoir to the particle reaction chamber is provided by spinning the disk at a rotational speed of 1 to 30,000 rpm. Specifically with reference to Figure 26, as the disk spins the dense magnetic particles are pelleted in a funnel at the end of the reaction chamber and deposited to waste. A valve controlling an imidazole reagent chamber containing 50 uL of 0.1M imidazole is then opened above the particles but below the decanting level and used to transfer the particles through a valve in the reaction chamber and into the next decanting reservoir. This decanting process can be repeated many times to affect a change in the liquid phase to the desired composition. Typically, three exchanges are sufficient. Alternatively, appropriate configuration of the reagent and reaction chambers allows the magnetic particles to be exchanged within a single reaction chamber by controlled addition and removal of imidazole from clusters of reagent reservoirs, or alternatively, a single reagent reservoir large enough to contain sufficient imidazole for the entire cycle of exchange.

After the exchange cycle is complete, the magnetic particles are transferred to a next reaction chamber containing 250 μg dry 1-ethyl-3(3-dimethylaminopropyl) carbodiimide (EDAC). A reagent reservoir containing 170 OD (170ng) 5'-aminated DNA oligonucleotide in 50 μL of 0.1 M imidazole solution is admitted to the reaction chamber prior to addition of the particles in order to dissolve the EDAC. The particles are then added through a valve in about 100 μL 0.1 M imidazole. Upon addition of the magnetic particles to the reaction chamber, the device is stopped and incubated 6 hours at 40°C. Heating can be effected by a heat source (such as Peltier heating device) embedded in the disk itself, or positioned in the instrument in a configuration permitting specific heating of the reaction chamber. In the latter alternative, the disk may be stopped at a predetermined position relative to the device to ensure specificity of heating of the reaction chamber.

After incubation, the particles are washed and exchanged into 100 μL portions of water by decanting as described above as the disk is spun. Three exchanges are typically performed to purify the particles. Product is advantageously collected in the extremity of the disk where it can easily be accessed for subsequent use. Disks comprising a multiplicity of these synthetic arrays, permitting simultaneous synthesis of a plurality of particle-linked oligonucleotides, are also provided.

### EXAMPLE 10

#### Micro-Extraction System

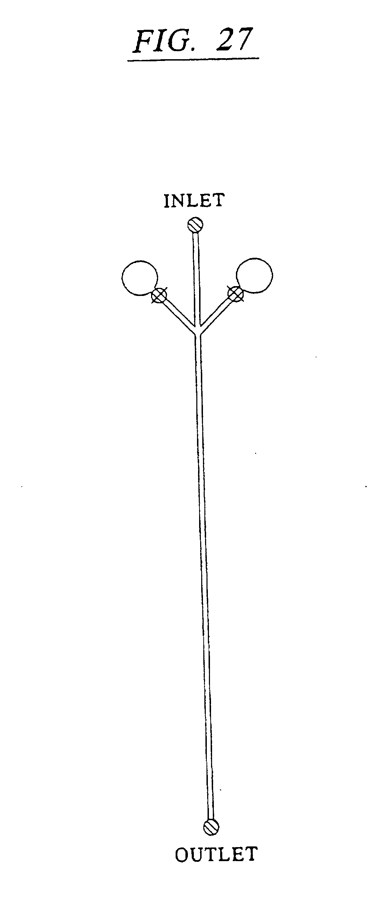

A disk as described in Example 1 (see Figure 27) is used for performing micro-extraction of a solute from a solution or of a component of a mixture as an alternative to HPLC or other conventional biochemical separation methodology. Specifically, a channel on the disk is coated with a compound (such as octanol) by standard procedures to provide a surface having an affinity for a component of a mixture, typically a complex chemical or biochemical mixture. With a silicon disk, for example, the surface of the channel is activated by filling the chamber with aqueous epoxysilane at 95° C for 1 hour. The disk is washed about five times with distilled water to remove unreacted silane, and aminooctane is added in an solvent and incubated at 95° C for 1 hour followed by solvent rinse to remove unreacted octane.

Sample mixture containing the component to be eluted is added to an injection port and moved through the coated separation channel by rotating the disk at 1 to 30,000 rpm. Reagent reservoirs are opened at the entrance of the channel and used to elute the sample retained on the coated channel to a collection reservoir. The isolated sample component is then collected at an outlet port.

### EXAMPLE 11

#### Free Zone Capillary Electrophoresis

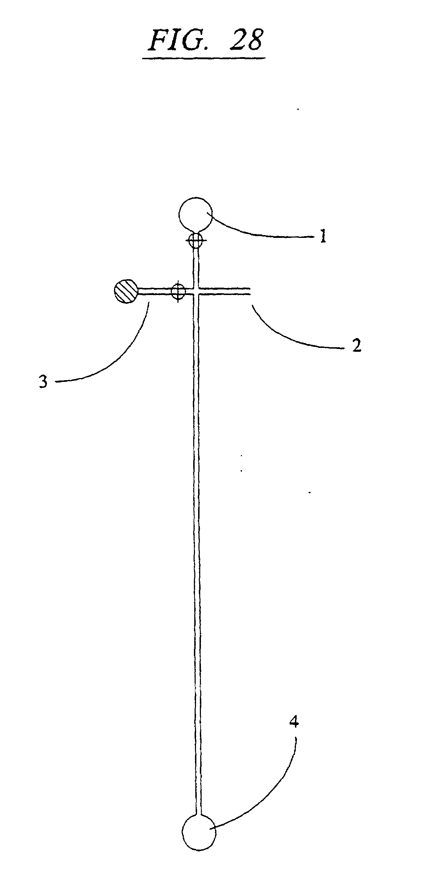

Free zone capillary electrophoresis is performed on a disk fabricated as described in Example 1 above, and schematically represented in Figure 28. Specifically, a 5μm x 75μm x 25mm capillary (it will be recognized that all dimensions are approximate within limits of precision in fabricating components such as capillaries in the disk), is lithographically etched onto a glass disk. Electrical connections are made using standard methods by plating platinum onto the non-etched surface of the glass before sealing the top to the device. The separation channel is intersected by a 15 mm sample introduction channel, positioned 3 mm away from a buffer reservoir. The intersecting channel has a sample inlet port at one end and electrical connections at either end to control sample application to the capillary.

In the practice of capillary electrophoresis on the disk, the separation channel is filled from the buffer reservoir by rotation of the disk at a speed of 1 to 30,000 rpm. Once the channel is filled, rotation is stopped until pressure needs to be applied to the channel again. Sample is introduced by applying a voltage between the intersecting analyte inlet and analyte outlet channels on the chip (*see* Figure 28). A 50 V potential drop is applied between the sample inlet and outlet ports while the separation channel ports float. The sample, comprising a solution of 5mM EDTA, 1mM Tris-HCl (pH 8) with 1 mM $Mg^{2+}$ and 1 mM $Ca^{2+}$ (typically prepared from the chloride salt). The running buffer consists of 10 mM Tris-HCl (pH 8), 5 mM EDTA. Separation toward the cathode is then performed by floating the electric potential at the sample reservoir and applying 250 V along the separation channel. Separation is monitored at a position 2 cm from the inlet port by monitoring, e.g. UV absorbance at 254 nm using a UV light source (mercury lamp) and a photodiode detector, positioned on the device to interest the capillary channel.

### EXAMPLE 12

#### DNA electrophoresis

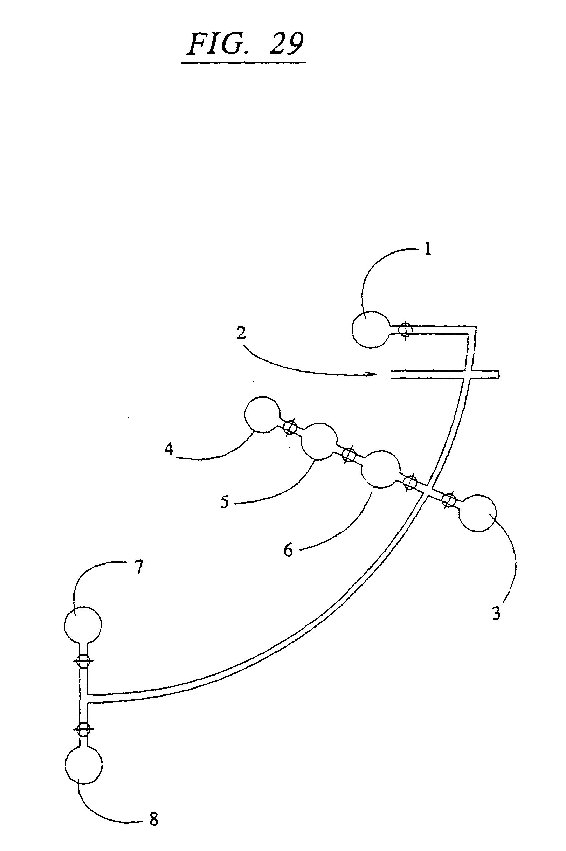

Gel electrophoresis is performed on a disk prepared as described in Example 1 above. For this application, a gel medium is prepared in the separation channel; however, such gel media must be protected from shear forces that develop with rotation of the disk during transfer of sample or buffer to the electrophoresis channel. Thus, the gel-filled capillary is advantageously arrayed concentrically on the disk, as shown schematically in Figure 29. As a result, the gel will only experience shear forces from centripetal-induced pressure during rotation if a fluid reservoir is in contact with the capillary during rotation of the disk. At rest, the planar geometry of the disk prevents hydrodynamic pressure on the capillary. This is an advantage over standard capillary electrophoresis systems, where hydrodynamic pressure is not so easily controlled because the buffer volumes and reservoir heights need to be carefully adjusted before each run to avoid hydrodynamic flow. This is also an advantage of capillary electrophoresis performed on the disks of the invention over electrophoresis performed on microchips, where buffer reservoirs are positioned above the plane of the separation channel and are thereby susceptible to hydrodynamic pressure-driven fluid flow.

Gel electrophoresis is performed on the disks of the invention to separate DNA fragments, including duplex PCR fragments, oligonucleotides and single-stranded , dideoxynucleotide-terminated enzymatic DNA sequencing components, the system is configured as shown in Figure 29. The disk is prepared comprising a polyacrylamide gel concentrically arrayed in a microetched separation channel in the disk. The polyacrylamide gel is prepared from an unpolymerized solution of 7M urea, 45mM Tris-borate buffer (pH 8.3), 1mM EDTA, 9% acrylamide, 0.1% TEMED and 10% ammonium persulfate. The disk can be prepared in the separation channel by mixing the components (wherein it will be recognized that unpolymerized polymerized polyacrylamide is susceptible to light-catalyzed polymerization upon storage) particularly by introducing TEMED and ammonium persulfate to the mixture. Sufficient gel mixture is added to the separation channel by opening a valve from a mixing chamber to the separation channel and rotating the disk at 1 to 30,000 rpm. The disk is stopped upon filing of the separation channel to permit gel polymerization. Shortly before polymerization is complete, the exit channel is flushed to eliminate bubbles and unpolymerized monomer by flushing the channel with buffer from a large buffer reservoir at the outlet side of the channel, controlled by a valve. A similar process is conducted on the inlet side of the gel.

To introduce a DNA sample, a valve is opened from an inlet port holding a solution of DNA fragments, or alternatively, the sample is pipetted directly onto the disk. The sample is applied to the separation channel by spinning the disk at 1 to 30,000 rpm, forcing sample and buffer into the buffer filled channel above the gel. Upon introduction of the sample to the separation channel and the sample inlet channel, sample concentrates at the gel/buffer interface before entering the separation matrix, analogous to sample concentration during conventional slab gel electrophoresis. Electrophoresis is performed at 250 V/cm to effect a separation of DNA fragments, the cathode (positive electrode) being positioned at the outlet end of the channel distal to the sample inlet channel. A laser induced fluorescence detector is positioned at the outlet of the gel filled capillary chamber to detect the labeled DNA fragments, as described above in Example 2.

## EXAMPLE 13

### Spectrophotometer Pathlength Extension

Spectrophotometric measurements in a rotating structure of the invention can be limited by the relatively small pathlengths provided by spectrophotometric illumination across the transverse dimension of the disk. The intensity of absorbance of a solution is dependent on the depth of the absorbing layer, as well as the concentration of the absorbing molecules (as described in the Lambert-Beer law).

Although a measurement cell in a rotating microsystem platform of the invention presents a short transverse pathlength, the lateral pathlength through the disk can be extensive (*i.e.,* centimeters *versus* millimeters). Spectral measurements can be enhanced by introducing light through the detection chamber in the lateral dimension.

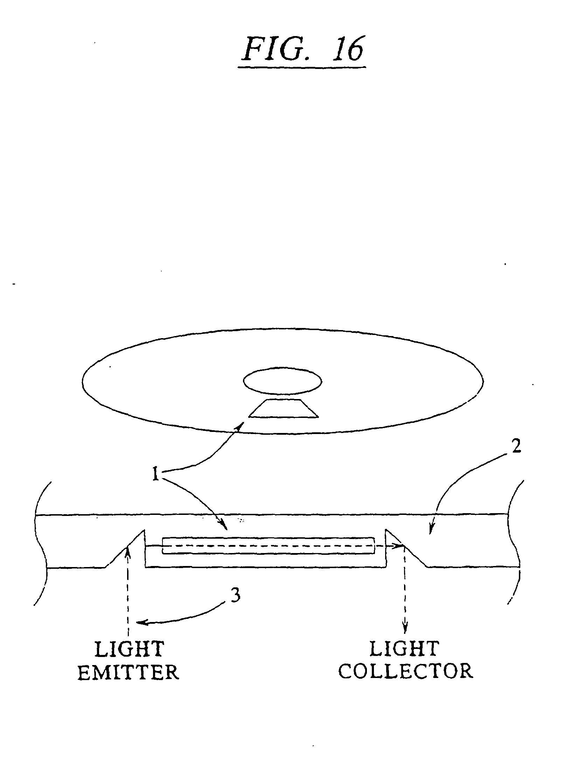

One arrangement providing transverse illumination in the lateral dimension is shown in Figure 16. Light is beamed in a perpendicular direction towards the disk. A mirror is positioned at a 43° angle to the direction of the illuminating beam, whereby the light is directed laterally through the detection chamber. Light passes through the detection cell and is redirected by another 45° mirror onto a photosensitive detector, such as a photodiode or photomultiplier tube. These mirrors can be inserted onto the disk, integrally molded into the disk or metallicized in the plastic or other substrate comprising the disk.

## EXAMPLE 14

### Cell Counting, Identification and Monitoring

Methods for identifying particular cells or cell types in a biological sample are provided. For example, a microplatform of the invention is prepared by having a surface adsorbly coated with monoclonal antibody specific to *E. coli.,* the remaining sites being blocked with BSA. A milk sample is introduced onto the disk and placed into contact with a reaction chamber comprising the surface coated with the antibody. The milk is incubated in this chamber for 30 min. The microsystem platform is then rotated to remove unwanted materials. An amount of a buffer appropriate for washing the microsystem chamber is then added to the surface or chamber through a microchannel from a reservoir containing washing buffer, said buffer being released by centrifugal force and opening of a microvalve. In a useful embodiment, the washing buffer comprises an *E. coli*-specific monoclonal antibody crosslinked to an enzyme (such as peroxidase). Thus incubation is allowed to proceed for 5 min. The disk is again spun with the opening of the appropriate microvalves to remove the washing solution from the chamber and to add a solution containing an enzymatic substrate (tetramethylbenzidine) and hydrogen peroxide, maintained heretofore in a reagent reservoir connected to the reaction chamber by a micro valve-controlled microchannel. The amount of *E. coli* bound in the reaction chamber is quantititated with regard to the amount of detected enzymatic activity, which is determined spectrophotometrically by the appearance of a light-absorbing product or the disappearance of a light-absorbing substrate.

**Claims**

1. A centripetally-motivated fluid micromanipulation apparatus that is a combination of

   a microsystem platform, comprising a substrate having a first flat, planar surface and a second flat, planar surface opposite thereto,

   wherein the first surface comprises a sample input means and a multiplicity of microchannels embedded in the first planar surface, each microchannel being vented to an air displacement channel to permit air displacement by movement of fluid through the microchannels to be vented through the air displacement channels thereby to reduce air pressure resistance to fluid movement, wherein the sample input means and the microchannels are connected and in fluidic contact, and

   wherein the second flat, planar surface opposite to the first flat planar surface of the platform is encoded with an electromagnetically-readable instruction set for controlling rotational speed, duration, or direction of the platform, and further comprises an instruction set comprising analytical, diagnostic or quality control instructions for performing an assay, the electromagnetically-readable instruction set being readable by a CD or CDV disc player, and

   a micromanipulation device, comprising a base, a rotating means for rotating the platform, a power supply and user interface and operations controlling means, wherein the rotating means is operatively linked to the microsystem platform and in rotational contact therewith

   wherein a volume of a fluid within the microchannels of the platform is moved through said microchannels by centripetal force arising from rotational motion of the platform for a time and a rotational velocity sufficient to move.the fluid through the microchannels.

2. Apparatus according to claim 1, wherein the operations controlling means comprises an integrated electronic processing system which is directed by instructions comprising the instruction set on the second planar surface of the platform.

3. Apparatus according to claim 1 or claim 2, wherein fluid flow through the microchannels is controlled by capillary microvalves which use rotationally-induced fluid pressure to overcome capillary forces.

4. Apparatus according to claim 3, wherein the microvalve comprises, an intersection between a first fluid passage selected from a microchannel, a reservoir, a reaction chamber, and a detection chamber, said first fluid passage having a first cross-section, and a second fluid passage into which fluid can flow from the first fluid passage upon operation of the apparatus, said second fluid passage being selected from a microchannel, a reservoir, a reaction chamber, and a detection chamber, and said second fluid passage having a second cross-section different from the first cross-section, the first cross-section being selected, in the case of a fluid which completely wets or partially wets the material of the fluid passages, to be narrower than the cross-section of the second fluid passage, and the cross-section of the first fluid passage being selected, in the case of a fluid which does not wet the material of the fluid passages, to be larger than the second cross-section.

5. Apparatus according to any one of claims 1 to 4, wherein the first flat, planar surface and second flat, planar surface of the microsystem platform form a disk.

6. Apparatus according to claim 5, wherein the microsystem platform is a disk having a radius of about 1 to 25 cm.

7. Apparatus according to any one of claims 1 to 6, wherein the first and second flat, planar surfaces of the microsystem platform define a centrally located aperture that is engaged to a spindle on the micromanipulation device, whereby rotational motion of the spindle is translated into rotational motion of the microsystem platform.

8. Apparatus according to any one of claims 1 to 7, wherein the microsystem platform is constructed of a material selected from an organic material, an inorganic material, a crystalline material and an amorphous material.

9. Apparatus according to claim 8, wherein the microsystem platform further comprises a material selected from silicon, silica, quartz, a ceramic, a metal and a plastic.

10. Apparatus according to any one of claims 1 to 9, wherein the microsystem platform has a thickness of about 0.1 to 10mm, and wherein the cross-sectional dimension of the microchannels between the first and second flat, planar surfaces is less than 500μm and from 1 to 90 percent of said cross-sectional dimension of the platform.

11. Apparatus according to any one of claims 1 to 10, wherein the microsystem platform further comprises a reaction chamber and a reagent reservoir embedded.within the first planar surface thereof.

12. Apparatus according to claim 11, wherein the sample input means, the microchannels, the reaction chamber and the reagent reservoir are connected and in fluidic contact, and wherein fluid motion from the microchannels, the reaction chambers and the reagent reservoir is controlled by microvalves connected thereto.

13. Apparatus according to claim 11 or claim 12, wherein the microsystem platform has a thickness of about 0.1 to 100mm, and wherein the cross-sectional dimension of the reaction chamber or the reagent reservoir between the first and second flat, planar surfaces is from 1 to 75 percent of said thickness of the platform.

14. Apparatus according to any one of claims 11 to 13, wherein the microsystem platform comprises a multiplicity of sample input means, reagent reservoirs, reaction chambers and microchannels connected thereto and embedded within the first planar surface thereof, wherein a volume of a fluid sample is moved on the platform from the sample input means into and out from the reaction chambers, and a volume of a reagent is moved from the reagent reservoirs into and out from the reaction chambers, by centripetal force arising from rotation of the microsystem platform.

15. Apparatus according to any one of claims 1 to 14, wherein the microsystem platform is arranged to be rotated at a rotational velocity of about 1 to about 30,000 rpm.

16. Apparatus according to any one claims 1 to 15, wherein the microsystem platform comprises a detecting chamber embedded within the first planar surface of the platform and connected to a microchannel, and wherein the micromanipulation device comprises a detecting means, whereby the detecting chamber is assayed by the detecting means to yield an assay output.

17. Apparatus according to claim 16, wherein the detecting means on the device is brought into alignment with the detection chamber on the platform by rotational motion of the microsystem platform.

18. Apparatus according to claim 16 or claim 17, wherein the detecting means comprises a light source and a photodetector.

19. Apparatus according to claim 18, wherein the light source illuminates the detection chamber wherein light is reflected transversely through the detection chamber and detected by a photodetector.

20. Apparatus according to claim 19, wherein the detection chamber on the microsystem platform is optically transparent.

21. Apparatus according to any one of claims 17 to 20, wherein the detecting means is stationary and samples the detection chamber at a frequency equal to the frequency of rotation of the platform or multiples thereof.

22. Apparatus according to claim 21, wherein the detecting means comprises a stroboscopic light source.

23. Apparatus according to claim 22, wherein the detecting means is a monochromatic light source.

24. Apparatus according to any one of claims 16 to 23, wherein the detecting means detects optical absorbance, fluorescence, chemiluminescence, light-scattering or radioactivity.

25. Apparatus according to any one of claims 1 to 24, further comprising a temperature controlling element in thermal contact with the microplatform.

26. Apparatus according to any one of claims 1 to 25, further comprising a thermal detecting unit in thermal contact with the microplatform.

27. Apparatus according to any one of claims 1 to 26, wherein the microsystem platform comprises a filtering means linked to a microchannel.

28. Apparatus according to any one of claims 1 to 26, wherein the microsystem platform comprises a mixing element connected to a reaction reservoir or a microchannel.

29. Apparatus according to claim 28, wherein the microsystem platform comprises a static mixer comprising a textured surface of a reaction reservoir or microchannel.

30. Apparatus according to any one of claims 1 to 29, wherein the microsystem platform comprises a capillary microvalve connected to a reaction chamber or microchannel.

31. Apparatus according to any one of claims 1 to 30, wherein the microsystem platform further comprises a multiplicity of air channels, exhaust air ports and air displacement channels.

32. Apparatus according to any one of claims 1 to 31, wherein the rotating means of the micromanipulation device is an electric motor.

33. Apparatus according to any one of claims 1 to 32, wherein the micromanipulatin device comprises a rotation motion controlling means for controlling the rotational acceleration and velocity of the microsystem platform.

34. Apparatus according to any one of claims 1 to 33, wherein the micromanipulation device includes a user interface comprising a monitor and an alphanumeric keypad.

35. Apparatus according to any one of claims 1 to 34, wherein the micromanipulation device comprises an alternatang current or direct current power supply.

36. Apparatus according to any one of claims 1 to 35, wherein the microsystem platform includes an electrical connector in contact with an electric connector connected to the micromanipulation device.

37. Apparatus according to any one of claims 1 to 36, wherein the micromanipulation device comprises a microprocessor and a memory connected thereto.

38. Apparatus according to any one of claims 1 to 37, wherein the micromanipulation device comprises a reading means for reading the instruction set or a writing means.

39. Apparatus according to claim 38, wherein the reading means is a compact disk laser reading means.

40. Apparatus according to claim 38, wherein the writing means is a compact disk writing means.

41. Apparatus according to any one of claims 1 to 40, wherein the second flat, planar surface of the microsystem platform is encoded with machine language instructions.

42. Apparatus according to claim 41, wherein the machine language instructions control operation of the platform, data acquisition or analysis from the platform, data storage and retrieval, communication to other devices, or direct apparatus performance diagnostics.

43. Apparatus according to any one of claims 1 to 42, wherein the micromanipulation device includes a read-only memory or permanent storage memory that is encoded with machine language instructions.

44. Apparatus according to claim 43, wherein the machine language instructions control operation of the platform, data acquisition or analysis from the platform, data storage and retrieval, communication to other devices, or direct apparatus performance diagnostics.

45. Apparatus according to any one of claims 1 to 44, further comprising first and second microsystem platforms in contact with one another across one planar surface of each microsystem platform.

46. Apparatus according to any one of claims 1 to 45, wherein fluid on the microsystem platform is moved within the microchannels of the platform with a fluid velocity of from about 0.1 cm/sec to about 1000 cm/sec.

47. Apparatus according to any one of claims 1 to 46, for measuring the amount of analyte in a biological sample, wherein the microsystem platform comprises

a multiplicity of sample inlet ports, arranged concentrically around the center of the platform, wherein each of the sample inlet ports is operatively linked to a respective one of the multiplicity of microchannels which are arrayed radially away from the center of the platform, said microchannels being operatively linked to

a multiplicity of reagent reservoirs containing a reagent specific for the analyte to be measured, wherein release of the reagent from each of the reservoirs is controlled by a microvalve, and wherein the multiplicity of microchannels is also operatively linked to

a multiplicity of analyte detection chambers arranged peripherally around the outer edge of the microplatform, wherein movement of the biological sample from the sample inlet port and through the microchannel, and movement of the reagent from the reagent reservoir and through the microchannel, is motivated by centripetal force generated by rotational motion of that microsystem platform.

48. Apparatus according to claim 47, wherein the analyte detection chambers are optically-transparent.

49. Apparatus according to claim 47 or claim 48, further comprising electrical wiring between each of the microvalves and an electrical controller unit, wherein valve opening and closing is controlled by electrical signals from the controller unit.

50. Apparatus according to any one of claims 47 to 49, wherein the microchannels are arrayed linearly from the center of the platform to the periphery.

51. Apparatus according to any one of claims 47 to 49, wherein the microchannels are arrayed concentrically from the center of the platform to the periphery.

52. Apparatus according to any one of claims 47 to 51, wherein the micromanipulation device comprises a detecting means.

53. Apparatus according to any one of claims 1 to 46, for detecting gas or particles comprising an environmental sample, wherein the microsystem platform comprises

a multiplicity of sample inlet ports, arranged concentrically around the center of the platform, wherein the sample ports comprise an air intake vent and connecting funnel channel, wherein each of the sample inlet ports is operatively lined to a respective one of the multiplicity of microchannels which are arrayed radially from the center of the platform, said microchannels being operatively linked to

a multiplicity of reagent reservoirs containing a reagent specific for the gas or particles to be detected, wherein release of the reagent from each of the reservoirs is controlled by a microvalve, wherein the microvalves are in electrical contact with a controller unit, and wherein the multiplicity of microchannels is also operatively linked to

a multiplicity of gas or particle detectors arranged peripherally on the microplatform, wherein movement of the environmental sample from the sample inlet port and through the microchannel, and movement of the reagent from the reagent reservoir and through the microchannel, is motivated by centripetal force generated by rotational motion of the microsystem platform.

54. Apparatus according to claim 53, wherein the environmental sample comprises air, water, soil, or disrupted biological matter.

55. Apparatus according to claim 53 or claim 54, wherein the detector comprises a gas sensor chip.

56. Apparatus according to any one of claims 53 to 55, wherein the detector comprises an optically-transparent particle collection chamber.

57. Apparatus according to claim 56, wherein the detector also comprises a coherent light source.

58. Apparatus according to claim 57, wherein the particles are detected by light scattering.

59. Apparatus according to any one of claims 53 to 55, wherein the detector comprises a particle collection chamber operatively connected by a microchannel to a reagent reservoir comprising a reagent for chemically testing the particles.

60. Apparatus according to any one of claims 1 to 46, for determining a hematocrit value from a blood sample, wherein the microsystem platform is comprised of a radial array of microchannels having a diameter of about 100μm wherein the microchannels are treated with heparin to prevent coagulation, and wherein the microchannels are open at one end proximal to the center of the disk, the apparatus also comprising a coherent light source and a recording means operatively connected thereto comprising the micromanipulation device, and wherein movement of the blood sample through the microchannel is motivated by centripetal force generated by rotational motion of the microsystem platform.

61. Apparatus according to claim 60, wherein the coherent light source is mounted on a movable track arrayed radially from the center of rotation of the platform.

62. Apparatus according to claim 60 or claim 61, further comprising a Clarke electrode operatively connected to each of the microchannels of the microsystem platform, wherein the electrode is arranged to be in contact with a blood sample within the microchannel.

63. Apparatus according to any one of claims 60 to 62, further comprising a Severing electrode operatively connected to each of the microchannels of the microsystem platform, wherein the electrode is arranged to be in contact with a blood sample within the microchannel.

64. Apparatus according to any one of claims 1 to 63, wherein the microsystem platform is comprised of a stacked layer of thin film disks comprising microchannels, sample inlet ports, reactant reservoirs, reaction chambers and sample outlet ports, wherein each of the stacked film disks is self-contained and provides a platform of the invention.

65. Apparatus according to any one of claims 1 to 64, wherein the microsystem platform comprises a multiplicity of sample input means, reactant reservoirs, reaction chambers, microvalves and microchannels operatively connected thereto and embedded therein, wherein the microsystem platform is comprised of a stacked array of layers wherein a first layer comprises the sample input means, reactant reservoirs, reaction chambers and microchannels, a second layer comprises the microvalves, a third layer comprises electrical connections from the microvalves to an electrical controller unit, and the fourth layer comprises a sealing layer, wherein the layers are stacked on top of the solid substrate of the microsystems platform and fused thereto.

66. A method for measuring the amount of an analyte in a biological sample, the method comprising the steps of
applying the biological sample to a sample inlet port of a microsystems platform according to any one of claims 47 to 52,
placing the microsystems platform in a micromanipulation device,
providing rotation motion to the microsystems platform for a time and at a velocity sufficient to motivate the biological sample containing the analyte from the sample. inlet port through the microchannel,
opening each of the microvalves controlling release of the reagent from the reagent reservoirs by generating a signal from the controlling unit, at a time and for a duration whereby the reagent moves into the microchannel and is mixed with the biological sample,
observing the mixture of the biological sample and the reagents in the analyte detection chamber, whereby a detector comprising the device detects a signal proportional to the amount of the analyte present in the biological sample, and
recording the measurement of the amount of the analyte in the biological sample.

67. A method according to claim 66, wherein the biological sample is blood, urine, cerebrospinal fluid, plasma, saliva, semen, or amniotic fluid.

68. A method according to claim 66 or claim 67, wherein the measurement of the amount of analyte in the sample is recorded in the device, on the microplatform, or both.

69. A method according to any one of claims 66 to 68, wherein the analyte detection chamber on the microsystem platform is optically transparent.

70. A method according to any one of claims 66 to 69, wherein the signal detected in the.analyte detection chamber is detected at a frequency equal to the frequency of rotation of the platform or multiples thereof.

71. A method according to any one of claims 66 to 70, wherein the signal detected is a monochromatic light signal.

72. A method according to claim 71, wherein the signal detected is a fluorescent signal, a chemiluminescence signal or a colorimetric signal.

73. A method for detecting gas or particles comprising an environmental sample, wherein the method comprises the steps of

contacting the environmental sample with a sample .inlet port of a microsystems platform according to any one of claims 53 to 59,

placing the microsystems platform in a micromanipulation device,

providing rotational motion to the microsystems platform of a time and at a velocity sufficient to motivate the gaseous or particulate environmental sample from the sample inlet port through the microchannel,

opening each of the microvalves controlling release of the reagent from the reagent reservoirs by generating a signal from the controlling unit, at a time and for a duration whereby the reagent moves into the microchannel and is mixed with the environmental sample,

detecting the mixture of the environmental sample and the reagent or the gaseous or particulate component of the environmental sample directly in the gas or particle detection chamber, whereby the detector detects a signal proportional to the amount of the gas or particulate present in the environmental sample, and

recording the measurement of the amount of the gas or particulate in the environmental sample.

74. A method according to claim 73, wherein the environmental sample comprises air, water, soil, or disrupted biological matter.

75. A method according to claim 73 or claim 74, wherein a gas is detected by a gas sensor chip.

76. A method according to any one of claims 73 to 75, wherein a particle is detected in an optically transparent particle collection chamber.

77. A method according to any one of claims 73 to 76, wherein the particle is detected by coherent light scattering.

78. A method according to any one of claims 73 to 77, wherein a particle is detected in a particle collection chamber operatively connected by a microchannel to a reagent reservoir comprising a reagent for chemically testing the particles, wherein the particulate is mixed and reacted with the reagent in the microchannel after release of the reagent by activation of a microvalve and rotation of the platform.

79. A method for determining a hematocrit value from a blood sample, the method comprising the steps of

applying the blood sample to the proximal end of a microchannel of a microsystems platform according to any one of claims 60 to 63,

placing the microsystems platform in a micromanipulation device,

providing rotational motion to the microsystems platform for a time and at a velocity sufficient to motivate the red blood cells comprising the blood sample to move along the extent of the microchannel,

scanning the microchannel along its length with the coherent light source,

detecting a change in light scatter at a position along the microchannel that defines a boundary between the red blood cells and blood plasma,

recording the position of the boundary for each microchannel, and

comparing the position of this boundary for each microchannel with a standard curve relating hematocrit values to the position of the boundary, and recording the hematocrit determined thereby.

80. A method for determining a blood oxygenation value from a blood sample, the method comprising the steps of

applying the blood sample to the proximal end of a microchannel of a microsystems platform according to claim 62,

placing the microsystems platform in a micromanipulation device,

providing rotation motion to the microsystems platform for a time and at a velocity sufficient to motivate the blood sample to come in contact with the Clarke electrode connected to the microchannel,

detecting a blood oxygenation value for the blood sample, and

recording the blood oxygenation value determined thereby.

**Patentansprüche**

1. Mikrohandhabungsapparat für zentripetal-bewegungsverursachte Flüssigkeit, das eine Kombination ist aus

   einer Mikrosystem-Plattform, die ein Substrat mit einer ersten, flachen, planaren Oberfläche und einer ihr gegenüberliegenden zweiten, flachen, planaren Oberfläche aufweist,

   wobei die erste Oberfläche eine Probeneingabeeinrichtung und eine Vielzahl von in die erste planare Oberfläche eingebetteten Mikrokanälen aufweist, jeder Mikrokanal zu einem Luftverdrängungskanal entlüftet ist, um Luftverdrängung durch Flüssigkeitsbewegung durch die durch die Luftverdrängungskanäle zu entlüftenden Mikrokanäle zu ermöglichen, um dadurch Luftdruckwiderstand gegen die Flüssigkeitsbewegung zu verringern, wobei die Probeneingabeeinrichtung und die Mikrokanäle verbunden und in Flüssigkeitsberührung sind, und

   wobei die der ersten flachen, planaren Oberfläche gegenüberliegende zweite flache planare Oberfläche der Plattform mit einem elektromagnetisch lesbaren Befehlsvorrat zur Steuerung von Drehgeschwindigkeit, -dauer oder -richtung der Plattform kodiert ist und ferner einen Befehlsvorrat aus analytischen, diagnostischen oder Qualitätskontrollbefehlen zur Durchführung eines Versuchs enthält, wobei der elektromagnetisch lesbare Befehlsvorrat durch einen CD- oder CDV-Plattenspieler lesbar ist, und

   einem Mikrohandhabungsgerät mit einer Basis, einer Dreheinrichtung zum Drehen der Plattform, einem Netzteil und Einrichtungen der Benutzer-Schnittstelle und Betriebssteuerung, wobei die Dreheinrichtung betriebsmäßig mit der Mikrosystem-Plattform verbunden und mit ihr in Drehkontakt ist,

   wobei ein Volumen einer Flüssigkeit in den Mikrokanälen der Plattform durch diese Mikrokanäle durch Zentripetalkraft bewegt wird, die durch die Drehbewegung der Plattform während einer Zeitdauer und mit einer Drehgeschwindigkeit entsteht, die zur Bewegung der Flüssigkeit durch die Mikrokanäle ausreichen.

2. Apparat nach Anspruch 1, bei dem die Einrichtung zur Betriebssteuerung ein integriertes elektronisches Verarbeitungssystem umfaßt, das durch Befehle gesteuert wird, die der Befehlsvorrat auf der zweiten planaren Oberfläche der Plattform enthält.

3. Apparat nach Anspruch 1 oder Anspruch 2, bei dem die Flüssigkeitsströmung durch die Mikrokanäle durch Kapillar-Mikroventile gesteuert wird, die drehinduzierten Flüssigkeitsdruck benutzen, um Kapillarkräfte zu überwinden.

4. Apparat nach Anspruch 3, bei dem das Mikroventil einen Schnittpunkt zwischen einem unter einem Mikrokanal, einem Behälter, einer Reaktionskammer und einer Nachweiskammer ausgewählten ersten Flüssigkeitsdurchgang mit einem ersten Querschnitt und einem zweiten Flüssigkeitsdurchgang aufweist, in den bei Betrieb des Apparats Flüssigkeit aus dem ersten Flüssigkeitsdurchgang einströmen kann, wobei der zweite Flüssigkeitsdurchgang unter einem Mikrokanal, einem Behälter, einer Reaktionskammer und einer Nachweiskammer ausgewählt ist und einen von dem ersten Querschnitt verschiedenen zweiten Querschnitt hat, wobei der erste Querschnitt im Falle einer den Werkstoff der Flüssigkeitsdurchgänge vollständig oder teilweise benetzenden Flüssigkeit enger als der Querschnitt des zweiten Flüssigkeitsdurchgangs gewählt ist und der Querschnitt des ersten Flüssigkeitsdurchgangs in dem Falle einer den Werkstoff der Flüssigkeitsdurchgänge nicht benetzenden Flüssigkeit größer als der zweite Querschnitt gewählt ist.

5. Apparat nach einem der Ansprüche 1 bis 4, bei dem die erste flache, planare Oberfläche und die zweite flache, planare Oberfläche der Mikrosystem-Plattform eine Scheibe bilden.

6. Apparat nach Anspruch 5, bei dem die Mikrosystem-Plattform eine Scheibe mit einem Radius von etwa 1 bis 25 cm ist.

7. Apparat nach einem der Ansprüche 1 bis 6, bei dem die erste und zweite flache, planare Oberfläche der Mikrosystem-Plattform eine mittig angeordnete Öffnung begrenzt, die mit einer Spindel auf dem Mikrohandhabungsgerät in Eingriff ist, wodurch eine Drehbewegung der Spindel in eine Drehbewegung der Mikrosystem-Plattform umgesetzt wird.

8. Apparat nach einem der Ansprüche 1 bis 7, bei dem die Mikrosystem-Plattform aus einem Material gebaut ist, das unter einem organischen Werkstoff, einem anorganischen Werkstoff, einem kristallinen Werkstoff und einem amorphen Werkstoff ausgewählt ist.

9. Apparat nach Anspruch 8, bei dem die Mikrosystem-Plattform ferner einen unter Silizium, Siliziumdioxid, Quarz, einer Keramik, einem Metall und einem Kunststoff ausgewählten Werkstoff aufweist.

10. Apparat nach einem der Ansprüche 1 bis 9, bei dem die Mikrosystem-Plattform eine Dicke von etwa 0,1 bis 100 mm hat und bei der die Querschnittsdimension der Mikrokanäle zwischen der ersten und zweiten flachen planaren Oberfläche kleiner als 500 μm ist und 1 bis 90 Prozent der genannten Querschnittsdimension der Plattform beträgt.

11. Apparat nach einem der Ansprüche 1 bis 10, bei dem die Mikrosystem-Plattform ferner eine Reaktionskammer und einen Reagenzbehälter umfaßt, die in seine erste planare Oberfläche eingebettet sind.

12. Apparat nach Anspruch 11, bei dem die Probeneingabeeinrichtung, die Mikrokanäle, die Reaktionskammer und der Reagenzbehälter verbunden und in Flüssigkeitskontakt sind und bei dem die Flüssigkeitsbewegung aus den Mikrokanälen, den Reaktionskammern und dem Reagenzbehälter durch an diese angeschlossene Mikroventile gesteuert wird.

13. Apparat nach Anspruch 11 oder Anspruch 12, bei dem die Mikrosystem-Plattform eine Dicke von etwa 0,1 bis 100 mm hat und bei dem die Querschnittsdimension der Reaktionskammer oder des Reagenzbehälters zwischen der ersten und zweiten flachen planaren Oberfläche 1 bis 75 Prozent der genannten Dicke der Plattform beträgt.

14. Apparat nach einem der Ansprüche 11 bis 13, bei dem die Mikrosystem-Plattform eine Vielzahl von Probeneingabeeinrichtungen, Reagenzbehältern, Reaktionskammern und damit verbundenen Mikrokanälen umfaßt, die in ihre erste planare Oberfläche eingebettet sind und bei dem ein Volumen einer Flüssigkeitsprobe durch aus der Rotation der Mikrosystem-Plattform resultierender Zentripetalkraft auf der Plattform von der Probeneingabeeinrichtung in die Reaktionskammern und aus diesen heraus bewegt wird und ein Volumen eines Reagenz aus den Reagenzbehältern in die Reaktionskammern und aus diesen heraus bewegt wird.

15. Apparat nach einem der Ansprüche 1 bis 14, bei dem die Mikrosystem-Plattform für eine Drehung mit einer Drehzahl von etwa 1 bis etwa 30000 UpM eingerichtet ist.

16. Apparat nach einem der Ansprüche 1 bis 15, bei dem die Mikrosystem-Plattform eine Nachweiskammer hat, die in die erste planare Oberfläche der Plattform eingebettet und mit einem Mikrokanal verbunden ist, und bei dem das Mikrohandhabungsgerät eine Nachweiseinrichtung umfaßt, wodurch die Nachweiskammer durch die Nachweiseinrichtung untersucht wird, um eine Untersuchungsausgangsgröße zu liefern.

17. Apparat nach Anspruch 16, bei dem die Nachweiseinrichtung an dem Gerät durch Drehbewegung der Mikrosystem-Plattform in Ausrichtung mit der Nachweiskammer auf der Plattform gebracht wird.

18. Apparat nach Anspruch 16 oder Anspruch 17, bei dem die Nachweiseinrichtung eine Lichtquelle und einen Photodetektor umfaßt.

19. Apparat nach Anspruch 18, bei dem die Lichtquelle die Nachweiskammer beleuchtet, in der Licht quer durch die Nachweiskammer reflektiert und durch den Photodetektor erfaßt wird.

20. Apparat nach Anspruch 19, bei dem die Nachweiskammer auf der Mikrosystem-Plattform optisch durchsichtig ist.

21. Apparat nach einem der Ansprüche 17 bis 20, bei dem die Nachweiseinrichtung stationär ist und die Nachweiskammer mit einer Frequenz abfragt, die gleich der Drehfrequenz der Plattform oder Vielfachen davon ist.

22. Apparat nach Anspruch 21, bei dem die Nachweiseinrichtung eine stroboskopische Lichtquelle ist

23. Apparat nach Anspruch 22, bei dem die Nachweiseinrichtung eine monochromatische Lichtquelle ist.

24. Apparat nach einem der Ansprüche 16 bis 23, bei dem die Nachweiseinrichtung das optische Absorbtionsvermögen, Fluoreszenz, Chemilumineszenz, Lichtstreuung oder Radioaktivität nachweist.

25. Apparat nach einem der Ansprüche 1 bis 24, ferner mit einem Temperatursteuerungselement in thermischem Kontakt mit der Mikroplattform.

26. Apparat nach einem der Ansprüche 1 bis 25, ferner mit einer thermischen Nachweiseinheit in thermischem Kontakt mit der Mikroplattform.

27. Apparat nach einem der Ansprüche 1 bis 26, bei dem die Mikrosystem-Plattform eine mit einem Mikrokanal verbundene Filtereinrichtung umfaßt.

28. Apparat nach einem der Ansprüche 1 bis 26, bei dem die Mikrosystem-Plattform ein an einen Reaktionsbehälter oder einen Mikrokanal angeschlossenes Mischelement umfaßt.

29. Apparat nach Anspruch 28, bei dem die Mikrosystem-Plattform einen statischen Mischer umfaßt, der eine strukturierte Oberfläche eines Reaktionsbehälters oder Mikrokanals enthält.

30. Apparat nach einem der Ansprüche 1 bis 29, bei dem die Mikrosystem-Plattform ein an eine Reaktionskammer oder einen Mikrokanal angeschlossenes Kapillar-Mikroventil aufweist.

31. Apparat nach einem der Ansprüche 1 bis 30, bei dem die Mikrosystem-Plattform ferner eine Vielzahl von Luftkanälen, Abluftöffnungen und Luftverdrängungskanälen aufweist.

32. Apparat nach einem der Ansprüche 1 bis 31, bei dem die Dreheinrichtung des Mikrohandhabungsgeräts ein Elektromotor ist.

33. Apparat nach einem der Ansprüche 1 bis 32, bei dem das Mikrohandhabungsgerät eine Drehsteuerungseinrichtung zur Steuerung der Drehbeschleunigung und -geschwindigkeit der Mikrosystem-Plattform umfaßt.

34. Apparat nach einem der Ansprüche 1 bis 33, bei dem das Mikrohandhabungsgerät eine Benutzer-Schnittstelle enthält, die einen Bildschirm und eine alphanumerische Kleintastatur umfaßt.

35. Apparat nach einem der Ansprüche 1 bis 34, bei dem das Mikrohandhabungsgerät einen Wechselstrom- oder Gleichstromnetzteil umfaßt.

36. Apparat nach einem der Ansprüche 1 bis 35, bei dem die Mikrosystem-Plattform einen elektrischen Stecker in Kontakt mit einem an das Mikrohandhabungsgerät angeschlossenen elektrischen Stecker enthält.

37. Apparat nach einem der Ansprüche 1 bis 36, bei dem das Mikrohandhabungsgerät einen Mikroprozessor und einen daran angeschlossenen Speicher umfaßt.

38. Apparat nach einem der Ansprüche 1 bis 37, bei dem das Mikrohandhabungsgerät eine Leseeinrichtung zum Einlesen des Befehlsvorrats oder eine Schreibeinrichtung umfaßt.

39. Apparat nach Anspruch 38, bei dem die Leseeinrichtung eine CD-Laser-Leseeinrichtung ist.

40. Apparat nach Anspruch 38, bei dem die Schreibeinrichtung eine CD-Schreibeinrichtung ist.

41. Apparat nach einem der Ansprüche 1 bis 40, bei dem die zweite flache planare Oberfläche der Mikrosystem-Plattform mit Maschinensprach-Befehlen kodiert ist.

42. Apparat nach Anspruch 41, bei dem die Maschinensprach-Befehle den Plattformbetrieb, Datenerfassung oder -analyse aus der Plattform, Datenspeicherung und -wiedergewinnung, Kommunikation mit anderen Geräten oder die direkte Diagnose der Geräteleistung steuern.

43. Apparat nach einem der Ansprüche 1 bis 42, bei dem das Mikrohandhabungsgerät einen Nur-Lese-Speicher oder einen Permanentspeicher umfaßt, der mit Maschinensprach-Befehlen kodiert ist.

44. Apparat nach Anspruch 43, bei dem die Maschinensprach-Befehle den Plattformbetrieb, Datenerfassung oder -analyse auf der Plattform, Datenspeicherung und -wiedergewinnung, Kommunikation mit anderen Geräten oder die direkte Diagnose der Geräteleistung steuern.

45. Apparat nach einem der Ansprüche 1 bis 44, ferner mit erster und zweiter Mikrosystem-Plattform, die über eine planare Oberfläche jeder Mikrosystem-Plattform miteinander in Kontakt sind.

46. Apparat nach einem der Ansprüche 1 bis 45, bei dem sich Flüssigkeit auf der Mikrosystem-Plattform in den Mikrokanälen der Plattform mit einer Flüssigkeitsgeschwindigkeit von etwa 0,1 cm/s bis etwa 1000 cm/s bewegt.

47. Apparat nach einem der Ansprüche 1 bis 46 zur Messung der Analytenmenge in einer biologischen Probe, bei dem die Mikrosystem-Plattform

eine Vielzahl von Probeneingabeöffnungen umfaßt, die konzentrisch um die Plattformmitte angeordnet sind, wobei jede der Probeneingabeöffnungen betriebsmäßig mit einem aus der Vielzahl der Mikrokanäle verbunden ist, die von der Plattformmitte weg radial angeordnet sind, wobei die Mikrokanäle betriebsmäßig verbunden sind mit

einer Vielzahl von Reagenzbehältern, die ein für den zu messenden Analyten spezifisches Reagenz enthalten, wobei die Reagenzabgabe aus jedem der Behälter durch ein Mikroventil gesteuert wird und wobei die Vielzahl der Mikrokanäle ebenfalls betriebsmäßig verbunden ist mit

einer Vielzahl von Analyt-Nachweiskammern, die um den Außenrand der Mikroplattform umfangsmäßig verteilt angeordnet sind,

wobei die Bewegung der biologischen Probe von der Probeneingabeöffnung weg und durch den Mikrokanal und die Bewegung des Reagenz aus dem Reagenzbehälter und durch den Mikrokanal durch die durch die Drehbewegung jener Mikrosystem-Plattform erzeugte Zentripetalkraft angetrieben wird.

48. Apparat nach Anspruch 47, bei dem die Analyt-Nachweiskammern optisch durchsichtig sind.

49. Apparat nach Anspruch 47 oder Anspruch 48, ferner mit elektrischer Verdrahtung zwischen jedem der Mikroventile und einer elektrischen Steuereinheit, wobei das Öffnen und Schließen der Ventile durch elektrische Signale von der Steuereinheit gesteuert werden.

50. Apparat nach einem der Ansprüche 47 bis 49, bei dem die Mikrokanäle von der Mitte der Plattform geradlinig zu dem Umfang hin angeordnet sind.

51. Apparat nach einem der Ansprüche 47 bis 49, bei dem die Mikrokanäle konzentrisch von der Mitte der Plattform zum Umfang hin angeordnet sind.

52. Apparat nach einem der Ansprüche 47 bis 51, bei dem das Mikrohandhabungsgerät eine Nachweiseinrichtung umfaßt.

53. Apparat nach einem der Ansprüche 1 bis 46 für den Nachweis von Gas oder Teilchen, die eine Umweltprobe aufweist, bei dem die Mikrosystem-Plattform

eine Vielzahl von Probeneingangsöffnungen umfaßt, die konzentrisch um die Plattformmitte angeordnet sind, wobei die Probenöffnungen eine Luftansaugöffnung und anschließenden Trichterkanal umfaßt, wobei jede der Probeneingabeöffnungen betriebsmäßig mit einem aus der Vielzahl der Mikrokanäle verbunden ist, die von der Plattformmitte weg radial angeordnet sind, wobei die Mikrokanäle betriebsmäßig verbunden sind mit

einer Vielzahl von Reagenzbehältern, die ein für das nachzuweisende Gas oder die nachzuweisenden Teilchen spezifisches Reagenz enthalten, wobei die Reagenzabgabe aus jedem der Behälter durch ein Mikroventil gesteuert wird, wobei die Mikroventile mit einer Steuereinheit in elektrischem Kontakt sind und wobei die Vielzahl der Mikrokanäle ebenfalls betriebsmäßig verbunden ist mit

einer Vielzahl von Gas- oder Teilchendetektoren, die am Umfang auf der Mikroplattform angeordnet sind,

wobei die Bewegung der Umweltprobe von der Probeneingabeöffnung weg und durch den Mikrokanal und die Bewegung des Reagenz aus dem Reagenzbehälter und durch den Mikrokanal durch die durch die Drehbewegung jener Mikrosystem-Plattform erzeugte Zentripetalkraft angetrieben wird.

54. Apparat nach Anspruch 53, bei dem die Umweltprobe Luft, Wasser, Boden oder zertrümmerte biologische Materie ist.

55. Apparat nach Anspruch 53 oder Anspruch 54, bei dem der Detektor ein Gassensor-Chip umfaßt.

56. Apparat nach einem der Ansprüche 53 bis 55, bei dem der Detektor eine optisch durchsichtige Teilchensammelkammer umfaßt.

57. Apparat nach Anspruch 56, bei dem der Detektor auch eine kohärente Lichtquelle umfaßt.

58. Apparat nach Anspruch 57, bei dem die Teilchen durch Lichtstreuung nachgewiesen werden.

**59.** Apparat nach einem der Ansprüche 53 bis 55, bei dem der Detektor eine Teilchen-Sammelkammer umfaßt, die durch einen Mikrokanal betriebsmäßig mit einem Reagenzbehälter verbunden ist, der ein Reagenz zur chemischen Prüfung der Teilchen enthält.

**60.** Apparat nach einem der Ansprüche 1 bis 46 zur Bestimmung eines Hämatokritwerts aus einer Blutprobe, bei dem die Mikrosystem-Plattform eine radiale Anordnung von Mikrokanälen mit einem Durchmesser von etwa 100 μm enthält, die zur Verhinderung einer Koagulation mit Heparin behandelt sind, und bei dem die Mikrokanäle an einem Ende in der Nähe der Mitte der Scheibe offen sind, wobei der Apparat auch eine kohärente Lichtquelle und eine damit betriebsmäßig verbundene, das Mikrohandhabungsgerät umfassende Aufzeichnungseinrichtung enthält, und bei dem die Bewegung der Blutprobe durch den Mikrokanal durch die durch die Drehbewegung der Mikrosystem-Plattform erzeugte Zentripetalkraft angetrieben wird.

**61.** Apparat nach Anspruch 60, bei dem die kohärente Lichtquelle mit einer beweglichen Schiene angebracht ist, die von dem Drehmittelpunkt der Plattform radial angeordnet ist.

**62.** Apparat nach Anspruch 60 oder Anspruch 61, ferner mit einer Clarke-Elektrode, die an jeden der Mikrokanäle der Mikrosystem-Plattform betriebsmäßig angeschlossen ist, wobei die Elektrode so angeordnet ist, daß sie mit der Blutprobe in dem Mikrokanal in Berührung ist.

**63.** Apparat nach einem der Ansprüche 60 bis 62, ferner mit einer Severing-Elektrode, die an jeden der Mikrokanäle der Mikrosystem-Plattform betriebsmäßig angeschlossen ist, wobei die Elektrode so angeordnet ist, daß sie mit der Blutprobe in dem Mikrokanal in Berührung ist.

**64.** Apparat nach einem der Ansprüche 1 bis 63, bei dem die Mikrosystem-Plattform aus einer gestapelten Schicht von Dünnfilmscheiben besteht, die Mikrokanäle, Probeneingabeöffnungen, Reaktionsmittelbehälter, Reaktionskammern und Probenaustrittsöffnungen aufweisen, wobei jeder der gestapelten Filmscheiben unabhängig ist und eine Plattform der Erfindung bildet.

**65.** Apparat nach einem der Ansprüche 1 bis 64, bei dem die Mikrosystem-Plattform eine Vielzahl von Probeneingabeeinrichtungen, Reaktionsmittelbehältern, Reaktionskammern, Mikroventilen und betriebsmäßig damit verbundenen und darin eingebetteten Mikrokanälen aufweist, wobei die Mikrosystem-Plattform aus einer gestapelten Anordnung von Schichten besteht, wobei eine erste Schicht die Probeneingabeeinrichtung, Reaktionsmittelbehälter, Reaktionskammern und Mikrokanäle aufweist, eine zweite Schicht die Mikroventile aufweist, eine dritte Schicht elektrische Verbindungen von den Mikroventilen zu der elektrischen Steuereinheit enthält und die vierte Schicht eine Dichtungsschicht bildet, wobei die Schichten auf die Oberseite des festen Substrats der Mikrosystem-Plattform gestapelt und mit diesem verschmolzen sind.

**66.** Verfahren zur Messung der Menge eines Analyten in einer biologischen Probe mit den Stufen der

Aufbringung der biologischen Probe auf die Probeneingabeöffnung einer Mikrosystem-Plattform nach einem der Ansprüche 47 bis 52,

Einsetzung der Mikrosystem-Plattform in ein Mikrohandhabungsgerät,

Aussetzung der Mikrosystem-Plattform einer Drehbewegung für eine Zeit und mit einer Geschwindigkeit, die ausreichen, um die den Analyten enthaltende biologische Probe von der Probeneintrittsöffnung durch den Mikrokanal zu treiben,

Öffnung jedes der die Abgabe des Reagenz aus den Reagenzbehältern steuernden Mikroventile durch Generierung eines Signals von der Steuereinheit zu einer Zeit und für eine Zeitdauer, wodurch sich das Reagenz in den Mikrokanal bewegt und mit der biologischen Probe gemischt wird,

Beobachtung des Gemisches der biologischen Probe und der Reagenzien in der Analyt-Nachweiskammer, wobei ein im Gerät enthaltener Detektor ein zu der in der biologischen Probe vorliegende Menge des Analyten proportionales Signal erfaßt, und

Aufzeichnung der Messung der Menge des Analyten in der biologischen Probe.

**67.** Verfahren nach Anspruch 66, bei dem die biologische Probe Blut, Urin, Hirn-Rückenmark-Flüssigkeit, Plasma, Speichel, Sperma oder Fruchtwasser ist.

**68.** Verfahren nach Anspruch 66 oder Anspruch 67, bei dem die Messung der Menge des Analyten in der Probe in dem Gerät, auf der Mikroplattform oder in beiden aufgezeichnet wird.

**69.** Verfahren nach einem der Ansprüche 66 bis 68, bei dem die Analyt-Nachweiskammer auf der Mikrosystem-Plattform optisch durchsichtig ist.

**70.** Verfahren nach einem der Ansprüche 66 bis 69, bei dem das in der Analyt-Nachweiskammer erfaßte Signal bei einer Frequenz erfaßt wird, die gleich der Drehfrequenz der Plattform oder Vielfachen davon ist.

**71.** Verfahren nach einem der Ansprüche 66 bis 70, bei dem das erfaßte Signal ein monochromatisches Lichtsignal ist.

**72.** Verfahren nach Anspruch 71, bei dem das erfaßte Signal ein Fluoreszenzsignal, ein Chemilumineszenzsignal oder ein kolorimetrisches Signal ist.

**73.** Verfahren zum Nachweis von Gas oder Teilchen, die in einer Umweltprobe enthalten sind, mit den Stufen der
Kontaktierung der Umweltprobe mit einer Probeneintrittsöffnung einer Mikrosystem-Plattform nach einem der Ansprüche 53 bis 59,
Einsetzung der Mikrosystem-Plattform in ein Mikrohandhabungsgerät,
Erteilung einer Drehbewegung der Mikrosystem-Plattform in einer Zeit und mit einer Geschwindigkeit, die ausreichen, um die gasförmige oder teilchenförmige Umweltprobe von der Probeneintrittsöffnung durch den Mikrokanal zu bewegen,
Öffnung jedes der die Abgabe des Reagenz aus den Reagenzbehältern steuernden Mikroventile durch Generierung eines Signals von der Steuereinheit zu einer Zeit und für eine Zeitdauer, wodurch sich das Reagenz in den Mikrokanal bewegt und mit der Umweltprobe gemischt wird,
Erfassung des Gemisches der Umweltprobe und des Reagenz oder der gasförmigen oder teilchenförmigen Komponente der Umweltprobe direkt in der Gas- oder Teilchen-Nachweiskammer, wobei der Detektor ein Signal erfaßt, das proportional der in der Umweltprobe vorliegenden Gas- oder Teilchenmenge ist, und
Aufzeichnung der Messung der Gas- oder Teilchenmenge in der Umweltprobe.

**74.** Verfahren nach Anspruch 73, bei dem die Umweltprobe Luft, Wasser, Boden oder zertrümmerte biologische Materie ist.

**75.** Verfahren nach Anspruch 73 oder Anspruch 74,bei dem ein Gas durch ein Gassensor-Chip nachgewiesen wird.

**76.** Verfahren nach einem der Ansprüche 73 bis 75, bei dem ein Teilchen in einer optisch durchsichtigen Teilchen-Sammelkammer nachgewiesen wird.

**77.** Verfahren nach einem der Ansprüche 73 bis 76, bei dem das Teilchen durch Streuung von kohärentem Licht nachgewiesen wird.

**78.** Verfahren nach einem der Ansprüche 73 bis 77, bei dem ein Teilchen in einer Teilchen-Sammelkammer nachgewiesen wird, die durch einen Mikrokanal betriebsmäßig mit einem Reagenzbehälter verbunden ist, der ein Reagenz für die chemische Prüfung der Teilchen enthält, wobei das Teilchenmaterial mit dem Reagenz in dem Mikrokanal gemischt und umgesetzt wird, nachdem das Reagenz durch Aktivierung eines Mikroventils und Rotation der Plattform freigegeben wird.

**79.** Verfahren zur Bestimmung eines Hämatokrit-Werts aus einer Blutprobe mit den Stufen der
Aufbringung der Blutprobe auf das proximale Ende eines Mikrokanals einer Mikrosystem-Plattform nach einem der Ansprüche 60 bis 63,
Einsetzung der Mikrosystem-Plattform in ein Mikrohandhabungsgerät,
Versetzung der Mikrosystem-Plattform in eine Drehbewegung für eine Zeit und mit einer Geschwindigkeit, die ausreichen, um die rote Blutkörperchen enthaltende Blutprobe längs der Strecke des Mikrokanals zu bewegen,
Abtastung des Mikrokanals längs seiner Länge mit der kohärenten Lichtquelle,
Erfassung einer Änderung der Lichtstreuung an einer Stelle entlang dem Mikrokanal, die eine Grenze zwischen Blutplasma und den roten Blutkörperchen definiert,
Aufzeichnung der Position der Grenze für jeden Mikrokanal und
Vergleichung der Position dieser Grenze für jeden Mikrokanal mit einer Standardkurve, die Hämatokritwerte mit der Lage der Grenze in Beziehung setzt, und Aufzeichnung des dadurch bestimmten Hämatokrits.

**80.** Verfahren zur Bestimmung eines Sauerstoffsättigungswertes des Bluts aus einer Blutprobe mit den Stufen der
Aufbringung der Blutprobe auf das proximale Ende eines Mikrokanals einer Mikrosystem-Plattform nach Anspruch 62,

Einsetzung der Mikrosystem-Plattform in ein Mikrohandhabungsgerät,

Aussetzung der Mikrosystem-Plattform einer Drehbewegung für eine Zeit und mit einer Geschwindigkeit, die ausreichen, um die Blutprobe zu bewegen, damit sie mit der an den Mikrokanal angeschlossenen Clarke-Elektrode in Kontakt kommt,

Erfassung eines Blut-Sauerstoffsättigungswertes für die Blutprobe, und

Aufzeichnung des dadurch bestimmten Blut-Sauerstoffsättigungswertes.

**Revendications**

1. Appareil de micromanipulation centripète des liquide qui est une combinaison de :

une plateforme de microsystème, comprenant un substrat ayant une première surface plane plate et une seconde surface plane plate à l'opposé de celle-ci,

dans lequel la première surface comprend un moyen d'entrée des échantillons et une multiplicité de microcanaux intégrés dans la première surface plane, chaque microcanal étant ventilé par un canal de déplacement d'air pour permettre le déplacement de l'air par mouvement de liquide à travers les microcanaux devant être ventilés par les canaux de déplacement de l'air de réduire ainsi la résistance de la pression de l'air au mouvement des liquides, dans lequel le moyen d'entrée des échantillons et les microcanaux sont reliés et en contact fluidique, et

dans lequel la seconde surface plane plate située à l'opposé de la première surface plane plate de la plateforme est encodée à l'aide d'un ensemble d'instructions électromagnétiquement lisibles pour contrôler la vitesse de rotation, la durée ou la direction de la plateforme et comprenant en outre une série d'instructions comprenant des instructions de contrôle analytique, de diagnostique ou de qualité pour effectuer une analyse, la série d'instructions électromagnétiquement lisibles étant lisible à l'aide d'un lecteur de disque CD OU DVD, et

un dispositif de micromanipulation, comprenant une base, un moyen de rotation pour faire tourner la plateforme, une source d'énergie et une interface utilisateur et un moyen de contrôle des opérations, dans lequel le moyen de rotation est relié de manière opérationnelle à la plateforme de microsystème et est en contact rotationnel avec celle-ci

dans lequel un volume de liquide dans les microcanaux de la plateforme est déplacé à travers lesdits microcanaux par force centrifuge provenant du mouvement de rotation de la plateforme pendant une durée et avec un vélocité rotationnelle suffisantes pour déplacer le liquide à travers les microcanaux.

2. Appareil selon la revendication 1, dans lequel le moyen de contrôle des opérations comprend un système de traitement électronique intégré qui est dirigé par des instructions comprenant la série d'instructions sur la seconde surface plane de la plateforme.

3. Appareil selon la revendication 1 ou la revendication 2, dans lequel le flux de liquide à travers les microcanaux est contrôlé par des microvalves capillaires qui utilisent la pression induite par la rotation des fluides pour surmonter les forces capillaires.

4. Appareil selon la revendication 4, dans lequel la microvalve comprend une intersection entre un premier passage pour liquide choisi parmi un microcanal, un réservoir, une chambre de réaction et une chambre de détection, ledit premier passage pour liquide ayant une première coupe transversale, et un second passage pour fluide dans lequel le liquide peut s'écouler depuis le premier passage pour liquide lorsqu'on fait fonctionner l'appareil, ledit second passage pour liquide étant choisi parmi un microcanal, un réservoir et une chambre de réaction et ledit second passage pour liquide ayant une première coupe transversale différente de la première coupe transversale, la première coupe transversale étant choisie, dans le cas d'un liquide qui mouille complètement ou qui mouille partiellement la matière des passages pour liquide, de manière à être plus étroite que la coupe transversale du second passage pour liquide, et la section transversale du premier passage pour liquide étant choisie, dans le cas de liquide qui ne mouille pas la matière des passages pour liquide, de manière à être plus large que la seconde coupe transversale.

5. Appareil selon l'une quelconque des revendications 1 à 4, dans lequel la première surface plane plate et la seconde surface plane plate de la plateforme de microsystème forment un disque.

6. Appareil selon la revendication 5, dans lequel 1a plateforme de microsystème est un disque ayant un rayon d'environ 1 à 25 cm.

7. Appareil selon l'une quelconque des revendications 1 à 6, dans lequel la première et la seconde surfaces planes plates de la plateforme de microsystème définit une ouverture centralement située qui est en prise avec une tige sur le dispositif de microcirculation, moyennant quoi le mouvement de rotation de la tige est traduit en mouvement de rotation de la plateforme de microsystème.

8. Appareil selon l'une quelconque des revendications 1 à 7, dans lequel la plateforme de microsystème est constituée d'une matière choisie parmi une matière organique, une matière inorganique, une matière cristalline et une matière amorphe.

9. Appareil selon la revendication 8, dans lequel la plateforme de microsystème comprend en outre une matière choisie parmi le silicium, la silice, le quartz, une céramique, un métal et un plastique.

10. Appareil selon l'une quelconque des revendications 1 à 9, dans lequel la plateforme de microsystème a une épaisseur d'environ 0,1 à 100 mm, et dans lequel la dimension de la coupe transversale des microcanaux entre la première et la seconde surfaces planes plates est inférieure à 500 µm et de 1 à 90 pour cent de ladite dimension de la plateforme.

11. Appareil selon l'une quelconque des revendications 1 à 10, dans lequel la plateforme de microsystème comprend en outre une chambre de réaction et un réservoir de réactif intégrée dans la surface plane plate de celui-ci.

12. Appareil selon la revendication 11, dans lequel le moyen d'entrée des échantillons, les microcanaux, la chambre de réaction et le réservoir de réactif sont reliés et en contact fluidique, et dans lequel le mouvement des liquides des microcanaux, des chambres de réaction et du réservoir de réactif est contrôlé par des microvalves reliées à ceux-ci.

13. Appareil selon la revendication 11 ou la revendication 12, dans lequel la plateforme de microsystème a une épaisseur d'environ 0,1 à 100 mm, et dans lequel la dimension de la coupe transversale de la chambre de réaction ou du réservoir de réactif entre la première et la seconde surfaces planes plates est comprise entre 1 et 75 pour cent de ladite épaisseur de la plateforme.

14. Appareil selon l'une quelconque des revendications 11 à 13, dans lequel la plateforme de microsystème comprend une multiplicité de moyens d'entrée des échantillons, de réservoirs de réactif, de chambres de réaction et de microcanaux reliés à celui-ci et intégrés dans la première surface plane plate de celui-ci, dans lequel un volume d'un échantillon de liquide est déplacé sur la plateforme depuis le moyen d'entrée des échantillons dans et hors des chambres de réaction, et un volume d'un réactif est déplacé depuis les réservoirs de réactif dans et hors des chambres de réaction, par force centripète provenant de la rotation de la plateforme de microsystème.

15. Appareil selon l'une quelconque des revendications 1 à 14, dans lequel la plateforme de microsystème est disposée de manière à être tournée avec une vélocité rotationnelle d'environ 1 à environ 30 000 tr./min.

16. Appareil selon la revendication 15, dans lequel la plateforme de microsystème comprend une chambre de détection intégrée dans la première surface plane de la plateforme et reliée à un microcanal, et dans lequel le dispositif de micromanipulation comprend un moyen de détection, moyennant quoi la chambre de détection est analysée par le moyen de détection pour produire un rendement d'analyse.

17. Appareil selon la revendication 16, dans lequel le moyen de détection situé sur le dispositif est disposé dans l'alignement avec la chambre de détection sur la plateforme par un mouvement rotationnel de la plateforme de microsystème.

18. Appareil selon la revendication 16 ou la revendication 17, dans lequel le moyen de détection comprend une source de lumière et un photodétecteur.

19. Appareil selon la revendication 18, dans lequel la source de lumière illumine la chambre de détection dans laquelle la lumière est réfléchie de manière transversale à travers la chambre de détection et détectée par un photodétecteur.

**20.** Appareil selon la revendication 19, dans lequel la chambre de détection sur la plateforme de microsystème est optiquement transparente.

**21.** Appareil selon l'une quelconque des revendications 17 à 20, dans lequel le moyen de détection est stationnaire et procède à l'échantillonnage de la chambre de détection à une fréquence égale à la fréquence de rotation de la plateforme ou à des multiples de celle-ci.

**22.** Appareil selon la revendication 21, dans lequel le moyen de détection comprend une source de lumière stroboscopique.

**23.** Appareil selon la revendication 22, dans lequel le moyen de détection est une source de lumière monochromatique.

**24.** Appareil selon l'une quelconque des revendications 16 à 23, dans lequel le moyen de détection détecte l'absorbance optique, la fluorescence, la chimioluminescence, la diffusion de la lumière ou la radioactivité.

**25.** Appareil selon l'une quelconque des revendications 1 à 24, comprenant en outre un élément de contrôle de la température en contact thermique avec la microplateforme.

**26.** Appareil selon l'une quelconque des revendications 1 à 25, comprenant en outre une unité de détection thermique en contact thermique avec la microplateforme.

**27.** Appareil selon l'une quelconque des revendications 1 à 26, dans lequel la plateforme de microsystème comprend un moyen de filtrage relié à un microcanal.

**28.** Appareil selon l'une quelconque des revendications 1 à 26, dans lequel la plateforme de microsystème comprend un élément de mélange relié à un réservoir de réaction ou à un microcanal.

**29.** Appareil selon la revendication 28, dans lequel la plateforme de microsystème comprend un mélangeur statique comprenant une surface texturée d'un réservoir de réaction ou d'un microcanal.

**30.** Appareil selon l'une quelconque des revendications 1 à 29, dans lequel la plateforme de microsystème comprend une microvalve capillaire reliée à une chambre de réaction ou à un microcanal.

**31.** Appareil selon l'une quelconque des revendications 1 à 30, dans lequel une plateforme de microsystème comprend en outre une multiplicité de canaux d'air, de voies d'accès pour l'air d'échappement et de canaux de déplacement de l'air.

**32.** Appareil selon l'une quelconque des revendications 1 à 31, dans lequel le moyen de rotation du dispositif de micromanipulation est un moteur électrique.

**33.** Appareil selon l'une quelconque des revendications 1 à 32, dans lequel le dispositif de micromanipulation comprend un moyen de contrôle du mouvement de rotation destiné à contrôler l'accélération rotationnelle et la vélocité de la plateforme de microsystème.

**34.** Appareil selon l'une quelconque des revendications 1 à 33, dans lequel le dispositif de micromanipulation inclut une interface utilisateur comprenant un écran et un clavier alphanumérique.

**35.** Appareil selon l'une quelconque des revendications 1 à 34, dans lequel le dispositif de micromanipulation comprend une source de courant alternatif ou de courant direct.

**36.** Appareil selon l'une quelconque des revendications 1 à 35, dans lequel la plateforme de microsystème comprend un connecteur électrique en contact avec un connecteur électrique relié au dispositif de micromanipulation.

**37.** Appareil selon l'une quelconque des revendications 1 à 36, dans lequel le dispositif de micromanipulation comprend un microprocesseur et une mémoire reliée à celui-ci.

**38.** Appareil selon l'une quelconque des revendications 1 à 37, dans lequel le dispositif de micromanipulation comprend un moyen de lecture destiné à lire la série d'instructions ou un moyen d'écriture.

**39.** Appareil selon la revendication 38, dans lequel le moyen de lecture est un moyen de lecture disque compact laser.

**40.** Appareil selon la revendication 38, dans lequel le moyen d'écriture est un moyen d'écriture disque compact.

**41.** Appareil selon l'une quelconque des revendications 1 à 40, dans lequel la seconde surface plane plate de la plateforme de microsystème est encodée à l'aide d'instructions machine.

**42.** Appareil selon la revendication 41, dans lequel les instructions machine contrôlent le fonctionnement de la plateforme, de l'acquisition ou de l'analyse des données par la plateforme, du stockage et du retrait des données, de la communication à d'autres dispositifs ou de la performance directe de diagnostiques par l'appareil.

**43.** Appareil selon l'une quelconque des revendications 1 à 42, dans lequel le dispositif de micromanipulation comprend une mémoire morte ou une mémoire de stockage permanent qui est encodée à l'aide d'instructions machine.

**44.** Appareil selon la revendication 43, dans lequel les instructions machine contrôlent le fonctionnement de la plateforme, de l'acquisition ou de l'analyse des données par la plateforme, du stockage et du retrait des données, de la communication à d'autres dispositifs ou de la performance directe de diagnostiques par l'appareil.

**45.** Appareil selon l'une quelconque des revendications 1 à 44, comprenant en outre une première et une seconde plateformes de microsystème en contact l'une avec l'autre à travers une surface plane de chaque plateforme de microsystème.

**46.** Appareil selon l'une quelconque des revendications 1 à 45, dans lequel le liquide sur la plateforme de microsystème est déplacé dans les microcanaux de la plateforme avec une vélocité de liquide allant d'environ 0,1 cm / sec à environ 1 000 cm / sec.

**47.** Appareil selon l'une quelconque des revendications 1 à 46, destiné à mesurer la quantité de substance à analyser dans un échantillon biologique, dans lequel la plateforme de microsystème comprend
une multiplicité de voies d'accès pour l'entrée des échantillons, disposées de manière concentrique autour du centre de la plateforme, dans lequel chacune des voies d'accès pour l'entrée des échantillons est opérationnellement liée à l'un des microcanaux respectifs de la multiplicité de microcanaux qui sont disposés radialement à distance du centre de la plateforme, lesdits microcanaux étant opérationnellement liés à
une multiplicité de réservoirs de réactif contenant un réactif spécifique pour la substance à analyser destinée à être mesurée, dans lequel la libération du réactif par chacun des réservoirs est contrôlée par une microvalve, et dans lequel une multiplicité de microcanaux est également opérationnellement liée à
une multiplicité de chambres de détection de la substance à analyser disposée de manière périphérique autour du bord externe de la microplateforme,
dans lequel le mouvement de l'échantillon biologique depuis la voie d'accès pour l'entrée des échantillons et par les microcanal, et le mouvement du réactif depuis le réservoir de réactif et par le microcanal, est motivé par la force centripète engendrée par le mouvement de rotation de cette plateforme de microsystème.

**48.** Appareil selon la revendication 47, dans lequel les chambres de détection de la substance à analyser sont optiquement transparentes.

**49.** Appareil selon la revendication 47 ou la revendication 48, comprenant en outre des câbles électriques entre chacune des microvalves et une unité de contrôle électrique, dans lequel l'ouverture et la fermeture des valves est contrôlé par des signaux électriques provenant de l'unité de contrôle.

**50.** Appareil selon l'une quelconque des revendications 47 à 49, dans lequel les microcanaux sont disposés de manière linéaire depuis le centre de la plateforme jusqu'à la périphérie.

**51.** Appareil selon l'une quelconque des revendications 47 à 49, dans lequel les microcanaux sont disposés de manière concentrique depuis le centre de la plateforme vers la périphérie.

**52.** Appareil selon l'une quelconque des revendications 47 à 51, dans lequel le dispositif de micromanipulation comprend un moyen de détection.

**53.** Appareil selon l'une quelconque des revendications 1 à 46, pour détecter du gaz ou des particules comprenant un échantillon environnemental, dans lequel la plateforme de microsystème comprend

une multiplicité de voies d'accès pour l'entrée des échantillons, disposées concentriquement autour du centre de la plateforme, dans lequel les voies d'accès pour les échantillons comprennent une ventilation d'apport d'air et un canal de connexion en forme d'entonnoir, dans lequel chacune des voies d'accès pour l'entrée des échantillons est liée de manière opérationnelle à l'un des microcanaux respectifs de la multiplicité de microcanaux qui sont disposés radialement à partir du centre de la plateforme, lesdits microcanaux étant opérationnellement liés à

une multiplicité de réservoirs de réactif contenant un réactif spécifique pour le gaz ou les particules à détecter, dans lequel la libération du réactif par chacun des réservoirs est contrôlée par une microvalve, dans lequel les microvalves sont en contact électrique avec une unité de contrôle, et dans lequel la multiplicité de microcanaux est également opérationnellement liée à

une multiplicité de détecteurs de gaz ou de particules disposée de manière périphérique sur la microplateforme,

dans lequel le mouvement de l'échantillon environnemental depuis la voie d'accès pour l'entrée des échantillons et à travers le microcanal, et le mouvement du réactif depuis le réservoir de réactif et à travers le microcanal, est motivé par la force centripète engendrée par le mouvement rotationnel de la plateforme de microsystème.

**54.** Appareil selon la revendication 53, dans lequel l'échantillon environnemental comprend de l'air, de l'eau, du sol ou de la matière biologique en vrac.

**55.** Appareil selon la revendication 53 ou la revendication 54, dans lequel le détecteur comprend une puce de captage du gaz.

**56.** Appareil selon l'une quelconque des revendications 53 à 55, dans lequel le détecteur comprend une chambre de collecte des particules optiquement transparente.

**57.** Appareil selon la revendication 56, dans lequel le détecteur comprend également une source de lumière cohérente.

**58.** Appareil selon la revendication 57, dans lequel les particules sont détectées par diffusion de lumière.

**59.** Appareil selon l'une quelconque des revendications 53 à 55, dans lequel le détecteur comprend une chambre de collecte des particules opérationnellement reliée à un microcanal à un réservoir de réactif comprenant un réactif pour tester chimiquement les particules.

**60.** Appareil selon l'une quelconque des revendications 1 à 46, pour déterminer une valeur de l'hématocrite d'un échantillon de sang, dans lequel la plateforme de microsystème est constituée d'une série radiale de microcanaux ayant un diamètre d'environ 100 μm dans lequel les microcanaux sont traités avec de l'héparine pour empêcher la coagulation, et dans lequel les microcanaux sont ouverts au niveau d'une extrémité proche du centre du disque, l'appareil comprenant également une source de lumière cohérente et un moyen d'enregistrement relié de manière opérationnelle à celui-ci comprenant le dispositif de micromanipulation, et dans lequel le mouvement de l'échantillon de sang à travers le microcanal est motivé par la force centripète engendrée par le mouvement de rotation de la plateforme de microsystème.

**61.** Appareil selon la revendication 60, dans lequel la source de lumière cohérente est montée sur un rail mobile rangé radialement à partir du centre de rotation de la plateforme.

**62.** Appareil selon l'une quelconque des revendications 60 à 61, comprenant en outre une électrode Clarke reliée de manière opérationnelle à chacun des microcanaux de la plateforme de microsystème, dans lequel l'électrode est disposée de manière à être en contact avec l'échantillon de sang dans le microcanal.

**63.** Appareil selon l'une quelconque des revendications 60 à 62, comprenant en outre une électrode Severing reliée de manière opérationnelle à chacun des microcanaux de la plateforme de microsystème, dans lequel l'électrode est disposée de manière à être en contact avec l'échantillon de sang dans le microcanal.

**64.** Appareil selon l'une quelconque des revendications 1 à 63, dans lequel la plateforme de microsystème est constituée d'une couche empilée de microcanaux comprenant des disques de film fin, de voies d'accès pour l'entrée des échantillons, de réservoirs de réactif, de chambres de réaction et de voies d'accès pour la sortie des échantillons, dans lequel chacun des disques de film empilé est indépendant et fournit une plateforme de l'invention.

**EP 0 865 606 B1**

**65.** Appareil selon l'une quelconque des revendications 1 à 64, dans lequel la plateforme de microsystème comprend une multiplicité de moyens d'entrée pour les échantillons, de réservoirs de réactif, de chambres de réaction, de microvalves et de microcanaux reliée de manière opérationnelle à celui-ci et intégrés dans celui-ci, dans lequel la plateforme de microsystème est constituée d'une série de couches empilées dans laquelle une première couche comprend les moyens d'entrée pour les échantillons, les réservoirs de réactif, les chambres de réaction et les microcanaux, et une seconde couche comprend les microvalves, une troisième couche comprend les connexions électriques allant des microvalves à une unité de contrôle électrique, et la quatrième couche comprend une couche de scellage, dans lequel les couches sont empilées au-dessus du substrat solide de la plateforme de microsystèmes et fondus à celle-ci.

**66.** Procédé de mesure de la quantité d'une substance à analyser dans un échantillon biologique, le procédé comprenant les étapes consistant à

appliquer l'échantillon biologique à une voie d'accès pour l'entrée des échantillons d'une plateforme de microsystèmes selon l'une quelconque des revendications 47 à 52,

placer la plateforme de microsystèmes dans un dispositif de manipulation,

fournir un mouvement de rotation à la plateforme de microsystèmes pendant une durée et avec une vélocité suffisantes pour faire déplacer l'échantillon biologique contenant la substance à analyser depuis la voie d'accès pour l'entrée des échantillons à travers le microcanal,

ouvrir chacune des microvalves contrôlant la libération du réactif des réservoirs de réactif en créant un signal de l'unité de contrôle, à un moment et pour une durée moyennant lesquelles le réactif se déplace dans le microcanal et est mélangé avec l'échantillon biologique,

observer le mélange de l'échantillon biologique et des réactifs dans la chambre de détection de la substance à analyser, moyennant quoi un détecteur comprenant le dispositif détecte un signal proportionnel à la quantité de substance à analyser présente dans l'échantillon biologique, et

enregistrer la mesure de la quantité de substance à analyser dans l'échantillon biologique.

**67.** Procédé selon la revendication 66, dans lequel l'échantillon biologique est du sang, de l'urine, du liquide cérébrospinal, du plasma, de la salive, du sperme ou du liquide amniotique.

**68.** Procédé selon la revendication 66 ou la revendication 67, dans lequel la mesure de la quantité de substance à analyser dans l'échantillon est enregistrée dans le dispositif, sur la microplateforme, ou sur les deux.

**69.** Procédé selon l'une quelconque des revendications 66 à 68, dans lequel la chambre de détection de la substance à analyser sur la plateforme de microsystème est optiquement transparente.

**70.** Procédé selon l'une quelconque des revendications 66 à 69, dans lequel le signal détecté dans la chambre de détection de la substance à analyser est détecté à une fréquence égale à la fréquence de rotation de la plateforme ou à des multiples de celle-ci.

**71.** Procédé selon l'une quelconque des revendications 66 à 70, dans lequel le signal détecté est un signal de lumière monochromatique.

**72.** Procédé selon la revendication 71, dans lequel le signal détecté est un signal fluorescent, un signal chimioluminescent ou un signal colorimétrique.

**73.** Procédé de détection de gaz ou de particules comprenant un échantillon environnemental, dans lequel le procédé comprend les étapes consistant à

mettre en contact l'échantillon environnemental avec une voie d'accès pour l'entrée des échantillons d'une plateforme de microsystèmes selon l'une quelconque des revendications 53 à 59,

placer la plateforme de microsystèmes dans un dispositif de micromanipulation,

fournir un mouvement rotationnel à la plateforme de microsystèmes d'une durée et d'une vélocité suffisantes pour faire déplacer l'échantillon environnemental gazeux ou particulaire de la voie d'accès pour l'entrée des échantillons à travers le microcanal,

ouvrir chacune des microvalves contrôlant la libération du réactif des réservoirs de réactif en créant un signal de l'unité de contrôle, à un moment et pour une durée moyennant lesquelles le réactif se déplace dans le microcanal et est mélangé avec l'échantillon environnemental,

détecter le mélange de l'échantillon environnemental et du réactif ou du composant gazeux ou particulaire de l'échantillon environnemental directement dans la chambre de détection du gaz ou des particules, moyennant

**54**

quoi le détecteur détecte un signal proportionnel à la quantité de gaz ou de particules présente dans l'échantillon environnemental, et

enregistrer la mesure de la quantité de gaz ou de particules dans l'échantillon environnemental.

**74.** Procédé selon la revendication 73, dans lequel l'échantillon environnemental comprend de l'air, de l'eau, du sol ou de la matière biologique en vrac.

**75.** Procédé selon la revendication 73 ou la revendication 74, dans lequel un gaz est détecté par une puce de captage du gaz.

**76.** Procédé selon l'une quelconque des revendications 73 à 75, dans lequel une particule est détectée dans une chambre de collecte des particules optiquement transparente.

**77.** Procédé selon l'une quelconque des revendications 73 à 76, dans lequel les particules sont détectées par diffusion de lumière cohérente.

**78.** Procédé selon l'une quelconque des revendications 73 à 77, dans lequel une particule est détectée dans une chambre de collecte des particules reliée de manière opérationnelle par un microcanal à un réservoir de réactif comprenant un réactif pour tester chimiquement les particules, dans lequel les particules sont mélangées et réagissent avec un réactif dans le microcanal après libération du réactif par activation d'une microvalve et rotation de la plateforme.

**79.** Procédé de détermination d'une valeur de l'hématocrite d'un échantillon de sang, le procédé comprenant les étapes consistant à

appliquer l'échantillon de sang à l'extrémité proximale d'un microcanal d'une plateforme de microsystèmes selon l'une quelconque des revendications 60 à 63,

placer la plateforme de microsystèmes dans un dispositif de micromanipulation,

fournir un mouvement rotationnel à la plateforme de microsystèmes pendant une durée et avec une vélocité suffisantes pour motiver le déplacement des cellules de globules rouges comprenant l'échantillon de sang le long de l'étendue du microcanal,

scanner le microcanal le long de sa longueur à l'aide de la source de lumière cohérente,

détecter une modification de la diffusion de lumière à une position le long du microcanal qui définit une frontière entre les globules rouges et le plasma du sang,

enregistrer la position de la frontière pour chacun des microcanaux, et

comparer la position de cette frontière pour chacun des microcanaux avec une courbe de référence mettant en relation les valeurs de l'hématocrite et la position de la frontière, et enregistrer l'hématocrite ainsi déterminée.

**80.** Procédé de détermination d'une valeur d'oxygénation du sang d'un échantillon de sang, le procédé comprenant les étapes consistant à

appliquer l'échantillon de sang à l'extrémité proximale d'un microcanal d'une plateforme de microsystèmes selon la revendication 62,

placer la plateforme de microsystèmes dans un dispositif de micromanipulation,

fournir un mouvement de rotation à la plateforme de microsystèmes pendant une durée et avec une vélocité suffisantes pour motiver le venue en contact de l'échantillon de sang avec l'électrode Clarke reliée au microcanal,

détecter une valeur d'oxygénation du sang pour l'échantillon de sang, et

enregistrer la valeur d'oxygénation du sang ainsi déterminée.

## FIG. 1A

## FIG. 1C

## FIG. 1B

## *FIG. 2A*

# FIG. 2B

## FIG. 3A

## FIG. 3B

30 cm

$u_o$ cm/sec

f

## FIG. 4A

## FIG. 4B

## FIG. 5A

## FIG. 5B

## FIG. 5C

# FIG. 5D

# FIG. 6

## FIG. 7

FLOW →

1

FLOW →

OPEN    CLOSE

2

# FIG. 8

AIR FLOW TO
PNEUMATIC VALVES

AIR FLOW TO
PNEUMATIC VALVES

PRESSURE DELIVERED
TO STANDING CORE

# *FIG. 9*

ACTUATION
DIRECTION

OUT

IN

## FIG. 10

## FIG. 11

VALVE FORMATION THROUGH
COLD STAMPING

BLOCKED CHANNEL

ACTUATION THROUGH
HEATING OF VALVE

OPEN CHANNEL

EP 0 865 606 B1

# FIG. 12A

1

2

3

4

5

# FIG. 12B

2

3

4

74

## *FIG. 13A*

LINEAR
CONFIGURATION

## *FIG. 13B*

RADIAL
CONFIGURATION

# *FIG.  13C*

RADIAL
CONFIGURATION

FIG. 14A

FIG. 14B

EP 0 865 606 B1

*FIG. 14C*

EP 0 865 606 B1

EP 0 865 606 B1

## FIG. 14D

The laser beam focuses on the pits and lands of the disc
and is reflected onto the photo diode.

Figure 14E

The photo diode converts the pulses into an electrical signal.

**Figure 14F**

## FIG. 15

```
┌─────────────────────────────┐        ┌──────────────────┐
│           TO DISK           │◄───────│      DISK        │
│                             │        │  COMMUNICATIONS  │
└─────────────────────────────┘        └──────────────────┘

┌──────────┐ ┌──────────┐ ┌──────────┐ ┌──────────┐ ┌──────────┐
│ EXTERNAL │ │ EXTERNAL │ │MECH DRIVE│ │DISK DATA │ │   DATA   │
│DETECTORS │ │ACTUATORS │ │   AND    │ │ OUT/IN   │ │PROCESSOR │
│          │ │          │ │MONITORING│ │          │ │          │
└──────────┘ └──────────┘ └──────────┘ └──────────┘ └──────────┘

┌──────────┐  ┌──────────────────────────┐  ┌──────────────┐
│  ASSAY   │  │     SYSTEM CONTROL:      │  │ FRONT PANEL  │
│PROCESSOR │  │ ROM/FPGAs/PLAs/ASICs, CPU│  │  CONTROLS,   │
│(INC A/D) │  │   AND SOFTWARE, ETC.     │  │ INDICATORS:  │
│          │  │                          │  │    USER      │
└──────────┘  └──────────────────────────┘  │  INTERFACE   │
                                             └──────────────┘

ANALOG OUTPUT

┌──────────┐ ┌──────────────┐ ┌──────────────┐ ┌──────────┐
│          │ │ DIGITAL I/O  │ │COMMUNICATIONS,│ │  DEVICE  │
│ANALOG I/O│ │   RS-232     │ │MODEM, TRANSFER,│ │ MEMORY   │
│          │ │   IEEE-488   │ │  LAN, ETC     │ │          │
│          │ │ SCSI BUS, ETC│ │               │ │          │
└──────────┘ └──────────────┘ └──────────────┘ └──────────┘
```

# *FIG. 16*

LIGHT
EMITTER

LIGHT
COLLECTOR

# FIG. 17A

## FIG. 17B

# FIG. 17C

# FIG. 17D

# FIG. 17E

## FIG. 17F

## FIG. 17G

# FIG. 17H

# *FIG. 17I*

AIR FLOW

AIR FLOW

# FIG. 17J

# FIG. 17K

# *FIG. 17L*

# FIG. 17M

# *FIG. 17N*

AIR FLOW

AIR FLOW

# FIG. 17O

# *FIG. 17P*

## FIG. 17Q

TO
WASTE

# FIG. 17R

TO WASTE

## FIG. 18

## FIG. 19

# *FIG. 20*

## FIG. 21

## FIG. 22

EP 0 865 606 B1

## FIG. 23A

TO
NEXT
ADDITION

*FIG. 23B*

## FIG. 24

## FIG. 25

## _FIG. 26_

OUTLET

# FIG. 27

INLET

OUTLET

# *FIG. 28*

## FIG. 29

## FIG. 30

CONTROL PROGRAM:

CONTROL PROGRAM:

| CD DATA | → | READ ELECTRONICS | → | RAM | → | PROGRAM RUN |

RUN PROGRAM:

| PROCESS BLOCK 1 | → | PROCESS BLOCK 2 | --→ | FINAL PROCESS BLOCK | →

| READ DATA | → | ANALYZE DATA | → | OUTPUT RESULT | → | END PROGRAM |

## FIG. 31A

PROGRAM START

| PROCESS BLOCK 1 | PROCESS BLOCK 2 | FINAL PROCESS BLOCK |

SET SPEED — SET SPEED — SET SPEED

SET RPM FOR VALVE 1 ACTUATION

SET RPM FOR VALVE 2 ACTUATION

OUTPUT RPM SET POINT TO SPEED CONTROL

WAIT UNTIL RPM ACHIEVED

READ RPM FROM SPEED CONTROL

IF RPM < SET

IF RPM = SET

SPIN AT RPM OR SET TIME

(A)    (B)    (C)

## FIG. 31B

(A)

(B)

(C)

CALL SYSTEM
CLOCK

IF TIME < SET

x

IF TIME = SET

STATUS CHECK
SUBROUTINES

STATUS CHECK
SUBROUTINES

GATE DATA READ

IF STATUS "BAD"
END WITH ERROR

IF STATUS "BAD"
END WITH ERROR

IF STATUS
"GOOD" GOTO
NEXT PROCESS

IF STATUS
"GOOD" GOTO
NEXT PROCESS

# FIG. 32A

PROGRAM START

DATA READ BLOCK

ACQUIRE DATA

DISK ENCODER GATE CHAMBER 1 ACQ.

DISK ENCODER GATE CHAMBER 2 ACQ.

DISK ENCODER GATE CHAMBER n ACQ.

IF TIME < SET

IF TIME = SET

(A)

DATA ANALYSIS BLOCK

ERROR FILLING SUBROUTINE

STATUS CHECK SUBROUTINES

IF STATUS "BAD" END WITH ERROR

IF STATUS "GOOD" GOTO RESULT ANALYSIS

RESULT OUTPUT BLOCK

WRITE RESULT TO STORAGE MEDIA

SEND RESULT TO USER INTERFACE

PROGRAM END

## _FIG. 32B_

(A)

STATUS CHECK
SUBROUTINES

IF STATUS "BAD"
END WITH ERROR

IF STATUS
"GOOD" GOTO
DATA ANALYSIS